# EUROPEAN PATENT APPLICATION

(11) **EP 2 471 925 A1**
(43) Date of publication of application: **04.07.2012**
(21) Application number: 12162435.7
(22) Date of filing: 22.03.2008
(51) Int. Cl.: C12N 15/11, C12N 15/115, A01N 43/78, A61K 31/7088

(54) **Methods and compositions related to riboswitches that control alternative splicing**

(30) Priority: 22.03.2007 US 919433 P
(62) Divisional of application: 08732765.6
(71) Applicant: Yale University, New Haven, CT 06510 (US)
(72) Inventor: Breaker, Ronald, Guilford, CT 06437 (US); Sudarsan, Narasimhan, New Haven, CT 06511 (US); Wachter, Andreas, 72116 Mössingen (DE); Cheah, Ming Tatt, Palo Alto, CA 94306 (US)
(74) Representative: Elbel, Michaela

(57) **Abstract**

Disclosed are methods and compositions related to riboswitches that control alternative splicing.

SEQUENCE LISTING

<110> Yale University

<120> METHODS AND COMPOSITIONS RELATED TO RIBOSWITCHES THAT CONTROL ALTERNATIVE
SPLICING

<130> P30033

<150> US60/919,433
<151> 2007-03-22

<160> 101

<170> BiSSAP 1.0

<210> 1
<211> 81
<212> RNA
<213> Artificial Sequence

<220>
<221> source
<222> 1..81
<223> /mol_type="RNA"
      /note="Description of Artificial Sequence; note =synthetic constr
      uct"
      /organism="Artificial Sequence"

<400> 1
agcuaccggg uguccauguu ggccacguug gucugagaaa accggcgaac uugacuggau     60

aauaccagcg aaggauuggc u                                               81



<210> 2
<211> 85
<212> RNA
<213> Artificial Sequence

<220>
<221> source
<222> 1..85
<223> /mol_type="RNA"
      /note="Description of Artificial Sequence; note =synthetic constr
      uct"
      /organism="Artificial Sequence"

<400> 2

acguaccggg uguccaugua ccggaaccac guuggucuga gaaaacgcgc gaacaagaca     60

ggauaauacg uccgaaggau ugcgu                                          85

<210> 3
<211> 18
<212> RNA
<213> Artificial Sequence

<220>
<221> source
<222> 1..18
<223> /mol_type="RNA"
      /note="Description of Artificial Sequence; note =synthetic constr
      uct"
      /organism="Artificial Sequence"

<400> 3
uccaagaucg agguaaag                                                  18

<210> 4
<211> 13
<212> RNA
<213> Artificial Sequence

<220>
<221> source
<222> 1..13
<223> /mol_type="RNA"
      /note="Description of Artificial Sequence; note =synthetic constr
      uct"
      /organism="Artificial Sequence"

<400> 4
aggucuaguu caa                                                       13

<210> 5
<211> 78
<212> RNA
<213> Artificial Sequence

<220>
<221> source
<222> 1..78
<223> /mol_type="RNA"
      /note="Description of Artificial Sequence; note =synthetic constr
      uct"

/organism="Artificial Sequence"

<400> 5
ccaaacgacu cggggugcug agaaauaccc guaucaccug aucuggauaa ugccagccag     60

cguagggaag ucacggac                                                   78

<210> 6
<211> 85
<212> RNA
<213> Artificial Sequence

<220>
<221> source
<222> 1..85
<223> /mol_type="RNA"
      /note="Description of Artificial Sequence; note =synthetic constr
      uct"
      /organism="Artificial Sequence"

<400> 6
uaauuucuug ucggagugcu gagaccguuu auucgggauc cgcggaaccu gaucaggcua     60

auaccugcga agggaacaag aguua                                           85

<210> 7
<211> 82
<212> RNA
<213> Artificial Sequence

<220>
<221> source
<222> 1..82
<223> /mol_type="RNA"
      /note="Description of Artificial Sequence; note =synthetic constr
      uct"
      /organism="Artificial Sequence"

<400> 7
uauuuuagcu aggggugcug agaggagaaa uccaacccuu ugaacuugau guaguuaaua     60

cuaccguagg gaagcagugc au                                              82

<210> 8
<211> 74
<212> RNA
<213> Artificial Sequence

<220>
<221> source
<222> 1..74
<223> /mol_type="RNA"
      /note="Description of Artificial Sequence; note =synthetic constr
      uct"
      /organism="Artificial Sequence"

<400> 8
caagagagcu accgggugcu gagaaauacc ggcgaacuug aucuggauaa uaccagcgaa      60

aggauuggcu ucuu                                                        74



<210> 9
<211> 76
<212> RNA
<213> Artificial Sequence

<220>
<221> source
<222> 1..76
<223> /mol_type="RNA"
      /note="Description of Artificial Sequence; note =synthetic constr
      uct"
      /organism="Artificial Sequence"

<400> 9
gaaugcauga gccggugcug agaauauacg gcgacgaacu ugaucuggau aauaccagcg      60

aaaggaucau ccuuca                                                      76



<210> 10
<211> 75
<212> RNA
<213> Artificial Sequence

<220>
<221> source
<222> 1..75
<223> /mol_type="RNA"
      /note="Description of Artificial Sequence; note =synthetic constr
      uct"
      /organism="Artificial Sequence"

<400> 10
cgcccucacu acgggcgcug agauuguacc gugauuacuc gaucaaguua augcuugcgu      60

gagaaacgug cugcc                                                    75

<210> 11
<211> 80
<212> RNA
<213> Artificial Sequence

<220>
<221> source
<222> 1..80
<223> /mol_type="RNA"
      /note="Description of Artificial Sequence; note =synthetic constr
      uct"
      /organism="Artificial Sequence"

<400> 11
cuuggcgugg gccggugcug agauuauacg gcuaaaacuu gaucuggaua auaccagcga      60

aaagggucaa ucaugccuuc                                                  80

<210> 12
<211> 75
<212> RNA
<213> Artificial Sequence

<220>
<221> source
<222> 1..75
<223> /mol_type="RNA"
      /note="Description of Artificial Sequence; note =synthetic constr
      uct"
      /organism="Artificial Sequence"

<400> 12
   cucccgcaug acaggugcug agauuauacu gugauaacuu gaucuagaua auacuagcga      60
   aaggacaugc guggc                                                       75

<210> 13
<211> 75
<212> RNA
<213> Artificial Sequence

<220>
<221> source
<222> 1..75
<223> /mol_type="RNA"

/note="Description of Artificial Sequence; note =synthetic constr
uct"
/organism="Artificial Sequence"

<400> 13
aucaugcaug agcccgugcu gagauuauac ggcaaaacuu gaucuggaua auaccagcga 60

aaggaucaug ucauc 75

<210> 14
<211> 77
<212> RNA
<213> Artificial Sequence

<220>
<221> source
<222> 1..77
<223> /mol_type="RNA"
/note="Description of Artificial Sequence; note =synthetic constr
uct"
/organism="Artificial Sequence"

<400> 14
aucaugcaug agccgcuccu cugagauuau acggcgaaac uugaucugga uaauaccacc 60

gaaaggauca ugcucuc 77

<210> 15
<211> 74
<212> RNA
<213> Artificial Sequence

<220>
<221> source
<222> 1..74
<223> /mol_type="RNA"
/note="Description of Artificial Sequence; note =synthetic constr
uct"
/organism="Artificial Sequence"

<400> 15
uccacgcacu gcgggugcug agaaaacacc gccgaacucg accaagauaa uacuugcgug 60

agaaagugca cauu 74

<210> 16
<211> 74

<212> RNA
<213> Artificial Sequence

<220>
<221> source
<222> 1..74
<223> /mol_type="RNA"
      /note="Description of Artificial Sequence; note =synthetic constr
      uct"
      /organism="Artificial Sequence"

<400> 16
gacuugcacu gcgggugcug agauuacacc gccacacucg aacaagauaa ugcuugcugg      60

agaaagugcg uuac                                                        74


<210> 17
<211> 75
<212> RNA
<213> Artificial Sequence

<220>
<221> source
<222> 1..75
<223> /mol_type="RNA"
      /note="Description of Artificial Sequence; note =synthetic constr
      uct"
      /organism="Artificial Sequence"

<400> 17
aagaacgcau ccgggugcug agaaauaccg gcgaacuuga ucuggauaau accuacgaaa      60

gcgacaugcu ucuug                                                       75


<210> 18
<211> 74
<212> RNA
<213> Artificial Sequence

<220>
<221> source
<222> 1..74
<223> /mol_type="RNA"
      /note="Description of Artificial Sequence; note =synthetic constr
      uct"
      /organism="Artificial Sequence"

<400> 18

ucaugcauga gccggugcug agaauauacg gcaaaacuug aucuggauaa uaccagcgaa     60

aggaucaugu ccuc                                                       74

<210> 19
<211> 75
<212> RNA
<213> Artificial Sequence

<220>
<221> source
<222> 1..75
<223> /mol_type="RNA"
      /note="Description of Artificial Sequence; note =synthetic constr
      uct"
      /organism="Artificial Sequence"

<400> 19
gcggcucacu acgggcgcug agauugaucc guaaauacuc gaucaaguua augcuugcgu     60

gggaaaagug uugcc                                                      75

<210> 20
<211> 78
<212> RNA
<213> Artificial Sequence

<220>
<221> source
<222> 1..78
<223> /mol_type="RNA"
      /note="Description of Artificial Sequence; note =synthetic constr
      uct"
      /organism="Artificial Sequence"

<400> 20
caaugagacu agcgggugcu gagaauacac cguuugaacu cgaucaaguu aguacuugcg     60

ugaggaaguu uuuguuug                                                   78

<210> 21
<211> 75
<212> RNA
<213> Artificial Sequence

<220>
<221> source

<222> 1..75
<223> /mol_type="RNA"
      /note="Description of Artificial Sequence; note =synthetic constr
      uct"
      /organism="Artificial Sequence"

<400> 21
uuaugcuaga gccggugcug agauuauacg gccuugaacu ugaucuggau gguaccagcg      60

aaaggaucau gcaug                                                       75



<210> 22
<211> 76
<212> RNA
<213> Artificial Sequence

<220>
<221> source
<222> 1..76
<223> /mol_type="RNA"
      /note="Description of Artificial Sequence; note =synthetic constr
      uct"
      /organism="Artificial Sequence"

<400> 22
gcggcucacu acgggcgcug agaguuguac cgugauuacu cgaucaagau aaugcuugcg      60

ugggaaacgu gcugcc                                                      76



<210> 23
<211> 75
<212> RNA
<213> Artificial Sequence

<220>
<221> source
<222> 1..75
<223> /mol_type="RNA"
      /note="Description of Artificial Sequence; note =synthetic constr
      uct"
      /organism="Artificial Sequence"

<400> 23
cauccgcaug acaggugcug agauuauacu gucauaacuu gaucuagaua auacuagcga      60

aaggacaugc guugc                                                       75

<210> 24
<211> 73
<212> RNA
<213> Artificial Sequence

<220>
<221> source
<222> 1..73
<223> /mol_type="RNA"
      /note="Description of Artificial Sequence; note =synthetic constr
      uct"
      /organism="Artificial Sequence"

<400> 24
ugcaguauga ccggugcuga gaucauacgg uugaacuuga ucuggauaau uccagcgaaa      60

gaacucaugc gcg                                                          73



<210> 25
<211> 76
<212> RNA
<213> Artificial Sequence

<220>
<221> source
<222> 1..76
<223> /mol_type="RNA"
      /note="Description of Artificial Sequence; note =synthetic constr
      uct"
      /organism="Artificial Sequence"

<400> 25
ucugggcgug gccggugcug agaauauacg cucaaaacuu gaucuggaua auaccagcga      60

aaggaucaug ccuucc                                                       76



<210> 26
<211> 74
<212> RNA
<213> Artificial Sequence

<220>
<221> source
<222> 1..74
<223> /mol_type="RNA"
      /note="Description of Artificial Sequence; note =synthetic constr
      uct"
      /organism="Artificial Sequence"

<400> 26
cucgcgcaug acagugcuga gauuauacug ucauaacuug aucuagauaa uacuagcgaa     60

aggacuagcg uggc                                                       74



<210> 27
<211> 75
<212> RNA
<213> Artificial Sequence

<220>
<221> source
<222> 1..75
<223> /mol_type="RNA"
      /note="Description of Artificial Sequence; note =synthetic constr
      uct"
      /organism="Artificial Sequence"

<400> 27
uugggcgugg gccggugcug agauuauacg gucaaaacuu gaucuggaua auaccagcga     60

aaggaucaug ccuuc                                                      75



<210> 28
<211> 75
<212> RNA
<213> Artificial Sequence

<220>
<221> source
<222> 1..75
<223> /mol_type="RNA"
      /note="Description of Artificial Sequence; note =synthetic constr
      uct"
      /organism="Artificial Sequence"

<400> 28
ucacauauca uccggugcug agaucauacg guugaacuug aucuggauaa uuccagcgaa     60

aggacucaug cgcgc                                                      75



<210> 29
<211> 76
<212> RNA
<213> Artificial Sequence

<220>
<221> source
<222> 1..76
<223> /mol_type="RNA"
      /note="Description of Artificial Sequence; note =synthetic constr
      uct"
      /organism="Artificial Sequence"

<400> 29
auugcugggu acgggugcug agaucaucac cgucucaacc ugcucgagac aaugcucgcg      60

aagggaaccc cgcuug                                                      76



<210> 30
<211> 73
<212> RNA
<213> Artificial Sequence

<220>
<221> source
<222> 1..73
<223> /mol_type="RNA"
      /note="Description of Artificial Sequence; note =synthetic constr
      uct"
      /organism="Artificial Sequence"

<400> 30
uaugagcaac acgggugcug agaucauacc gucgagcgga acaggauaau gccugcgucg      60

cuaguugcuu ccc                                                         73



<210> 31
<211> 392
<212> RNA
<213> Artificial Sequence

<220>
<221> source
<222> 1..392
<223> /mol_type="RNA"
      /note="Description of Artificial Sequence; note =synthetic constr
      uct"
      /organism="Artificial Sequence"

<400> 31
cucuugacuu ugaaucaaca accugcuaua ccuuugguac gccgccagcg uccgaagagc      60

caugaaaucu aucacaaugg gaacuccaag aucgagguaa aguaaacaag acagcuaccg     120

gguguccaug uugguacagc cacaguuggu ucacccuuau cgcucuucca ugccuucugu 180

gacaaggucu uuggaaugag uuugauacga ugggccaacu gcggcgguac cacguugguc 240

ugagaaauac cggcgaacuu gaucuggaua auaccagcga aaggauuggc uucuugggac 300

cccuuccccu ccccucauug gagaucaacu aacucuaggg uugcagcuuu gcucguguug 360

cgccaacaug ucuaccgaua agauaacuuu cc 392

<210> 32
<211> 527
<212> RNA
<213> Artificial Sequence

<220>
<221> source
<222> 1..527
<223> /mol_type="RNA"
      /note="Description of Artificial Sequence; note =synthetic constr
      uct"
      /organism="Artificial Sequence"

<400> 32
cugucuucgu uccaaggacu accagcaaua aauaccaaca cgcaaguauc accaagcuug 60

uucgggcuuc aucugucucc ucucccccau gguguugaua uacuaacacc cucaagcaag 120

cagacagacu cucaacacca ggguacagac cacccaccuc cuccaccaag auggccacaa 180

accaucguuc caggaggagc ugaagccaug aacuccccau cggcugguug auuauaccau 240

gauucgauca cguucaaaca uacuuucaga gguaacuauc ugaaugcaug agccggugcc 300

aacuucuguc cuggguauua ggucgcuucu agcaccugua cccaggggag aaguacaggc 360

ugagaauaua cggcgacgaa cuugaucugg auaauaccag cgaaaggauc augcuucaac 420

uuuccucccc cgcucccccc uucucccgua cagucauucu aacauuccca gauaacucug 480

auccaucauc accaugucuc ccucuguucu cgagccgcag agcgucc 527

<210> 33
<211> 400
<212> RNA
<213> Artificial Sequence

<220>
<221> source
<222> 1..400
<223> /mol_type="RNA"
      /note="Description of Artificial Sequence; note =synthetic constr
      uct"
      /organism="Artificial Sequence"

<400> 33
auggauauua cucaagguua gaugagaggu ugcggcucac uacgggcgcc gagucaaaag      60

cuugcucgcc uggauccaac cuccugcucu uucauccucg ucuuacacaa aagccuccua     120

ggggaauaug guauuuccga ggauagagag gaugaacaca gaggagaucu ggcgacuugc     180

uggacgacga agcugagauu guaccgugau uacucgauca aguuaaugcu ugcgugagaa     240

acgugcugcc gcuccgaucu auuccccccc ucccuacucc cccuuccucu cuccccuuua     300

cucuuguggg uuggaaaaac uaaccugcgu agcucucggu ucuggaaucc uuuucacuua     360

uccagaaauc gccacguugg cuggucuuca gggcguccuu                           400


<210> 34
<211> 21
<212> DNA
<213> Artificial Sequence

<220>
<221> source
<222> 1..21
<223> /mol_type="DNA"
      /note="Description of Artificial Sequence; note =synthetic constr
      uct"
      /organism="Artificial Sequence"

<400> 34
cattgcaaaa acggcattgg a                                                21


<210> 35
<211> 21
<212> DNA
<213> Artificial Sequence

<220>
<221> source
<222> 1..21
<223> /mol_type="DNA"

/note="Description of Artificial Sequence; note =synthetic constr
uct"
/organism="Artificial Sequence"

<400> 35
tgtggggact tttcatgata c                                                21

<210> 36
<211> 100
<212> RNA
<213> Artificial Sequence

<220>
<221> source
<222> 1..100
<223> /mol_type="RNA"
/note="Description of Artificial Sequence; note =synthetic constr
uct"
/organism="Artificial Sequence"

<400> 36
ggaaacaaga agcuaccggg uguccauguu ggccacguug gucugagaaa uaccggcgaa      60

cuugacugga uaauaccagc gaaaggauug gcuucuuggg                           100

<210> 37
<211> 99
<212> RNA
<213> Artificial Sequence

<220>
<221> source
<222> 1..99
<223> /mol_type="RNA"
/note="Description of Artificial Sequence; note =synthetic constr
uct"
/organism="Artificial Sequence"

<400> 37
gguacagcca caguugguuc acccuuaucg cucuuccaug ccuucuguga caaggucuuu      60

ggaaugaguu ugauacgaug ggccaacugc ggcgguacc                             99

<210> 38
<211> 14
<212> RNA
<213> Artificial Sequence

<220>
<221> source
<222> 1..14
<223> /mol_type="RNA"
/note="Description of Artificial Sequence; note =synthetic constr
uct"
/organism="Artificial Sequence"

<400> 38
uccaagaucg aggu 14

<210> 39
<211> 13
<212> RNA
<213> Artificial Sequence

<220>
<221> source
<222> 1..13
<223> /mol_type="RNA"
/note="Description of Artificial Sequence; note =synthetic constr
uct"
/organism="Artificial Sequence"

<400> 39
aggucuacuu caa 13

<210> 40
<211> 26
<212> RNA
<213> Artificial Sequence

<220>
<221> source
<222> 1..26
<223> /mol_type="RNA"
/note="Description of Artificial Sequence; note =synthetic constr
uct"
/organism="Artificial Sequence"

<400> 40
gggucaaggu aucguacaua guauaa 26

<210> 41
<211> 22
<212> RNA

<213> Artificial Sequence

<220>
<221> source
<222> 1..22
<223> /mol_type="RNA"
      /note="Description of Artificial Sequence; note =synthetic constr
      uct"
      /organism="Artificial Sequence"

<400> 41
ucuaguucaa uacugucaua uu                                                22


<210> 42
<211> 18
<212> RNA
<213> Artificial Sequence

<220>
<221> source
<222> 1..18
<223> /mol_type="RNA"
      /note="Description of Artificial Sequence; note =synthetic constr
      uct"
      /organism="Artificial Sequence"

<400> 42
uauaccaagu aucaaggu                                                     18


<210> 43
<211> 17
<212> RNA
<213> Artificial Sequence

<220>
<221> source
<222> 1..17
<223> /mol_type="RNA"
      /note="Description of Artificial Sequence; note =synthetic constr
      uct"
      /organism="Artificial Sequence"

<400> 43
auaauagguc auaguuc                                                      17


<210> 44
<211> 33

<212> DNA
<213> Artificial Sequence

<220>
<221> source
<222> 1..33
<223> /mol_type="DNA"
      /note="Description of Artificial Sequence; note =synthetic constr
      uct"
      /organism="Artificial Sequence"

<400> 44
ccccgggtct agcatctaga ggacgccaag aac                                    33


<210> 45
<211> 33
<212> DNA
<213> Artificial Sequence

<220>
<221> source
<222> 1..33
<223> /mol_type="DNA"
      /note="Description of Artificial Sequence; note =synthetic constr
      uct"
      /organism="Artificial Sequence"

<400> 45
gttcttggcg tcctctagat gctagacccg ggg                                    33


<210> 46
<211> 36
<212> DNA
<213> Artificial Sequence

<220>
<221> source
<222> 1..36
<223> /mol_type="DNA"
      /note="Description of Artificial Sequence; note =synthetic constr
      uct"
      /organism="Artificial Sequence"

<400> 46
gatccaattg gtcgacaggg ggccttccac ccttcc                                 36


<210> 47

<211> 36
<212> DNA
<213> Artificial Sequence

<220>
<221> source
<222> 1..36
<223> /mol_type="DNA"
      /note="Description of Artificial Sequence; note =synthetic constr
      uct"
      /organism="Artificial Sequence"

<400> 47
gatcgaattc gacggtttgg tgatgacgaa cacggg                                36


<210> 48
<211> 42
<212> DNA
<213> Artificial Sequence

<220>
<221> source
<222> 1..42
<223> /mol_type="DNA"
      /note="Description of Artificial Sequence; note =synthetic constr
      uct"
      /organism="Artificial Sequence"

<400> 48
gatcgaattc ctcttgactt tgaatcaaca acctgctata cc                         42


<210> 49
<211> 38
<212> DNA
<213> Artificial Sequence

<220>
<221> source
<222> 1..38
<223> /mol_type="DNA"
      /note="Description of Artificial Sequence; note =synthetic constr
      uct"
      /organism="Artificial Sequence"

<400> 49
gatctctaga tcggtagaca tgttggcgca acacgagc                              38

<210> 50
<211> 32
<212> DNA
<213> Artificial Sequence

<220>
<221> source
<222> 1..32
<223> /mol_type="DNA"
      /note="Description of Artificial Sequence; note =synthetic constr
      uct"
      /organism="Artificial Sequence"

<400> 50
ggtaaagtaa acaagacacg taccgggtgt cc                                32


<210> 51
<211> 32
<212> DNA
<213> Artificial Sequence

<220>
<221> source
<222> 1..32
<223> /mol_type="DNA"
      /note="Description of Artificial Sequence; note =synthetic constr
      uct"
      /organism="Artificial Sequence"

<400> 51
ggacacccgg tacgtgtctt gtttacttta cc                                32


<210> 52
<211> 30
<212> DNA
<213> Artificial Sequence

<220>
<221> source
<222> 1..30
<223> /mol_type="DNA"
      /note="Description of Artificial Sequence; note =synthetic constr
      uct"
      /organism="Artificial Sequence"

<400> 52
ccagcgaaag gattgcgttc ttgggacccc                                   30

<210> 53
<211> 30
<212> DNA
<213> Artificial Sequence

<220>
<221> source
<222> 1..30
<223> /mol_type="DNA"
      /note="Description of Artificial Sequence; note =synthetic constr
      uct"
      /organism="Artificial Sequence"

<400> 53
ggggtcccaa gaacgcaatc ctttcgctgg                                        30


<210> 54
<211> 33
<212> DNA
<213> Artificial Sequence

<220>
<221> source
<222> 1..33
<223> /mol_type="DNA"
      /note="Description of Artificial Sequence; note =synthetic constr
      uct"
      /organism="Artificial Sequence"

<400> 54
gtaaacaaga cagctacgcg gtgtccatgt tgg                                    33


<210> 55
<211> 33
<212> DNA
<213> Artificial Sequence

<220>
<221> source
<222> 1..33
<223> /mol_type="DNA"
      /note="Description of Artificial Sequence; note =synthetic constr
      uct"
      /organism="Artificial Sequence"

<400> 55
ccaacatgga caccgcgtag ctgtcttgtt tac                                    33

<210> 56
<211> 31
<212> DNA
<213> Artificial Sequence

<220>
<221> source
<222> 1..31
<223> /mol_type="DNA"
      /note="Description of Artificial Sequence; note =synthetic constr
      uct"
      /organism="Artificial Sequence"

<400> 56
ggtctgagaa atacgcgcga acttgatctg g                                    31



<210> 57
<211> 31
<212> DNA
<213> Artificial Sequence

<220>
<221> source
<222> 1..31
<223> /mol_type="DNA"
      /note="Description of Artificial Sequence; note =synthetic constr
      uct"
      /organism="Artificial Sequence"

<400> 57
ccagatcaag ttcgcgcgta tttctcagac c                                    31



<210> 58
<211> 32
<212> DNA
<213> Artificial Sequence

<220>
<221> source
<222> 1..32
<223> /mol_type="DNA"
      /note="Description of Artificial Sequence; note =synthetic constr
      uct"
      /organism="Artificial Sequence"

<400> 58

ccgggtgtcc atgtacctac agccacagtt gg                                    32

<210> 59
<211> 32
<212> DNA
<213> Artificial Sequence

<220>
<221> source
<222> 1..32
<223> /mol_type="DNA"
      /note="Description of Artificial Sequence; note =synthetic constr
      uct"
      /organism="Artificial Sequence"

<400> 59
ccaactgtgg ctgtaggtac atggacaccc gg                                    32

<210> 60
<211> 32
<212> DNA
<213> Artificial Sequence

<220>
<221> source
<222> 1..32
<223> /mol_type="DNA"
      /note="Description of Artificial Sequence; note =synthetic constr
      uct"
      /organism="Artificial Sequence"

<400> 60
gaaataccgg cgaacaagat ctggataata cc                                    32

<210> 61
<211> 32
<212> DNA
<213> Artificial Sequence

<220>
<221> source
<222> 1..32
<223> /mol_type="DNA"
      /note="Description of Artificial Sequence; note =synthetic constr
      uct"
      /organism="Artificial Sequence"

<400> 61
ggtattatcc agatcttgtt cgccggtatt tc                                    32

<210> 62
<211> 32
<212> DNA
<213> Artificial Sequence

<220>
<221> source
<222> 1..32
<223> /mol_type="DNA"
      /note="Description of Artificial Sequence; note =synthetic constr
      uct"
      /organism="Artificial Sequence"

<400> 62
ggataatacc agcgattgga ttggcttctt gg                                    32

<210> 63
<211> 32
<212> DNA
<213> Artificial Sequence

<220>
<221> source
<222> 1..32
<223> /mol_type="DNA"
      /note="Description of Artificial Sequence; note =synthetic constr
      uct"
      /organism="Artificial Sequence"

<400> 63
ccaagaagcc aatccaatcg ctggtattat cc                                    32

<210> 64
<211> 30
<212> DNA
<213> Artificial Sequence

<220>
<221> source
<222> 1..30
<223> /mol_type="DNA"
      /note="Description of Artificial Sequence; note =synthetic constr
      uct"
      /organism="Artificial Sequence"

<400> 64
ccggcgaact tgatgacgat aataccagcg                                        30

<210> 65
<211> 30
<212> DNA
<213> Artificial Sequence

<220>
<221> source
<222> 1..30
<223> /mol_type="DNA"
      /note="Description of Artificial Sequence; note =synthetic constr
      uct"
      /organism="Artificial Sequence"

<400> 65
cgctggtatt atcgtcatca agttcgccgg                                        30

<210> 66
<211> 33
<212> DNA
<213> Artificial Sequence

<220>
<221> source
<222> 1..33
<223> /mol_type="DNA"
      /note="Description of Artificial Sequence; note =synthetic constr
      uct"
      /organism="Artificial Sequence"

<400> 66
cttgatgacg ataatacgtc cgaaaggatt ggc                                    33

<210> 67
<211> 33
<212> DNA
<213> Artificial Sequence

<220>
<221> source
<222> 1..33
<223> /mol_type="DNA"
      /note="Description of Artificial Sequence; note =synthetic constr
      uct"

      /organism="Artificial Sequence"

<400> 67
gccaatcctt tcggacgtat tatcgtcatc aag                                    33

<210> 68
<211> 42
<212> DNA
<213> Artificial Sequence

<220>
<221> source
<222> 1..42
<223> /mol_type="DNA"
      /note="Description of Artificial Sequence; note =synthetic constr
      uct"
      /organism="Artificial Sequence"

<400> 68
gatcgaattc ctcttgactt tgaatcaaca acctgctata cc                          42

<210> 69
<211> 36
<212> DNA
<213> Artificial Sequence

<220>
<221> source
<222> 1..36
<223> /mol_type="DNA"
      /note="Description of Artificial Sequence; note =synthetic constr
      uct"
      /organism="Artificial Sequence"

<400> 69
gaccaacgtg gttccccaac atggacaccc ggtagc                                 36

<210> 70
<211> 23
<212> DNA
<213> Artificial Sequence

<220>
<221> source
<222> 1..23
<223> /mol_type="DNA"
      /note="Description of Artificial Sequence; note =synthetic constr

uct"
      /organism="Artificial Sequence"

<400> 70
ggaaccacgt tggtctgaga aat                                              23



<210> 71
<211> 38
<212> DNA
<213> Artificial Sequence

<220>
<221> source
<222> 1..38
<223> /mol_type="DNA"
      /note="Description of Artificial Sequence; note =synthetic constr
      uct"
      /organism="Artificial Sequence"

<400> 71
gatctctaga tcggtagaca tgttggcgca acacgagc                              38



<210> 72
<211> 24
<212> DNA
<213> Artificial Sequence

<220>
<221> source
<222> 1..24
<223> /mol_type="DNA"
      /note="Description of Artificial Sequence; note =synthetic constr
      uct"
      /organism="Artificial Sequence"

<400> 72
ccgaggagcc aagaaatcta tcac                                             24



<210> 73
<211> 24
<212> DNA
<213> Artificial Sequence

<220>
<221> source
<222> 1..24
<223> /mol_type="DNA"

/note="Description of Artificial Sequence; note =synthetic constr
uct"
/organism="Artificial Sequence"

<400> 73
gtgatagatt tcttggctcc tcgg                                              24

<210> 74
<211> 27
<212> DNA
<213> Artificial Sequence

<220>
<221> source
<222> 1..27
<223> /mol_type="DNA"
/note="Description of Artificial Sequence; note =synthetic constr
uct"
/organism="Artificial Sequence"

<400> 74
gaaatctatc acaaagggaa ctccaag                                           27

<210> 75
<211> 27
<212> DNA
<213> Artificial Sequence

<220>
<221> source
<222> 1..27
<223> /mol_type="DNA"
/note="Description of Artificial Sequence; note =synthetic constr
uct"
/organism="Artificial Sequence"

<400> 75
cttggagttc cctttgtgat agatttc                                           27

<210> 76
<211> 32
<212> DNA
<213> Artificial Sequence

<220>
<221> source
<222> 1..32

<223> /mol_type="DNA"
      /note="Description of Artificial Sequence; note =synthetic constr
      uct"
      /organism="Artificial Sequence"

<400> 76
cctgctatac ctttgcgacg ccgccagcgt cc                                    32


<210> 77
<211> 32
<212> DNA
<213> Artificial Sequence

<220>
<221> source
<222> 1..32
<223> /mol_type="DNA"
      /note="Description of Artificial Sequence; note =synthetic constr
      uct"
      /organism="Artificial Sequence"

<400> 77
ggacgctggc ggcgtcgcaa aggtatagca gg                                    32


<210> 78
<211> 29
<212> DNA
<213> Artificial Sequence

<220>
<221> source
<222> 1..29
<223> /mol_type="DNA"
      /note="Description of Artificial Sequence; note =synthetic constr
      uct"
      /organism="Artificial Sequence"

<400> 78
cattggagat caacgacctc tagggttgc                                        29


<210> 79
<211> 29
<212> DNA
<213> Artificial Sequence

<220>
<221> source

<222> 1..29
<223> /mol_type="DNA"
      /note="Description of Artificial Sequence; note =synthetic constr
      uct"
      /organism="Artificial Sequence"

<400> 79
gcaaccctag aggtcgttga tctccaatg                                         29


<210> 80
<211> 29
<212> DNA
<213> Artificial Sequence

<220>
<221> source
<222> 1..29
<223> /mol_type="DNA"
      /note="Description of Artificial Sequence; note =synthetic constr
      uct"
      /organism="Artificial Sequence"

<400> 80
ctctagggtt gcgactttgc tcgtgttgc                                         29


<210> 81
<211> 29
<212> DNA
<213> Artificial Sequence

<220>
<221> source
<222> 1..29
<223> /mol_type="DNA"
      /note="Description of Artificial Sequence; note =synthetic constr
      uct"
      /organism="Artificial Sequence"

<400> 81
gcaacacgag caaagtcgca accctagag                                         29


<210> 82
<211> 36
<212> DNA
<213> Artificial Sequence

<220>

<221> source
<222> 1..36
<223> /mol_type="DNA"
      /note="Description of Artificial Sequence; note =synthetic constr
      uct"
      /organism="Artificial Sequence"

<400> 82
ctcttgactt tgaatcaaca acctgctata cctttg                                36

<210> 83
<211> 19
<212> DNA
<213> Artificial Sequence

<220>
<221> source
<222> 1..19
<223> /mol_type="DNA"
      /note="Description of Artificial Sequence; note =synthetic constr
      uct"
      /organism="Artificial Sequence"

<400> 83
cgtagcgtgc cagttggcg                                                   19

<210> 84
<211> 37
<212> DNA
<213> Artificial Sequence

<220>
<221> source
<222> 1..37
<223> /mol_type="DNA"
      /note="Description of Artificial Sequence; note =synthetic constr
      uct"
      /organism="Artificial Sequence"

<400> 84
ctgtcttcgt tccaaggact accatcaatt aaatacc                               37

<210> 85
<211> 24
<212> DNA
<213> Artificial Sequence

<220>
<221> source
<222> 1..24
<223> /mol_type="DNA"
      /note="Description of Artificial Sequence; note =synthetic constr
      uct"
      /organism="Artificial Sequence"

<400> 85
gctccagaag aggcttggac aagc                                              24


<210> 86
<211> 32
<212> DNA
<213> Artificial Sequence

<220>
<221> source
<222> 1..32
<223> /mol_type="DNA"
      /note="Description of Artificial Sequence; note =synthetic constr
      uct"
      /organism="Artificial Sequence"

<400> 86
atgcgaattc cacccaatct ccaacctatc tt                                     32


<210> 87
<211> 29
<212> DNA
<213> Artificial Sequence

<220>
<221> source
<222> 1..29
<223> /mol_type="DNA"
      /note="Description of Artificial Sequence; note =synthetic constr
      uct"
      /organism="Artificial Sequence"

<400> 87
atgctctaga ccagccaacg tggcgattt                                         29


<210> 88
<211> 36
<212> DNA
<213> Artificial Sequence

<220>
<221> source
<222> 1..36
<223> /mol_type="DNA"
      /note="Description of Artificial Sequence; note =synthetic constr
      uct"
      /organism="Artificial Sequence"

<400> 88
ctcttgactt tgaatcaaca acctgctata cctttg                                36


<210> 89
<211> 19
<212> DNA
<213> Artificial Sequence

<220>
<221> source
<222> 1..19
<223> /mol_type="DNA"
      /note="Description of Artificial Sequence; note =synthetic constr
      uct"
      /organism="Artificial Sequence"

<400> 89
gatgtgggcg tcggtgaag                                                   19


<210> 90
<211> 40
<212> DNA
<213> Artificial Sequence

<220>
<221> source
<222> 1..40
<223> /mol_type="DNA"
      /note="Description of Artificial Sequence; note =synthetic constr
      uct"
      /organism="Artificial Sequence"

<400> 90
taatacgact cactatagga aacaagacag ctaccgggtg                            40


<210> 91
<211> 22
<212> DNA

<213> Artificial Sequence

<220>
<221> source
<222> 1..22
<223> /mol_type="DNA"
      /note="Description of Artificial Sequence; note =synthetic constr
      uct"
      /organism="Artificial Sequence"

<400> 91
cccaagaagc caatcctttc gc                                                22


<210> 92
<211> 40
<212> DNA
<213> Artificial Sequence

<220>
<221> source
<222> 1..40
<223> /mol_type="DNA"
      /note="Description of Artificial Sequence; note =synthetic constr
      uct"
      /organism="Artificial Sequence"

<400> 92
taatacgact cactataggc tatacctttg gtacgccgcc                             40


<210> 93
<211> 31
<212> DNA
<213> Artificial Sequence

<220>
<221> source
<222> 1..31
<223> /mol_type="DNA"
      /note="Description of Artificial Sequence; note =synthetic constr
      uct"
      /organism="Artificial Sequence"

<400> 93
caagaagcca atcctttcgc tggtattatc c                                      31


<210> 94
<211> 35

<212> DNA
<213> Artificial Sequence

<220>
<221> source
<222> 1..35
<223> /mol_type="DNA"
      /note="Description of Artificial Sequence; note =synthetic constr
      uct"
      /organism="Artificial Sequence"

<400> 94
taatacgact cactatagga cagctaccgg gtgtc                                35

<210> 95
<211> 25
<212> DNA
<213> Artificial Sequence

<220>
<221> source
<222> 1..25
<223> /mol_type="DNA"
      /note="Description of Artificial Sequence; note =synthetic constr
      uct"
      /organism="Artificial Sequence"

<400> 95
acatgttggc gcaacacgag caaag                                           25

<210> 96
<211> 24
<212> RNA
<213> Artificial Sequence

<220>
<221> source
<222> 1..24
<223> /mol_type="RNA"
      /note="Description of Artificial Sequence; note =synthetic constr
      uct"
      /organism="Artificial Sequence"

<400> 96
gggucaaggu aucguacaga uuau                                            24

<210> 97

<211> 20
<212> RNA
<213> Artificial Sequence

<220>
<221> source
<222> 1..20
<223> /mol_type="RNA"
      /note="Description of Artificial Sequence; note =synthetic constr
      uct"
      /organism="Artificial Sequence"

<400> 97
ucuaguucaa uacugucaua                                                20



<210> 98
<211> 22
<212> RNA
<213> Artificial Sequence

<220>
<221> source
<222> 1..22
<223> /mol_type="RNA"
      /note="Description of Artificial Sequence; note =synthetic constr
      uct"
      /organism="Artificial Sequence"

<400> 98
auucaauuca aaucaucaag gu                                             22



<210> 99
<211> 17
<212> RNA
<213> Artificial Sequence

<220>
<221> source
<222> 1..17
<223> /mol_type="RNA"
      /note="Description of Artificial Sequence; note =synthetic constr
      uct"
      /organism="Artificial Sequence"

<400> 99
uaauaggucu aguucaa                                                   17

<210> 100
<211> 28
<212> RNA
<213> Artificial Sequence

<220>
<221> source
<222> 1..28
<223> /mol_type="RNA"
      /note="Description of Artificial Sequence; note =synthetic constr
      uct"
      /organism="Artificial Sequence"

<400> 100
ugggucaagg uaucguagau uguauaau                                        28


<210> 101
<211> 26
<212> RNA
<213> Artificial Sequence

<220>
<221> source
<222> 1..26
<223> /mol_type="RNA"
      /note="Description of Artificial Sequence; note =synthetic constr
      uct"
      /organism="Artificial Sequence"

<400> 101
aucuaguucc aauacuuguc auauua                                          26

## Description

### FIELD OF THE INVENTION

The disclosed invention is generally in the field of gene expression and specifically in the area of regulation of gene expression.

### BACKGROUND OF THE INVENTION

Precision genetic control is an essential feature of living systems, as cells must respond to a multitude of biochemical signals and environmental cues by varying genetic expression patterns. Most known mechanisms of genetic control involve the use of protein factors that sense chemical or physical stimuli and then modulate gene expression by selectively interacting with the relevant DNA or messenger RNA sequence. Proteins can adopt complex shapes and carry out a variety of functions that permit living systems to sense accurately their chemical and physical environments. Protein factors that respond to metabolites typically act by binding DNA to modulate transcription initiation (e.g. the lac repressor protein; Matthews, K.S., and Nichols, J.C., 1998, Prog. Nucleic Acids Res. Mol. Biol. 58, 127-164) or by binding RNA to control either transcription termination (e.g. the PyrR protein; Switzer, R.L., et al., 1999, Prog. Nucleic Acids Res. Mol. Biol. 62, 329-367) or translation (e.g. the TRAP protein; Babitzke, P., and Gollnick, P., 2001, J. Bacteriol. 183, 5795-5802). Protein factors respond to environmental stimuli by various mechanisms such as allosteric modulation or post-translational modification, and are adept at exploiting these mechanisms to serve as highly responsive genetic switches (e.g. see Ptashne, M., and Gann, A. (2002). Genes and Signals. Cold Spring Harbor Laboratory Press, Cold Spring Harbor, NY).

In addition to the widespread participation of protein factors in genetic control, it is also known that RNA can take an active role in genetic regulation. Recent studies have begun to reveal the substantial role that small non-coding RNAs play in selectively targeting mRNAs for destruction, which results in down-regulation of gene expression (*e.g.* see Hannon, G.J. 2002, Nature 418, 244-251 and references therein). This process of RNA interference takes advantage of the ability of short RNAs to recognize the intended mRNA target selectively via Watson-Crick base complementation, after which the bound mRNAs are destroyed by the action of proteins. RNAs are ideal agents for molecular recognition in this system because it is far easier to generate new target-specific RNA factors through evolutionary processes than it would be to generate protein factors with novel but highly specific RNA binding sites.

Although proteins fulfill most requirements that biology has for enzyme, receptor and structural functions, RNA also can serve in these capacities. For example, RNA has sufficient structural plasticity to form numerous ribozyme domains (Cech & Golden, Building a catalytic active site using only RNA. In: The RNA World R. F. Gesteland, T. R. Cech, J. F. Atkins, eds., pp.321-350 (1998); Breaker, In vitro selection of catalytic polynucleotides. Chem. Rev. 97, 371-390 (1997)) and receptor domains (Osborne & Ellington, Nucleic acid selection and the challenge of combinatorial chemistry. Chem. Rev. 97, 349-370 (1997); Hermann & Patel, Adaptive recognition by nucleic acid aptamers. Science 287, 820-825 (2000)) that exhibit considerable enzymatic power and precise molecular recognition. Furthermore, these activities can be combined to create allosteric ribozymes (Soukup & Breaker, Engineering precision RNA molecular switches. Proc. Natl. Acad. Sci. USA 96, 3584-3589 (1999); Seetharaman et al., Immobilized riboswitches for the analysis of complex chemical and biological mixtures. Nature Biotechnol. 19, 336-341 (2001)) that are selectively modulated by effector molecules.

Alternative splicing is a process which involves the selective use of splice sites on a mRNA precursor. Alternative splicing allows the production of many proteins from a single gene and therefore allows the generation of proteins with distinct functions. Alternative splicing events can occur through a variety of ways including exon skipping, the use of mutually exclusive exons and the differential selection of 5' and/or 3' splice sites. For many genes (e.g., homeogenes, oncogenes, neuropeptides, extracellular matrix proteins, muscle contractile proteins), alternative splicing is regulated in a developmental or tissue-specific fashion. Alternative splicing therefore plays a critical role in gene expression. Recent studies have revealed the importance of alternative splicing in the expression strategies of complex organisms.

Alternative splicing of mRNA precursors (pre-mRNAs) plays an important role in the regulation of mammalian gene expression. The regulation of alternative splicing occurs in cells of various lineages and is part of the expression program of a large number of genes. Recently, it has become clear that alternative splicing controls the production of proteins isoforms which, sometimes, have completely different functions. Oncogene and proto-oncogene protein isoforms with different and sometimes antagonistic properties on cell transformation are produced via alternative splicing. Examples of this kind are found in Makela, T. P. et al. 1992, Science 256:373; Yen, J. et al. 1991, Proc. Natl. Acad. Sci. U.S.A. 88:5077; Mumberg, D. et al. 1991, Genes Dev. 5:1212; Foulkes, N. S. and Sassone-Corsi, P. 1992, Cell 68:411. Also, alternative splicing is often used to control the production of proteins involved in programmed cell death such as Fas, Bcl-2, Bax, and Ced-4 (Jiang, Z. H. and Wu J. Y., 1999, Proc Soc Exp Biol Med 220: 64). Alternative splicing of a pre-mRNA can produce a repressor protein, while an activator may be produced from the same pre-mRNA in different conditions (Black D. L. 2000, Cell 103:367; Graveley, B. R. 2001, Trends Genet. 17:100). What is needed in the art are methods and compositions that can be used to regulate alternative splicing via riboswitches.

### BRIEF SUMMARY OF THE INVENTION

Disclosed herein is a regulatable gene expression construct comprising a nucleic acid molecule encoding an RNA comprising a riboswitch operably linked to a coding region, wherein the riboswitch regulates splicing of the RNA, wherein the riboswitch and coding region are heterologous. The riboswitch can regulate alternative spicing of the RNA. The riboswitch can comprise an aptamer domain and an expression platform domain, wherein the aptamer domain and the expression platform domain are heterologous. The RNA can further comprises an intron, wherein the expression platform domain comprises an alternative splice junction in the intron. The RNA can further comprise an intron, wherein the expression platform domain comprises a splice junction at an end of the intron (that is, the 5' splice junction or the 3' splice junction). The RNA can further comprises an intron, wherein the expression platform domain comprises the branch site in the intron. The alternative splice junction can be active when the riboswitch is activated. The alternative splice junction can be active when the riboswitch is not activated.The riboswitch can be activated by a trigger molecule, such as thiamine pyrophosphate (TPP). The riboswitch can be a TPP-responsive riboswitch. The riboswitch can activate alternative splicing. The riboswitch can repress alternative splicing. The riboswitch can alter splicing of the RNA.The RNA can have a branched structure. The RNA can be pre-mRNA. The region of the aptamer with splicing control can be located, for example, in the P4 and P5 stem. The region of the aptamer with splicing control can also found, for example, in loop 5. The region of the aptamer with splicing control can also found, for example, in stem P2. Thus, for example, an expression platform domain can interact with the P4 and P5 sequences, the loop 5 sequence and/or the P2 sequences. Such aptamer sequences generally can be available for interaction with the expression platform domain only when a trigger molecule is not bound to the aptamer domain. The splice sites and/or branch sites can be located, for example, at positions between -6 to -24 relative to the 5' end of the aptamer. The splice sites can follow, for example, the sequence GUA.

Also disclosed is a method for regulating splicing of RNA comprising introducing into the RNA a construct comprising a riboswitch, wherein the riboswitch is capable of regulating splicing of RNA. The riboswitch can comprise an aptamer domain and an expression platform domain, wherein the aptamer domain and the expression platform domain are heterologous. The riboswitch can be in an intron of the RNA. The riboswitch can be activated by a trigger molecule, such as TPP. The riboswitch can be a TPP-responsive riboswitch. The riboswitch can activate alternative splicing. The riboswitch can repress alternative splicing. The riboswitch can alter splicing of the RNA.The splicing can occur non-naturally. The region of the aptamer with alternative splicing control can be found, for example, in loop 5. The region of the aptamer with alternative splicing control can also found, for example, in stem P2. The splice sites can be located, for example, at positions between -6 to -24 relative to the 5' end of the aptamer. The splice sites can follow, for example, the sequence GUA in the aptamer.

Also disclosed is a method of inhibiting fungal growth, the method comprising: identifying a subject with a fungal infection; administering to the subject an effective amount of a compound that inhibits a TPP-responsive riboswitch, thereby inhibiting fungal growth. Inhibiting fungal growth can comprise a 10% or more reduction in fungal biomass.

A first object of the present invention is a regulatable gene expression construct comprising a nucleic acid molecule encoding an RNA comprising a riboswitch operably linked to a coding region, wherein the riboswitch regulates splicing of the RNA, wherein the riboswitch and coding region are heterologous.

In a preferred embodiment, the construct regulates alternative splicing.

In a preferred embodiment, the riboswitch comprises an aptamer domain and an expression platform domain, wherein the aptamer domain and the expression platform domain are heterologous.

In a preferred embodiment, the RNA further comprises an intron, wherein the expression platform domain comprises an alternative splice junction in the intron.

In a preferred embodiment, the RNA further comprises an intron, wherein the expression platform domain comprises a splice junction at an end of the intron.

In a preferred embodiment, the alternative splice junction is active when the riboswitch is activated.

In a preferred embodiment, the alternative splice junction is active when the riboswitch is not activated.

In a preferred embodiment, the riboswitch is activated by a trigger molecule.

In a preferred embodiment, the trigger molecule is TPP.

In a preferred embodiment, the riboswitch is a TPP-responsive riboswitch.

In a preferred embodiment, the riboswitch activates alternative splicing.

In a preferred embodiment, the riboswitch represses alternative splicing.

In a preferred embodiment, the RNA has a branched structure.

In a preferred embodiment, the RNA is pre-mRNA.

In a preferred embodiment, the region of the aptamer domain with splicing control is located in the P4 and P5 stem.

In a preferred embodiment, the region of the aptamer domain with splicing control is also located in loop 5.

In a preferred embodiment, the region of the aptamer domain with splicing control is also located in stem P2.

In a preferred embodiment, the splice sites are located at positions between -6 to -24 relative to the 5' end of the aptamer domain.

In a preferred embodiment, the splice sites follow the sequence GUA.

A further object of the present invention is a method for regulating splicing of RNA comprising introducing into the RNA a construct comprising a riboswitch, wherein the riboswitch is capable of regulating splicing of RNA.

In a preferred embodiment, the riboswitch comprises an aptamer domain and an expression platform domain, wherein the aptamer domain and the expression platform domain are heterologous.

In a preferred embodiment, the riboswitch is in an intron of the RNA.

In a preferred embodiment, the riboswitch is activated by a trigger molecule.

In a preferred embodiment, the trigger molecule is TPP.

In a preferred embodiment, the riboswitch is a TPP-responsive riboswitch.

In a preferred embodiment, the riboswitch activates alternative splicing.

In a preferred embodiment, the riboswitch represses alternative splicing.

In a preferred embodiment, said splicing does not occur naturally.

In a preferred embodiment, the region of the aptamer domain with splicing control is located in loop 5.

In a preferred embodiment, the region of the aptamer domain with splicing control is located in stem P2.

In a preferred embodiment, the splice sites are located at positions between -6 to -24 relative to the 5' end of the aptamer domain.

In a preferred embodiment, the splice sites follow the sequence GUA in the aptamer domain.

A further object of the present invention is a method of inhibiting fungal growth, the method comprising: identifying a subject with a fungal infection; administering to the subject an effective amount of a compound that inhibits a TPP-responsive riboswitch, thereby inhibiting fungal growth.

In a preferred embodiment, inhibiting fungal growth comprises a 10% or more reduction in fungal biomass.

Additional advantages of the disclosed method and compositions will be set forth in part in the description which follows, and in part will be understood from the

description, or can be learned by practice of the disclosed method and compositions. The advantages of the disclosed method and compositions will be realized and attained by means of the elements and combinations particularly pointed out in the appended claims. It is to be understood that both the foregoing general description and the following detailed description are exemplary and explanatory only and are not restrictive of the invention as claimed.

### BRIEF DESCRIPTION OF THE DRAWINGS

The accompanying drawings, which are incorporated in and constitute a part of this specification, illustrate several embodiments of the disclosed method and compositions and together with the description, serve to explain the principles of the disclosed method and compositions.

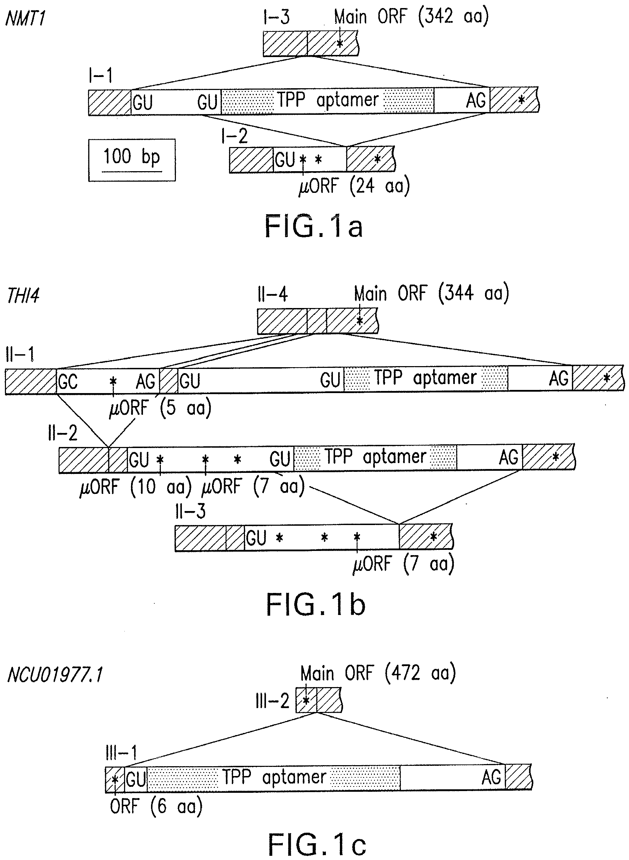

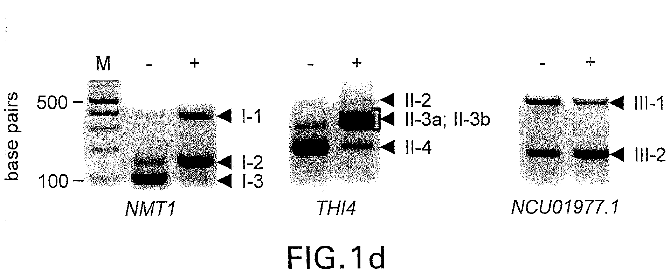

Figure 1 shows three *N. crassa* genes carry TPP riboswitches in 5' introns. a, Precursor 5' UTR (I-1) and alternatively spliced products (I-2 and I-3) for the *NMT1* mRNA. Exons and introns are dark gray and unshaded/light gray rectangles, respectively. 5' (GU) and 3' (AG) splice sites, putative start codons (*) and the corresponding translation products from the uORF and the main *NMT1* ORF are depicted. b and c, 5' UTRs of precursor mRNAs and their spliced products for *THI4* and *NCU01977.1,* respectively. d, RT-PCR detection of mRNA 5' regionss from *N. crassa* grown in the absence (-) or presence (+) of 30 µM thiamine. Bands II-3a and II-3b for *THI4* represent the splice form II-3 with the upstream intron remaining (3a) or removed (3b). Marker DNAs (M) are in 100 base pair increments.

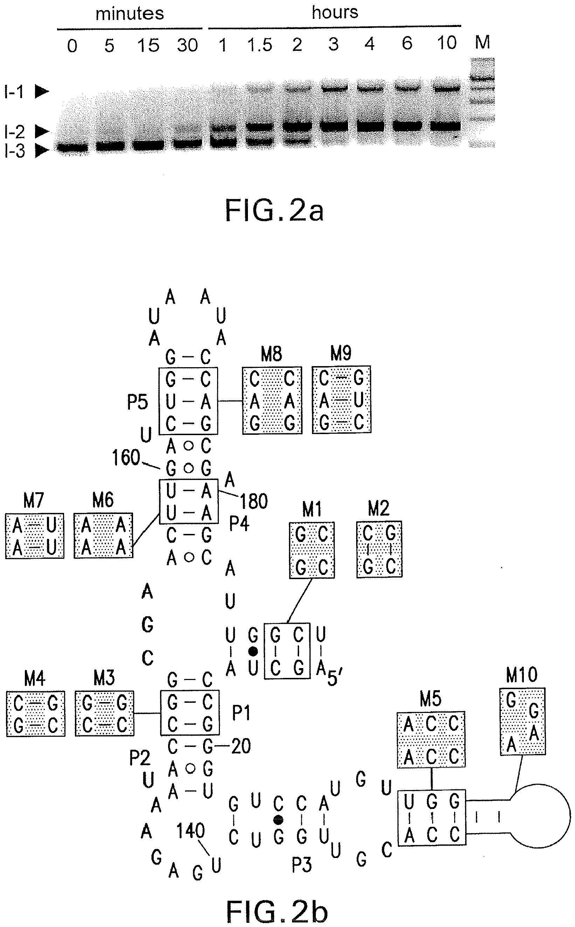

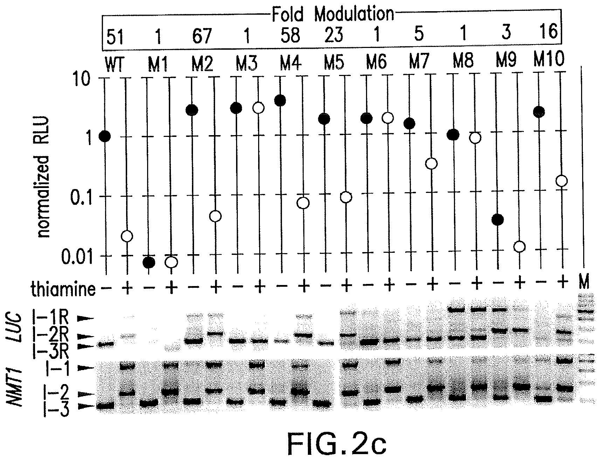

Figure 2 shows alternative splicing and gene control by the *NMT1* TPP riboswitch. a, Change in *NMT1* transcript splicing (RT-PCR products) after addition of 30 µM thiamine (t = 0) to a culture *of N. crassa* grown in thiamine-free medium. b, Reporter constructs of wild-type (WT) or various mutant *NMT1* riboswitches (M1 through M10) fused upstream of a luciferase (*LUC*) ORF (SEQ ID NOS: 1 and 2). c, Top: relative light units (RLU) from the WT-*LUC* construct (normalized RLU = 1) versus various mutant *NMT1-LUC* constructs grown in the absence (filled circles) or presence (open circles) of 30 µM thiamine. Values are the averages from three independent assay repeats and standard deviation error bars are smaller than the diameter of the symbols. Bottom: RT-PCR analyses of the 5' UTRs from the *LUC* fusions (upper panel) and the native *NMT1* RNA (lower panel) for each transformant. Details are described in Fig. 1.

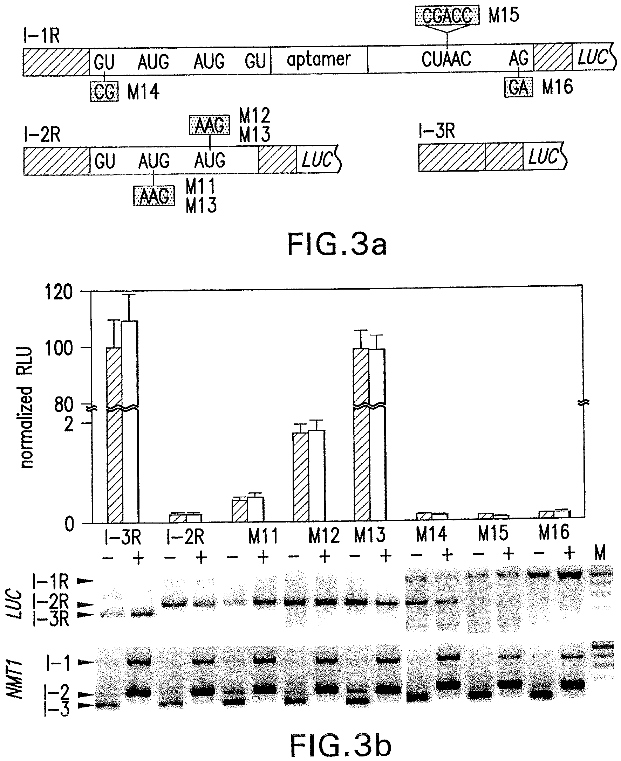

Figure 3 shows short uORFs in unspliced and alternatively spliced mRNAs cause *NMT1* repression. a, Wild-type and mutant constructs fused to a *LUC* reporter to simulate unspliced RNA (I-1R) and spliced RNAs (I-2R and I-3R). b, (Top) *LUC* activity in the absence (-, filled bars) or presence (+, open bars) of 30 µM thiamine in the medium. Expression was normalized relative to the value of the wild-type I-3R construct without addition of thiamine. Values are the averages from three independent assay repeats and standard deviation error bars are shown. (Bottom) RT-PCR analysis of the *LUC* fusion (upper panel) and native *NMT1* (lower panel) transcripts for *N crassa* grown without (-) or with (+) thiamine. Other notations are as described in the legend to Fig. 2c.

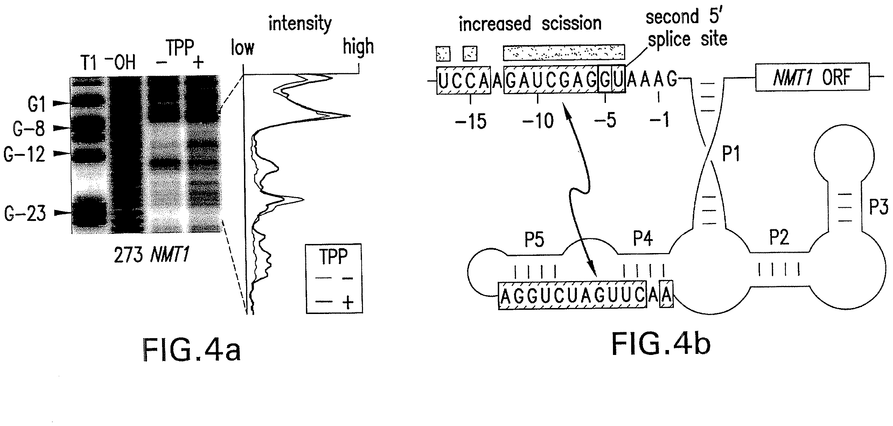

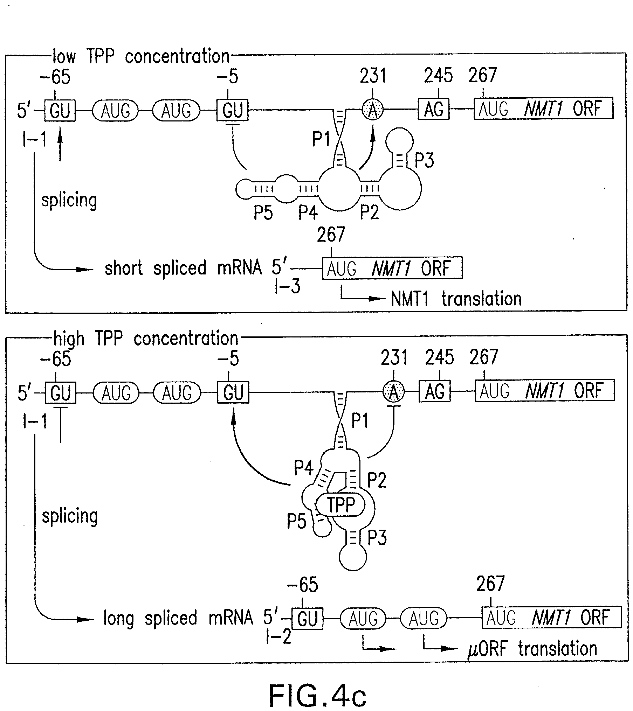

Figure 4 shows the mechanism of TPP riboswitch-mediated alternative splicing of mRNA in *N. crassa.* a, TPP-induced modulation of structures near the second 5' splice site. Spontaneous cleavage products of 5' ³²P-labeled 273 *NMT1* RNA (nts -78 through 195) were separated by PAGE and quantified to reveal locations of 10 µM TPP-mediated changes in structure. b, Some P4-P5 nucleotides are complementary to nucleotides near the second 5' splice site that are modulated by TPP (SEQ ID NOS: 3-4). c, Mechanism for riboswitch control *of NMT1* expression where key splicing determinants are activated or inhibited during different occupancy states of the aptamer.

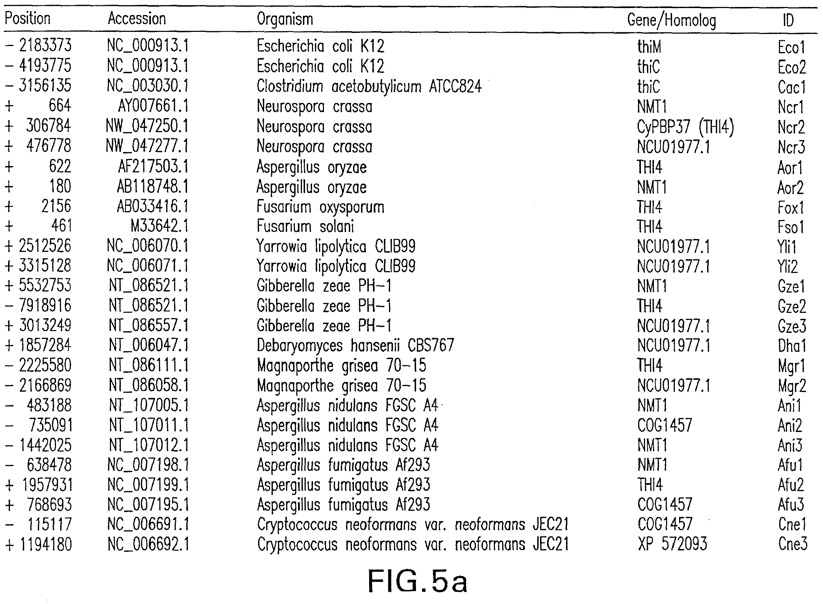

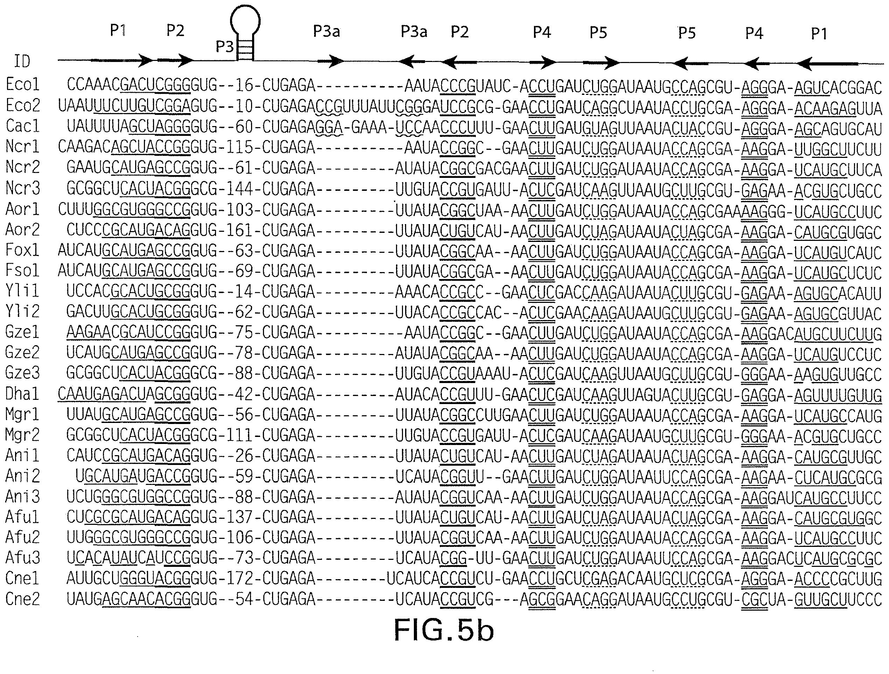

Figure 5 shows sequence alignments for three bacterial and 23 fungal TPP riboswitch aptamers (SEQ ID NOS: 5-30). Highlighted regions correspond to stem partners indicated by arrow diagram at top.

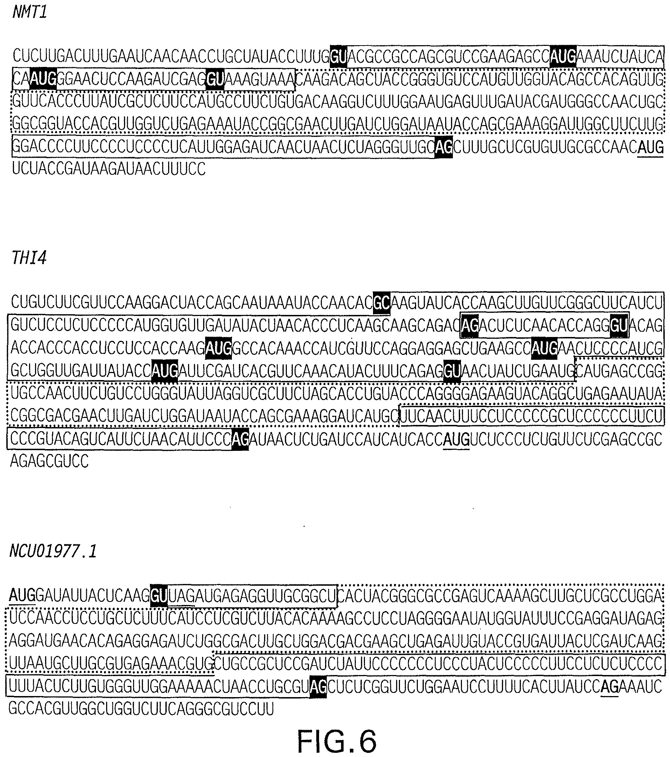

Figure 6 shows sequence contexts of three TPP riboswitches from N. crassa. AUG represents the main start codon; 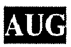 identifies alternative start codons (not in main ORF); 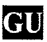 and 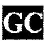 designate 5' splice sites; and 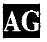 designate 3' splice sites. For each gene, the shaded nucleotides identify an intron and the dark-shaded region of the intron identifies the TPP aptamer (SEQ ID NOS: 31-33). **AG** identifies a predicted 3' splice site for NCU01977.1 present in the database that is not used based on sequencing spliced products. UAG identifies a stop codon in NCU01977.1 that would terminate translation unless splicing occurs.

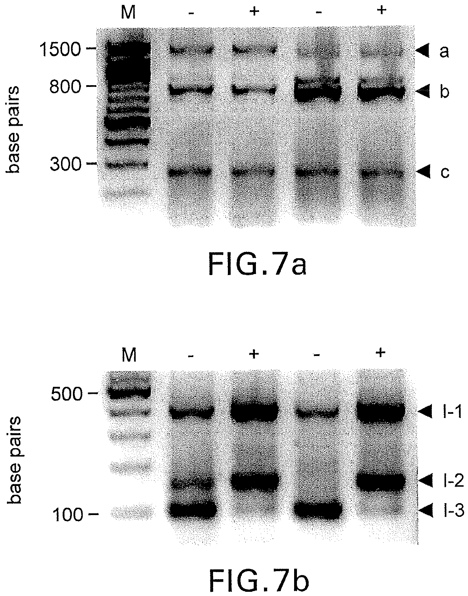

Figure 7 shows thiamine dependent changes in splicing require TPP riboswitches. RT-PCR analyses of alternatively spliced 5' regions of a, *FREQUENCY* (*FRQ*) and b, *NMT1* transcripts from *Neurospora* grown in minimal medium in the absence (-) or presence (+) of 30 µM thiamine. Two replicates for each mRNA are depicted. The different splice forms of *FRQ* (a, b and c) as reported previously, do not change in number relative to each other after thiamine treatment. RT-PCR products for *FRQ* were generated using primers 5'-CATTGCAAAAACGGCATTGGA (SEQ ID NO: 34) and 5'-TGTGGGGACTTTTCATGATAC (SEQ ID NO: 35). Products were separated using agarose gel (2%) electrophoresis. "M" designates the size marker containing DNAs of 100 base pair increments. DNAs were visualized by ethidium bromide staining and UV illumination.

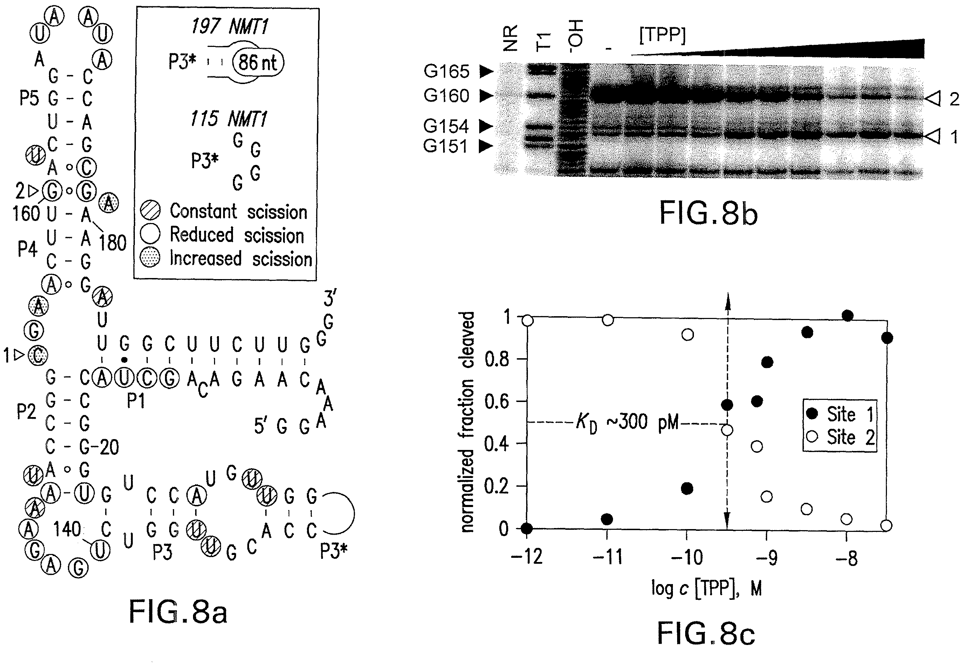

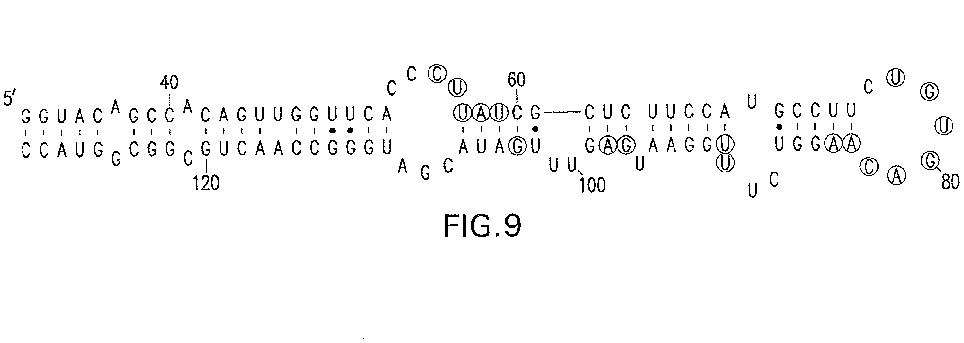

Figure 8 shows TPP binding to *NMT1* mRNA. a, Sequence, secondary structure, and TPP-induced modulation of the *NMT1* riboswitch aptamer (SEQ ID NO: 36, representing the 115 *NMT1* aptamer). The model was modified from those presented previously to reflect available atomic-resolution structural data. Construct 197 *NMT1* includes the natural P3 stem (Fig. 9) whereas 82 nts of this stem are deleted in construct 115 *NMT1.* Sites of structural modulation were established by in-line probing of 115 *NMT1* depicted in b. Constant scission was found for nucleotides 30, 31, 43, 44, 54, 56, 71 and 93 of SEQ ID NO:36. Reduced scission was found for nucleotides 13-16, 22, 27, 49-53, 55, 63, 65, 69, 77-81, 86 and 87 of SEQ ID NO:36. Increased scission was found for nucleotides 62, 64 and 88 of SEQ ID NO:36. b, In-line probing analysis of 115 *NMT1* reveals TPP-induced RNA structure modulations of which sites 1 and 2 were used to quantify ligand affinity in c. Lanes include precursor RNAs loaded after no reaction (NR), after partial digestion with RNase T1 (T1) or after partial digestion with alkali (-OH). A U2G change was made to facilitate preparation by *in vitro* transcription. c, Plot depicting the normalized fraction of RNA spontaneously cleaved versus the logarithm of the concentration of TPP for sites 1 and 2 as depicted in b.
Figure 9 shows sequence and in-line probing results for the extended P3 stem of construct 197 *NMT1.* Shaded nucleotides represent positions within the RNA that undergo spontaneous cleavage both in the absence and presence of up to 1 mM TPP (SEQ ID NO: 37).

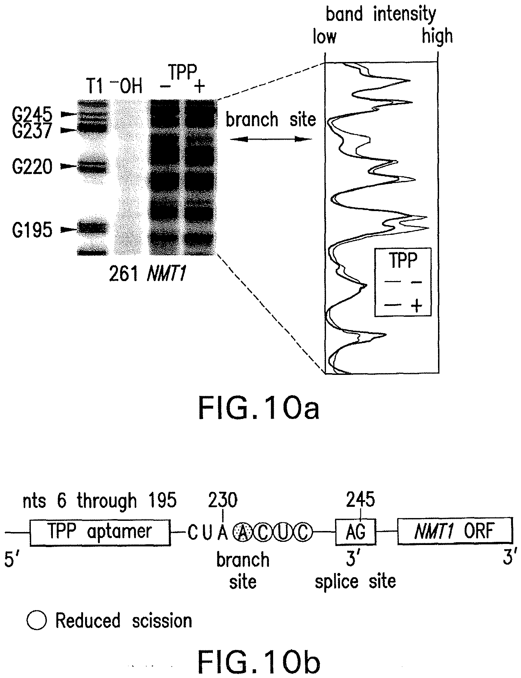

Figure 10 shows in-line probing analysis of the 261 *NMT1* RNA construct reveals modest structural changes at the branch site. a, PAGE separation of spontaneous RNA cleavage products from an in-line probing assay conducted using 5' ³²P-labeled 261 *NMT1* RNA (nts 11 through 270 plus an additional 5' G to facilitate *in vitro* transcription). Band intensities were quantified to reveal locations of 10 µM TPP-mediated changes in RNA structure. See the legends to Fig. 4 and Fig. 8 for additional details.

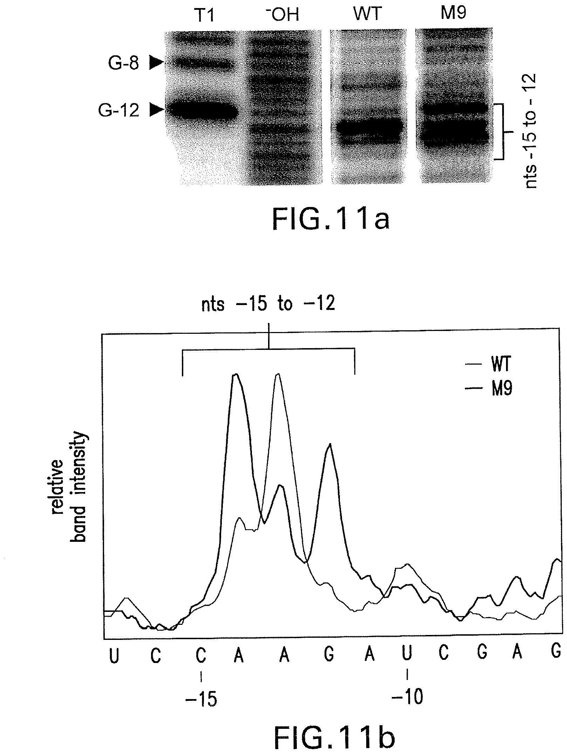

Figure 11 shows in-line probing analysis of the 273 *NMT1* wild-type (WT) and M9 *NMT1* RNA constructs in the absence of TPP reveal differences at the second 5' splice site. a, PAGE separation of spontaneous RNA cleavage products from an in-line probing assay conducted using 5' ³²P-labeled WT and M9 273 *NMT1* RNAs (nts -78 through 195) as indicated. b, Relative band intensities were determined to reveal locations of structural changes caused by the introduction of mutations in M9 relative to WT. See the legends to Fig. 8 and 9 for additional details. Note that nucleotide -14, -13, and -12 exhibit substantial spontaneous cleavage with M9 and can be be unpaired relative to the WT construct, where only nucleotide -13 is expected to be unpaired in the absence of TPP.

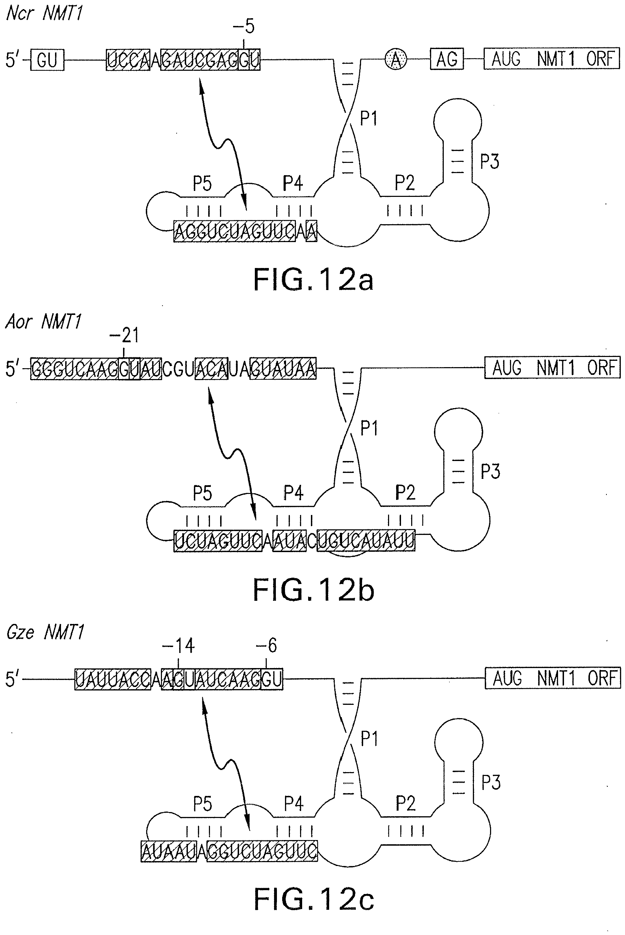

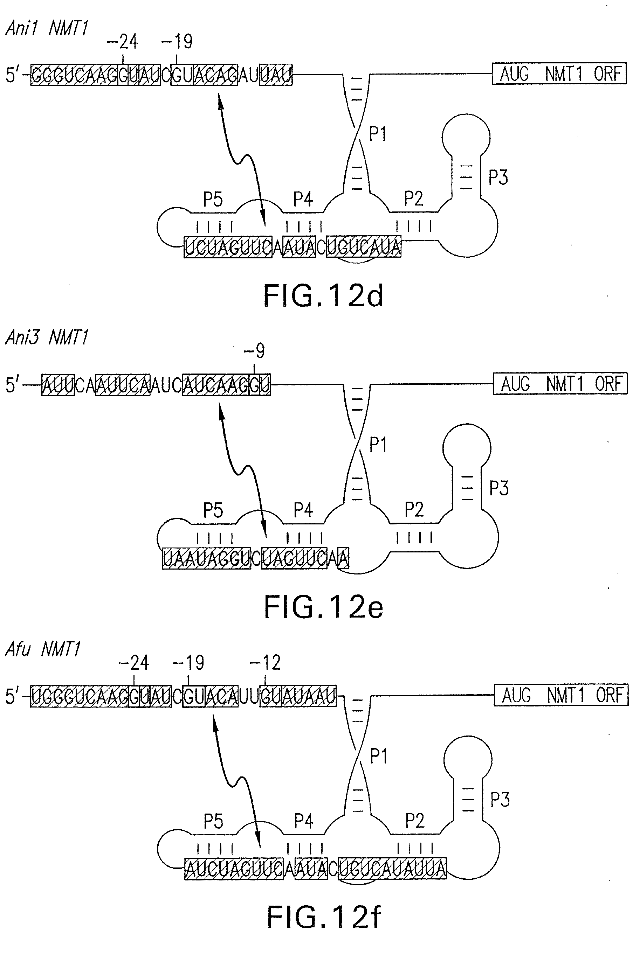

Figure 12 shows alternative base pairing between the flanking region of the second 5' splice site and the TPP aptamer *of NMT1* genes from various fungal species (SEQ ID NOS: 38-43 and 96-101). a, Schematic representation of the *NMT1* gene from *Neurospora crassa.* The two alternative 5' splice sites, branch point and 3' splice site are shown relative to the positions of the TPP aptamer and the main ORF. Nucleotides shaded orange identify base pairing potential between the sequence flanking the second 5' splice site and a homologous region in the aptamer. b-f, Complementary sequences of the region surrounding putative 5' splice sites next to the aptamer and parts of the aptamer for *NMT1* genes from different fungal species. The region of the aptamer with alternative base pairing potential is mainly located in one side of the P4 and P5 stem, but in some cases can also extend to loop 5 and stem P2. The putative alternative 5' splice sites are located at positions between -6 to -24 relative to the 5' end of the aptamer. The use of the indicated splice sites is confirmed by transcript data for the *NMT1* genes from *Neurospora* (see Fig. 1) and *Aspergillus oryzae* (b, AB226284). For some genes, several potential 5' splice sites following the consensus sequence "GUA" are found in the complementary region.

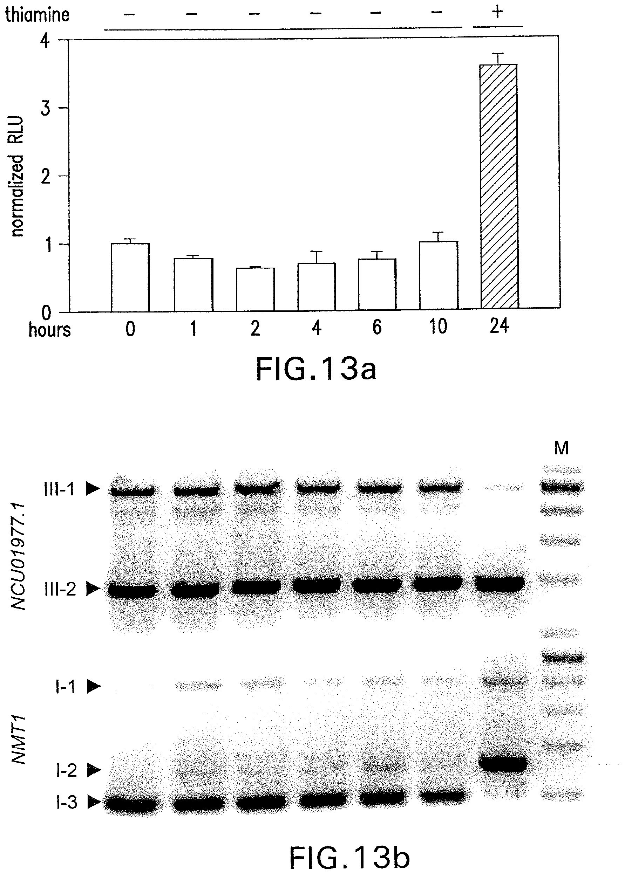

Figure 13 shows gene activation by the TPP riboswitch in the *N. crassa NCU01977.1* mRNA. LUC activity (a) and RT-PCR analysis of the riboswitch regions of native *NCU01977.1* and *NMT1* transcripts (b) in the absence or presence of 30 µM thiamine in *N. crassa* growth medium. *N. crassa* was transformed with a construct containing the 5' portion *of NCU01977.1* including the start codon fused in frame with the *LUC* reporter gene and was grown overnight in the absence (-) of thiamine. Fungal tissue was then transferred into fresh medium without (0 h) or with 30 µM thiamine and grown for further 24 h. Samples were taken at several time points from the culture grown in the absence of thiamine (-) and 24 h after addition of thiamine (+). *LUC* activity was normalized to the value measured at t = 0 h. RT-PCR analysis was performed on the native transcripts. M indicates the size marker of 100 bp increments with the bottom band representing 100 bp. The presence of substantial amounts of spliced product III-2 in the absence of added thiamine might indicate that the cell makes sufficient quantities of TPP under these conditions to mostly trigger riboswitch-induced splicing.

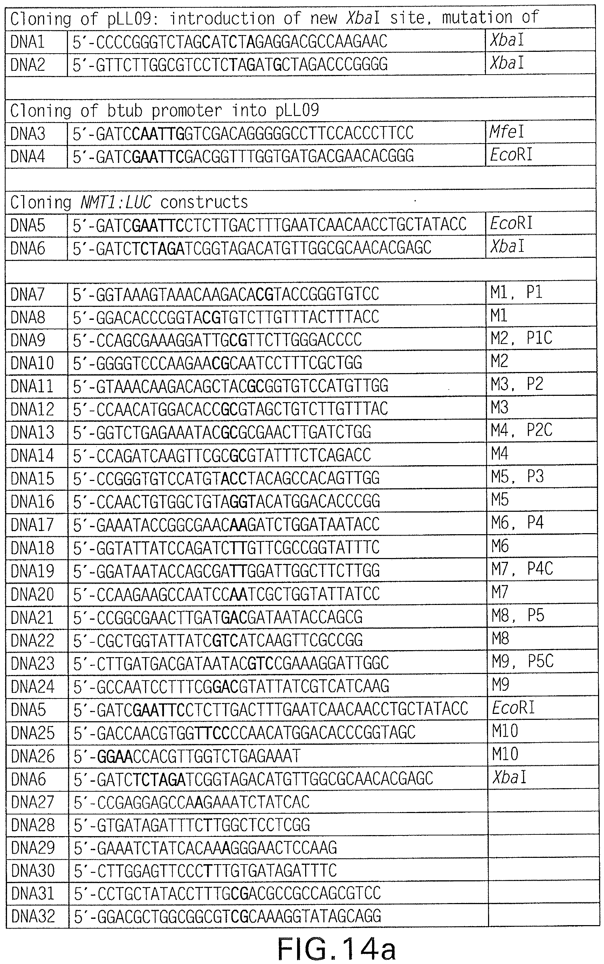

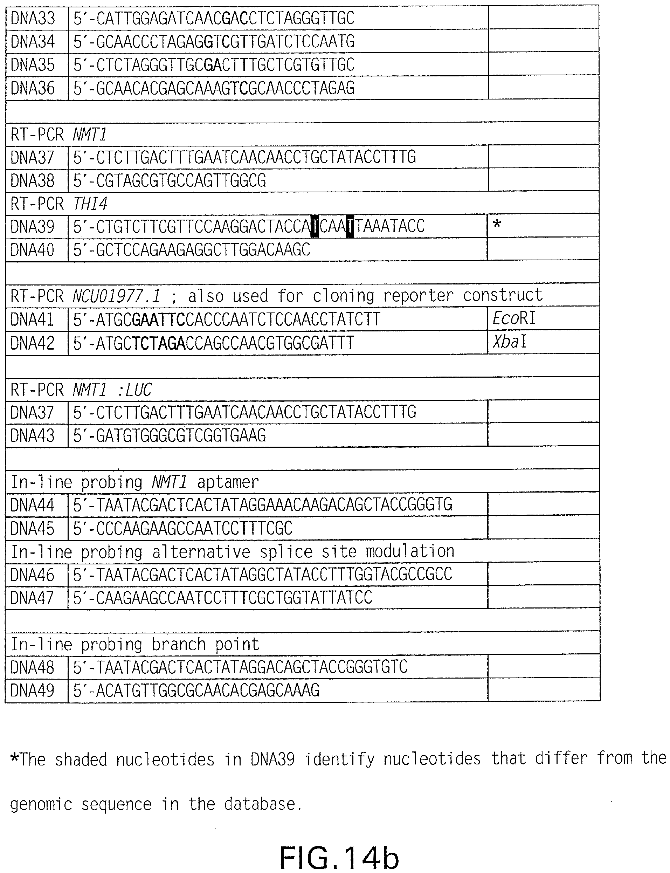

Figure 14 shows sequences of DNA primers (SEQ ID NOS: 44-95).

### DETAILED DESCRIPTION OF THE INVENTION

The disclosed methods and compositions can be understood more readily by reference to the following detailed description of particular embodiments and the Examples included therein and to the Figures and their previous and following description.

Messenger RNAs are typically thought of as passive carriers of genetic information that are acted upon by protein- or small RNA-regulatory factors and by ribosomes during the process of translation. It was discovered that certain mRNAs carry natural aptamer domains and that binding of specific metabolites directly to these RNA domains leads to modulation of gene expression. Natural riboswitches exhibit two surprising functions that are not typically associated with natural RNAs. First, the mRNA element can adopt distinct structural states wherein one structure serves as a precise binding pocket for its target metabolite. Second, the metabolite-induced allosteric interconversion between structural states causes a change in the level of gene expression by one of several distinct mechanisms. Riboswitches typically can be dissected into two separate domains: one that selectively binds the target (aptamer domain) and another that influences genetic control (expression platform). It is the dynamic interplay between these two domains that results in metabolite-dependent allosteric control of gene expression.

Distinct classes of riboswitches have been identified and are shown to selectively recognize activating compounds (referred to herein as trigger molecules). For example, coenzyme B₁₂, glycine, thiamine pyrophosphate (TPP), and flavin mononucleotide (FMN) activate riboswitches present in genes encoding key enzymes in metabolic or transport pathways of these compounds. The aptamer domain of each riboswitch class conforms to a highly conserved consensus sequence and structure. Thus, sequence homology searches can be used to identify related riboswitch domains. Riboswitch domains have been discovered in various organisms from bacteria, archaea, and eukarya.

Eleven structural classes of riboswitches have been reported in eubacteria that sense 10 different metabolites (Mandal 2004; Winkler 2005; Breaker 2006; Fuchs 2006; Roth). A eubacterial riboswitch selective for the queuosine precursor preQ₁ contains an unusually small aptamer domain. Nat. Struct. Mol. Biol. (2007), and numerous other classes are currently being characterized. The aptamer domain of each riboswitch is distinguished by its nucleotide sequence (Rodionov 2002; Vitreschak 2002; Vitreschak 2003) and folded structure (Nahvi 2004; Batey 2004; Serganov 2004; Montange 2006; Thore 2006; Serganov 2006; Edwards 2006) which remain highly conserved even between distantly related organisms. Riboswitches usually include an expression platform that modulates gene expression in response to metabolite binding by the aptamer, although expression platforms can differ extensively in sequence, structure, and control mechanism.

The exceptional level of aptamer conservation enables the use of bioinformatics to identify similar riboswitch representatives in diverse organisms. Currently, only sequences that conform to the TPP riboswitch aptamer consensus have been identified in organisms from all three domains of life (Sudarsan 2003). Although some predicted eukaryotic TPP aptamers from fungi (Sudarsan 2003; Galagan 2005) (Fig. 5) and plants were shown to bind TPP (Sudarsan 2003Yamauchi), the precise mechanisms by which metabolite binding controls gene expression were unknown. In fungi, each TPP aptamer resides within an intron in the 5' untranslated region (UTR) or the protein coding region of an mRNA, implying that mRNA splicing is controlled by metabolite binding (Sudarsan 2003; Kubodera 2003).

### A. General Organization of Riboswitch RNAs

Bacterial riboswitch RNAs are genetic control elements that are located primarily within the 5'-untranslated region (5'-UTR) of the main coding region of a particular mRNA. Structural probing studies (discussed further below) reveal that riboswitch elements are generally composed of two domains: a natural aptamer (T. Hermann, D. J. Patel, Science 2000, 287, 820; L. Gold, et al., Annual Review of Biochemistry 1995, 64, 763) that serves as the ligand-binding domain, and an 'expression platform' that interfaces with RNA elements that are involved in gene expression (e.g. Shine-Dalgarno (SD) elements; transcription terminator stems). These conclusions are drawn from the observation that aptamer domains synthesized *in vitro* bind the appropriate ligand in the absence of the expression platform (see Examples 2, 3 and 6 of U.S. Application Publication No. 2005-0053951). Moreover, structural probing investigations suggest that the aptamer domain of most riboswitches adopts a particular secondary- and tertiary-structure fold when examined independently, that is essentially identical to the aptamer structure when examined in the context of the entire 5' leader RNA. This indicates that, in many cases, the aptamer domain is a modular unit that folds independently of the expression platform (see Examples 2, 3 and 6 of U.S. Application Publication No. 2005-0053951).

Ultimately, the ligand-bound or unbound status of the aptamer domain is interpreted through the expression platform, which is responsible for exerting an influence upon gene expression. The view of a riboswitch as a modular element is further supported by the fact that aptamer domains are highly conserved amongst various organisms (and even between kingdoms as is observed for the TPP riboswitch), (N. Sudarsan, et al., RNA 2003, 9, 644) whereas the expression platform varies in sequence, structure, and in the mechanism by which expression of the appended open reading frame is controlled. For example, ligand binding to the TPP riboswitch of the *tenA* mRNA *of B. subtilis* causes transcription termination (A. S. Mironov, et al., Cell 2002, 111, 747). This expression platform is distinct in sequence and structure compared to the expression platform of the TPP riboswitch in the *thiM* mRNA from *E. coli,* wherein TPP binding causes inhibition of translation by a SD blocking mechanism (see Example 2 of U.S. Application Publication No. 2005-0053951). The TPP aptamer domain is easily recognizable and of near identical functional character between these two transcriptional units, but the genetic control mechanisms and the expression platforms that carry them out are very different.

Aptamer domains for riboswitch RNAs typically range from ∼70 to 170 nt in length (Figure 11 of U.S. Application Publication No. 2005-0053951). This observation was somewhat unexpected given that *in vitro* evolution experiments identified a wide variety of small molecule-binding aptamers, which are considerably shorter in length and structural intricacy (T. Hermann, D. J. Patel, Science 2000, 287, 820; L. Gold, et al., Annual Review of Biochemistry 1995, 64, 763; M. Famulok, Current Opinion in Structural Biology 1999, 9, 324). Although the reasons for the substantial increase in complexity and information content of the natural aptamer sequences relative to artificial aptamers remains to be proven, this complexity is believed required to form RNA receptors that function with high affinity and selectivity. Apparent *K*_{D} values for the ligand-riboswitch complexes range from low nanomolar to low micromolar. It is also worth noting that some aptamer domains, when isolated from the appended expression platform, exhibit improved affinity for the target ligand over that of the intact riboswitch. (∼10 to 100-fold) (see Example 2 of U.S. Application Publication No. 2005-0053951). Presumably, there is an energetic cost in sampling the multiple distinct RNA conformations required by a fully intact riboswitch RNA, which is reflected by a loss in ligand affinity. Since the aptamer domain must serve as a molecular switch, this might also add to the functional demands on natural aptamers that might help rationalize their more sophisticated structures.

### B. The TPP Riboswitch

The coenzyme thiamine pyrophosphate (TPP) is an active form of vitamin B1, an essential participant in many protein-catalysed reactions. Organisms from all three domains of life, including bacteria, plants and fungi, use TPP-sensing riboswitches to control genes responsible for importing or synthesizing thiamine and its phosphorylated derivatives, making this riboswitch class the most widely distributed member of the metabolite-sensing RNA regulatory system. The structure reveals a folded RNA in which one subdomain forms an intercalation pocket for the 4-amino-5-hydroxymethyl-2-methylpyrimidine moiety of TPP, whereas another subdomain forms a wider pocket that uses bivalent metal ions and water molecules to make bridging contacts to the pyrophosphate moiety of the ligand. The two pockets are positioned to function as a molecular measuring device that recognizes TPP in an extended conformation. The central thiazole moiety is not recognized by the RNA, which explains why the antimicrobial compound pyrithiamine pyrophosphate targets this riboswitch and downregulates the expression of thiamine metabolic genes. Both the natural ligand and its drug-like analogue stabilize secondary and tertiary structure elements that are harnessed by the riboswitch to modulate the synthesis of the proteins coded by the mRNA. In addition, this structure provides insight into how folded RNAs can form precision binding pockets that rival those formed by protein genetic factors.

Three TPP riboswitches were examined in the filamentous fungus *Neurospora crassa,* and it was found that one activates and two repress gene expression by controlling mRNA splicing. A detailed mechanism involving riboswitch-mediated base-pairing changes and alternative splicing control was elucidated for precursor *NMT1* mRNAs, which code for a protein involved in TPP metabolism (Example 1). These results demonstrate that eukaryotic cells employ metabolite-binding RNAs to regulate RNA splicing events important for the control of key biochemical processes. TPP riboswitches are also described in U.S. Patent Application Publication No. US-2005-0053951, which is incorporated herein in its entirety and also in particular is incorpoarated by reference for its description of TTP riboswitch structure, function and use. It is specifically contemplated that any of the subject matter and description of U.S. Patent Application Publication No. US-2005-0053951, and in particular any description of TTP riboswitch structure, function and use in U.S. Patent Application Publication No. US-2005-0053951 can be specifically included or excluded from the other subject matter disclosed herein.

It is to be understood that the disclosed method and compositions are not limited to specific synthetic methods, specific analytical techniques, or to particular reagents unless otherwise specified, and, as such, can vary. It is also to be understood that the terminology used herein is for the purpose of describing particular embodiments only and is not intended to be limiting.

### Materials

Disclosed are materials, compositions, and components that can be used for, can be used in conjunction with, can be used in preparation for, or are products of the disclosed methods and compositions. These and other materials are disclosed herein, and it is understood that when combinations, subsets, interactions, groups, etc. of these materials are disclosed that while specific reference to each of various individual and collective combinations and permutation of these compounds can not be explicitly disclosed, each is specifically contemplated and described herein. For example, if a riboswitch or aptamer domain is disclosed and discussed and a number of modifications that can be made to a number of molecules including the riboswitch or aptamer domain are discussed, each and every combination and permutation of riboswitch or aptamer domain and the modifications that are possible are specifically contemplated unless specifically indicated to the contrary. Thus, if a class of molecules A, B, and C are disclosed as well as a class of molecules D, E, and F and an example of a combination molecule, A-D is disclosed, then even if each is not individually recited, each is individually and collectively contemplated. Thus, in this example, each of the combinations A-E, A-F, B-D, B-E, B-F, C-D, C-E, and C-F are specifically contemplated and should be considered disclosed from disclosure of A, B, and C; D, E, and F; and the example combination A-D. Likewise, any subset or combination of these is also specifically contemplated and disclosed. Thus, for example, the sub-group of A-E, B-F, and C-E are specifically contemplated and should be considered disclosed from disclosure of A, B, and C; D, E, and F; and the example combination A-D. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods, and that each such combination is specifically contemplated and should be considered disclosed.

### A. Riboswitches

Riboswitches are expression control elements that are part of an RNA molecule to be expressed and that change state when bound by a trigger molecule. Riboswitches typically can be dissected into two separate domains: one that selectively binds the target (aptamer domain) and another that influences genetic control (expression platform domain). It is the dynamic interplay between these two domains that results in metabolite-dependent allosteric control of gene expression. Disclosed are isolated and recombinant riboswitches, recombinant constructs containing such riboswitches, heterologous sequences operably linked to such riboswitches, and cells and transgenic organisms harboring such riboswitches, riboswitch recombinant constructs, and riboswitches operably linked to heterologous sequences. The heterologous sequences can be, for example, sequences encoding proteins or peptides of interest, including reporter proteins or peptides. Preferred riboswitches are, or are derived from, naturally occurring riboswitches. For example, the aptamer domain can be, or be derived from, the aptamer doamin of a naturally occurring riboswitch. The riboswitch can include or, optionlly, exclude, artificial aptamers. For example, artificial apatmers include apatamers that are designed or selected via in vitro evolution and/or in vitro selection. The riboswtiches can comprise the consensus sequence of naturally occurring riboswitches. Consensus sequences for a variety of riboswitches are described in U.S. Application Publication No. 2005-0053951, such as in Figure 11.

Disclosed herein is a regulatable gene expression construct comprising a nucleic acid molecule encoding an RNA comprising a riboswitch operably linked to a coding region, wherein the riboswitch regulates splicing of the RNA, wherein the riboswitch and coding region are heterologous. The riboswitch can regulate alternative spicing of the RNA. The riboswitch can comprise an aptamer domain and an expression platform domain, wherein the aptamer domain and the expression platform domain are heterologous. The RNA can further comprises an intron, wherein the expression platform domain comprises an alternative splice junction in the intron or at the end of the intron (that is, the 5' splice junction or the 3' splice junction). The RNA can further comprises an intron, wherein the expression platform domain comprises the branch site in the intron. The alternative splice junction can be active when the riboswitch is activated, or not activated. The riboswitch can be activated by a trigger molecule, such as thiamine pyrophosphate (TPP). The riboswitch can be a TPP-responsive riboswitch.

The riboswitch can alter splicing of the RNA. For example, activation of the riboswitch can allow or promote alternative splicing, prevent or reduce splicing or the predominate splicing, prevent or reduce alternative splicing, or allow or promote splicing or the predominate splicing. As other examples, a deactive ribowitch or deactivation of the riboswitch can allow or promote alternative splicing, prevent or reduce splicing or the predominate splicing, prevent or reduce alternative splicing, or allow or promote splicing or the predominate splicing. Generally, the form of splicing regulation can be determined by the physical relationship of the riboswitch to the splice junctions, alterantive splice junctions and branch sites in the RNA molecule. For example, activation/deactivation of riboswirches generally involves formation and/or disruption of alternative secondary structures (for exmaple, base paried stems) in RNA and this change in strucutre can be used to hide or expose functional RNA sequences. The expression platform domain of a riboswitch generally comprises such functional RNA sequences. Thus, for example, by including a slice junction or a branch site in the expression platform domain of a riboswitch in such a way that the spice junction or branch site is alternately hidden or exposed as the riboswitch is activated or deactivated, or vice versa, splicing of the RNA can be regulated or affected.

The riboswitch can activate or repress splicing. By "activate splicing" is meant that the riboswitch can either directly or indirectly act upon RNA to allow splicing to take place. This can include, for example, allowing any splicing to take place (such as a single splice versus no splice) or allowing alternative splicing to take place. This can increase splicing by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% or more compared to the number of splicing events that would have taken place without the riboswitch.

By "repress splicing" is meant that the riboswitch can either directly or indirectly act upon RNA to suppress splicing. This can include, for example, preventing any splicing or reducing splicing from taking place (such as no splice versus a single splice) or preventing or reducing alternative splicing from taking place. This can decrease alternative splicing by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, b5, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% compared to the number of alternative splicing events that would have taken place without the riboswitch.

The riboswitch can activate or repress alternative splicing. By "activate alternative splicing" is meant that the riboswitch can either directly or indirectly act upon RNA to allow alternative splicing to take place. This can increase alternative splicing by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% or more compared to the number of alternative splicing events that would have taken place without the riboswitch.

By "repress alternative splicing" is meant that the riboswitch can either directly or indirectly act upon RNA to suppress alternative splicing. This can decrease alternative splicing by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% compared to the number of alternative splicing events that would have taken place without the riboswitch.

The riboswitch can affect expression of a protein encoded by the RNA. For example, regulation of splicing or alternative splicing can affect the ability of the RNA t be translated, alter the coding region, or alter the translation initiation or termination. Alternative splicing can, for example, cause a start or stop codon (or both) to appear in the processed transcript that is not present in normally processed transcripts. As another example, alternative splicing can cause the normal start or stop codon to be removed from the processed transcript. A useful mode for using riboswitch-regulated splicing to regulate expression of a protein encoded by an RNA is to introduce a riboswitch in an intron in the 5' untranslated region of the RNA and include or make use of a start codon in the intron such that the start codon in the intron will be the first start codon in the alternatively spliced RNA. Another useful mode for using riboswitch-regulated splicing to regulate expression of a protein encoded by an RNA is to introduce a riboswitch in an intron in the 5' untranslated region of the RNA and include or make use of a short open reading frame in the intron such that the reading frame will appear first in the alternatively spliced RNA.

The RNA molecule can have a branched structure. For example, in the TPP riboswitch (Example 1), when TPP concentration is low, the newly transcribed mRNA adopts a structure that occludes the second 5' splice site, while leaving the branch site available for splicing. Pre-mRNA splicing from the first 5' splice site leads to production of the I-3 form of mRNA and expression of the NMT1 protein. When TPP concentration is high, ligand binding to the TPP aptamer causes allosteric changes in RNA folding to increase the structural flexibility near the second 5' splice site and to occlude nucleotides near the branch site.

The region of the aptamer with splicing control can be located in, for example, the P4 and P5 stem. The region of the aptamer with alternative splicing control can also found, for example, in loop 5, and in stem P2. The splice sites can be located, for example, at positions between -6 to -24 relative to the 5' end of the aptamer. Thus, for example, an expression platform domain can interact with the P4 and P5 sequences, the loop 5 sequence and/or the P2 sequences. Such aptamer sequences generally can be available for interaction with the expression platform domain only when a trigger molecule is not bound to the aptamer domain. The splice sites can follow the sequence GUA. Example 1 discusses the specific locations of the aptamer on the riboswitch.

For many bacterial riboswitches, metabolite binding alters folding of the expression platform located downstream of the aptamer without involving proteins (Winkler et al. (2002), Mironov et al. (2002), Serganov et al. (2006)). To assess this in a TPP riboswitch that regulates alternative splicing, it was tested to determine if splicing regulation by the NMT1 TPP riboswitch is due to protein-independent structural modulation of the aptamer flanks. NMT1 UTR constructs were subjected to in-line probing (Soukup & Breaker (1999)). Interestingly, the addition of TPP causes nucleotides at the branch site to become more structured (Figure 10), and yields a more flexible structure at the second 5' splice site (Fig. 4a). Furthermore, it was observed that 12 nucleotides of the P4 and P5 elements of the aptamer are complementary to most of the nucleotides at the second 5' splice site that are structurally sequestered when ligand is absent (Fig. 4b). The P4 and P5 elements are required for recognition of the pyrophosphate moiety of TPP and, therefore, TPP binding and 5' splice site occlusion are mutually exclusive (Example 1).

The disclosed riboswitches, including the derivatives and recombinant forms thereof, generally can be from any source, including naturally occurring riboswitches and riboswitches designed de novo. Any such riboswitches, as long as they have been determined to regulate alternative splicing, can be used in or with the disclosed methods. However, different types of riboswitches can be defined and some such sub-types can be useful in or with particular methods (generally as described elsewhere herein). Types of riboswitches include, for example, naturally occurring riboswitches, derivatives and modified forms of naturally occurring riboswitches, chimeric riboswitches, and recombinant riboswitches. A naturally occurring riboswitch is a riboswitch having the sequence of a riboswitch as found in nature. Such a naturally occurring riboswitch can be an isolated or recombinant form of the naturally occurring riboswitch as it occurs in nature. That is, the riboswitch has the same primary structure but has been isolated or engineered in a new genetic or nucleic acid context. Chimeric riboswitches can be made up of, for example, part of a riboswitch of any or of a particular class or type of riboswitch and part of a different riboswitch of the same or of any different class or type of riboswitch; part of a riboswitch of any or of a particular class or type of riboswitch and any non-riboswitch sequence or component. Recombinant riboswitches are riboswitches that have been isolated or engineered in a new genetic or nucleic acid context.

Riboswitches can have single or multiple aptamer domains. Aptamer domains in riboswitches having multiple aptamer domains can exhibit cooperative binding of trigger molecules or can not exhibit cooperative binding of trigger molecules (that is, the aptamers need not exhibit cooperative binding). In the latter case, the aptamer domains can be said to be independent binders. Riboswitches having multiple aptamers can have one or multiple expression platform domains. For example, a riboswitch having two aptamer domains that exhibit cooperative binding of their trigger molecules can be linked to a single expression platform domain that is regulated by both aptamer domains. Riboswitches having multiple aptamers can have one or more of the aptamers joined via a linker. Where such aptamers exhibit cooperative binding of trigger molecules, the linker can be a cooperative linker.

Aptamer domains can be said to exhibit cooperative binding if they have a Hill coefficient n between x and x-1, where x is the number of aptamer domains (or the number of binding sites on the aptamer domains) that are being analyzed for cooperative binding. Thus, for example, a riboswitch having two aptamer domains (such as glycine-responsive riboswitches) can be said to exhibit cooperative binding if the riboswitch has Hill coefficient between 2 and 1. It should be understood that the value of x used depends on the number of aptamer domains being analyzed for cooperative binding, not necessarily the number of aptamer domains present in the riboswitch. This makes sense because a riboswitch can have multiple aptamer domains where only some exhibit cooperative binding.

Disclosed are chimeric riboswitches containing heterologous aptamer domains and expression platform domains. That is, chimeric riboswitches are made up an aptamer domain from one source and an expression platform domain from another source. The heterologous sources can be from, for example, different specific riboswitches, different types of riboswitches, or different classes of riboswitches. The heterologous aptamers can also come from non-riboswitch aptamers. The heterologous expression platform domains can also come from non-riboswitch sources.

Modified or derivative riboswitches can be produced using *in vitro* selection and evolution techniques. In general, *in vitro* evolution techniques as applied to riboswitches involve producing a set of variant riboswitches where part(s) of the riboswitch sequence is varied while other parts of the riboswitch are held constant. Activation, deactivation or blocking (or other functional or structural criteria) of the set of variant riboswitches can then be assessed and those variant riboswitches meeting the criteria of interest are selected for use or further rounds of evolution. Useful base riboswitches for generation of variants are the specific and consensus riboswitches disclosed herein. Consensus riboswitches can be used to inform which part(s) of a riboswitch to vary for *in vitro* selection and evolution.

Also disclosed are modified riboswitches with altered regulation. The regulation of a riboswitch can be altered by operably linking an aptamer domain to the expression platform domain of the riboswitch (which is a chimeric riboswitch). The aptamer domain can then mediate regulation of the riboswitch through the action of, for example, a trigger molecule for the aptamer domain. Aptamer domains can be operably linked to expression platform domains of riboswitches in any suitable manner, including, for example, by replacing the normal or natural aptamer domain of the riboswitch with the new aptamer domain. Generally, any compound or condition that can activate, deactivate or block the riboswitch from which the aptamer domain is derived can be used to activate, deactivate or block the chimeric riboswitch.

Also disclosed are inactivated riboswitches. Riboswitches can be inactivated by covalently altering the riboswitch (by, for example, crosslinking parts of the riboswitch or coupling a compound to the riboswitch). Inactivation of a riboswitch in this manner can result from, for example, an alteration that prevents the trigger molecule for the riboswitch from binding, that prevents the change in state of the riboswitch upon binding of the trigger molecule, or that prevents the expression platform domain of the riboswitch from affecting expression upon binding of the trigger molecule.

Also disclosed are biosensor riboswitches. Biosensor riboswitches are engineered riboswitches that produce a detectable signal in the presence of their cognate trigger molecule. Useful biosensor riboswitches can be triggered at or above threshold levels of the trigger molecules. Biosensor riboswitches can be designed for use *in vivo or in vitro.* For example, biosensor riboswitches operably linked to a reporter RNA that encodes a protein that serves as or is involved in producing a signal can be used *in vivo* by engineering a cell or organism to harbor a nucleic acid construct encoding the riboswitch/reporter RNA. An example of a biosensor riboswitch for use *in vitro* is a riboswitch that includes a conformation dependent label, the signal from which changes depending on the activation state of the riboswitch. Such a biosensor riboswitch preferably uses an aptamer domain from or derived from a naturally occurring riboswitch. Biosensor riboswitches can be used in various situations and platforms. For example, biosensor riboswitches can be used with solid supports, such as plates, chips, strips and wells.

Also disclosed are modified or derivative riboswitches that recognize new trigger molecules. New riboswitches and/or new aptamers that recognize new trigger molecules can be selected for, designed or derived from known riboswitches. This can be accomplished by, for example, producing a set of aptamer variants in a riboswitch, assessing the activation of the variant riboswitches in the presence of a compound of interest, selecting variant riboswitches that were activated (or, for example, the riboswitches that were the most highly or the most selectively activated), and repeating these steps until a variant riboswitch of a desired activity, specificity, combination of activity and specificity, or other combination of properties results.

In general, any aptamer domain can be adapted for use with any expression platform domain by designing or adapting a regulated strand in the expression platform domain to be complementary to the control strand of the aptamer domain. Alternatively, the sequence of the aptamer and control strands of an aptamer domain can be adapted so that the control strand is complementary to a functionally significant sequence in an expression platform.

Disclosed are RNA molecules comprising heterologous riboswitch and coding regions. That is, such RNA molecules are made up of a riboswitch from one source and a coding region from another source. The heterologous sources can be from, for example, different RNA molecules, different transcripts, RNA or transcripts from different genes, RNA or transcripts from different cells, RNA or transcripts from different organisms, RNA or transcripts from different species, natural sequences and artificial or engineered sequences, specific riboswitches, different types of riboswitches, or different classes of riboswitches.

As disclosed herein, the term "coding region" refers to any region of a nucleic acid that codes for amino acids. This can include both a nucleic acid strand that contains the codons or the template for codons and the complement of such a nucleic acid strand in the case of double stranded nuclec acid molecules. Regions of nucleic acids that are not coding regions can be referred to as noncoding regions. Messenger RNA molecules as transcribed typically include noncoding regions at both the 5' and 3' ends. Eucaryotic mRNA molecules can also include internal noncoding regions such as introns. Some types of RNA molecules do not include functional coding regions, such as tRNA and rRNA molecules.

### 1. Aptamer Domains

Aptamers are nucleic acid segments and structures that can bind selectively to particular compounds and classes of compounds. Riboswitches have aptamer domains that, upon binding of a trigger molecule result in a change in the state or structure of the riboswitch. In functional riboswitches, the state or structure of the expression platform domain linked to the aptamer domain changes when the trigger molecule binds to the aptamer domain. Aptamer domains of riboswitches can be derived from any source, including, for example, natural aptamer domains of riboswitches, artificial aptamers, engineered, selected, evolved or derived aptamers or aptamer domains. Aptamers in riboswitches generally have at least one portion that can interact, such as by forming a stem structure, with a portion of the linked expression platform domain. This stem structure will either form or be disrupted upon binding of the trigger molecule.

Consensus aptamer domains of a variety of natural riboswitches are shown in Figure 11 of U.S. Application Publication No. 2005-0053951 and elsewhere herein. These aptamer domains (including all of the direct variants embodied therein) can be used in riboswitches. The consensus sequences and structures indicate variations in sequence and structure. Aptamer domains that are within the indicated variations are referred to herein as direct variants. These aptamer domains can be modified to produce modified or variant aptamer domains. Conservative modifications include any change in base paired nucleotides such that the nucleotides in the pair remain complementary. Moderate modifications include changes in the length of stems or of loops (for which a length or length range is indicated) of less than or equal to 20% of the length range indicated. Loop and stem lengths are considered to be "indicated" where the consensus structure shows a stem or loop of a particular length or where a range of lengths is listed or depicted. Moderate modifications include changes in the length of stems or of loops (for which a length or length range is not indicated) of less than or equal to 40% of the length range indicated. Moderate modifications also include and functional variants of unspecified portions of the aptamer domain.

Aptamer domains of the disclosed riboswitches can also be used for any other purpose, and in any other context, as aptamers. For example, aptamers can be used to control ribozymes, other molecular switches, and any RNA molecule where a change in structure can affect function of the RNA.

### 2. Expression Platform Domains

Expression platform domains are a part of riboswitches that affect expression of the RNA molecule that contains the riboswitch. Expression platform domains generally have at least one portion that can interact, such as by forming a stem structure, with a portion of the linked aptamer domain. This stem structure will either form or be disrupted upon binding of the trigger molecule. The stem structure generally either is, or prevents formation of, an expression regulatory structure. An expression regulatory structure is a structure that allows, prevents, enhances or inhibits expression of an RNA molecule containing the structure. Examples include Shine-Dalgarno sequences, initiation codons, transcription terminators, and stability signals, and processing signals, such as RNA splicing junctions and control elements. For regulation of splicing, it is useful to include a splice junction, an alternative splice junction, and/or a branch site of an intron in the expression platform domain. Interaction of such platform expression domains with sequnces in the aptamer domain of a riboswitch can be mediated by complemenary sequences between the expression platform domain and the apatamer domain.

### B. Trigger Molecules

Trigger molecules are molecules and compounds that can activate a riboswitch. This includes the natural or normal trigger molecule for the riboswitch and other compounds that can activate the riboswitch. Natural or normal trigger molecules are the trigger molecule for a given riboswitch in nature or, in the case of some non-natural riboswitches, the trigger molecule for which the riboswitch was designed or with which the riboswitch was selected (as in, for example, *in vitro* selection or *in vitro* evolution techniques).

### C. Compounds

Also disclosed are compounds, and compositions containing such compounds, that can activate, deactivate or block a riboswitch. Riboswitches function to control gene expression through the binding or removal of a trigger molecule. Compounds can be used to activate, deactivate or block a riboswitch. The trigger molecule for a riboswitch (as well as other activating compounds) can be used to activate a riboswitch. Compounds other than the trigger molecule generally can be used to deactivate or block a riboswitch. Riboswitches can also be deactivated by, for example, removing trigger molecules from the presence of the riboswitch. A riboswitch can be blocked by, for example, binding of an analog of the trigger molecule that does not activate the riboswitch.

Also disclosed are compounds for altering expression of an RNA molecule, or of a gene encoding an RNA molecule, where the RNA molecule includes a riboswitch. This can be accomplished by bringing a compound into contact with the RNA molecule. Riboswitches function to control gene expression through the binding or removal of a trigger molecule. Thus, subjecting an RNA molecule of interest that includes a riboswitch to conditions that activate, deactivate or block the riboswitch can be used to alter expression of the RNA. Expression can be altered as a result of, for example, termination of transcription or blocking of ribosome binding to the RNA. Binding of a trigger molecule can, depending on the nature of the riboswitch, reduce or prevent expression of the RNA molecule or promote or increase expression of the RNA molecule. Also disclosed are compounds for regulating expression of an RNA molecule, or of a gene encoding an RNA molecule. Also disclosed are compounds for regulating expression of a naturally occurring gene or RNA that contains a riboswitch by activating, deactivating or blocking the riboswitch. If the gene is essential for survival of a cell or organism that harbors it, activating, deactivating or blocking the riboswitch can in death, stasis or debilitation of the cell or organism.

Also disclosed are compounds for regulating expression of an isolated, engineered or recombinant gene or RNA that contains a riboswitch by activating, deactivating or blocking the riboswitch. Since the riboswitches disclosed herein control alternative splicing, activating, deactivating, or blocking the riboswitch can regulate expression of a gene. An advantage of riboswitches as the primary control for such regulation is that riboswitch trigger molecules can be small, non-antigenic molecules.
Also disclosed are methods of identifying compounds that activate, deactivate or block a riboswitch. For examples, compounds that activate a riboswitch can be identified by bringing into contact a test compound and a riboswitch and assessing activation of the riboswitch. If the riboswitch is activated, the test compound is identified as a compound that activates the riboswitch. Activation of a riboswitch can be assessed in any suitable manner. For example, the riboswitch can be linked to a reporter RNA and expression, expression level, or change in expression level of the reporter RNA can be measured in the presence and absence of the test compound. As another example, the riboswitch can include a conformation dependent label, the signal from which changes depending on the activation state of the riboswitch. Such a riboswitch preferably uses an aptamer domain from or derived from a naturally occurring riboswitch. As can be seen, assessment of activation of a riboswitch can be performed with the use of a control assay or measurement or without the use of a control assay or measurement. Methods for identifying compounds that deactivate a riboswitch can be performed in analogous ways. Identification of compounds that block a riboswitch can be accomplished in any suitable manner. For example, an assay can be performed for assessing activation or deactivation of a riboswitch in the presence of a compound known to activate or deactivate the riboswitch and in the presence of a test compound. If activation or deactivation is not observed as would be observed in the absence of the test compound, then the test compound is identified as a compound that blocks activation or deactivation of the riboswitch.

Also disclosed are compounds made by identifying a compound that activates, deactivates or blocks a riboswitch and manufacturing the identified compound. This can be accomplished by, for example, combining compound identification methods as disclosed elsewhere herein with methods for manufacturing the identified compounds. For example, compounds can be made by bringing into contact a test compound and a riboswitch, assessing activation of the riboswitch, and, if the riboswitch is activated by the test compound, manufacturing the test compound that activates the riboswitch as the compound.

Also disclosed are compounds made by checking activation, deactivation or blocking of a riboswitch by a compound and manufacturing the checked compound. This can be accomplished by, for example, combining compound activation, deactivation or blocking assessment methods as disclosed elsewhere herein with methods for manufacturing the checked compounds. For example, compounds can be made by bringing into contact a test compound and a riboswitch, assessing activation of the riboswitch, and, if the riboswitch is activated by the test compound, manufacturing the test compound that activates the riboswitch as the compound. Checking compounds for their ability to activate, deactivate or block a riboswitch refers to both identification of compounds previously unknown to activate, deactivate or block a riboswitch and to assessing the ability of a compound to activate, deactivate or block a riboswitch where the compound was already known to activate, deactivate or block the riboswitch.

Specific compounds that can be used to activate riboswitches are also disclosed. Compounds useful with TPP-responsive riboswitches include compounds having the formula: 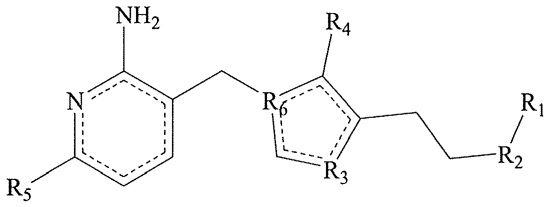 where the compound can bind a TPP-responsive riboswitch or derivative thereof, where R₁ is positively charged, where R₂ and R₃ are each independently C, O, or S, where R₄ is CH₃, NH₂, OH, SH, H or not present, where R₅ is CH₃, NH₂, OH, SH, or H, where R₆ is C or N, and where ------ each independently represent a single or double bond. Also contemplated are compounds as defined above where R₁ is phosphate, diphosphate or triphosphate.

Every compound within the above definition is intended to be and should be considered to be specifically disclosed herein. Further, every subgroup that can be identified within the above definition is intended to be and should be considered to be specifically disclosed herein. As a result, it is specifically contemplated that any compound, or subgroup of compounds can be either specifically included for or excluded from use or included in or excluded from a list of compounds. For example, as one option, a group of compounds is contemplated where each compound is as defined above but is not TPP, TP or thiamine. As another example, a group of compounds is contemplated where each compound is as defined above and is able to activate a TPP-responsive riboswitch. Thiamine pyrophosphate (TPP) is the trigger molecule for TPP-responsive riboswitches and can active TPP-responsive riboswitches. Pyrithiamine pyrophosphate can active TPP-responsive riboswitches. Pyrithiamine and pyrithiamine pyrophosphate can be independently and specifically included or excluded from the compounds, trigger molecules and methods disclosed herein. Thiamine and thiamine pyrophosphate can be independently and specifically included or excluded from the compounds, trigger molecules and methods disclosed herein.

### D. Constructs, Vectors and Expression Systems

The disclosed riboswitches can be used with any suitable expression system. Recombinant expression is usefully accomplished using a vector, such as a plasmid. The vector can include a promoter operably linked to riboswitch-encoding sequence and RNA to be expression (e.g., RNA encoding a protein). The vector can also include other elements required for transcription and translation. As used herein, vector refers to any carrier containing exogenous DNA. Thus, vectors are agents that transport the exogenous nucleic acid into a cell without degradation and include a promoter yielding expression of the nucleic acid in the cells into which it is delivered. Vectors include but are not limited to plasmids, viral nucleic acids, viruses, phage nucleic acids, phages, cosmids, and artificial chromosomes. A variety of prokaryotic and eukaryotic expression vectors suitable for carrying riboswitch-regulated constructs can be produced. Such expression vectors include, for example, pET, pET3d, pCR2.1, pBAD, pUC, and yeast vectors. The vectors can be used, for example, in a variety *of in vivo* and *in vitro* situation.

Viral vectors include adenovirus, adeno-associated virus, herpes virus, vaccinia virus, polio virus, AIDS virus, neuronal trophic virus, Sindbis and other RNA viruses, including these viruses with the HIV backbone. Also useful are any viral families which share the properties of these viruses which make them suitable for use as vectors. Retroviral vectors, which are described in Verma (1985), include Murine Maloney Leukemia virus, MMLV, and retroviruses that express the desirable properties of MMLV as a vector. Typically, viral vectors contain, nonstructural early genes, structural late genes, an RNA polymerase III transcript, inverted terminal repeats necessary for replication and encapsidation, and promoters to control the transcription and replication of the viral genome. When engineered as vectors, viruses typically have one or more of the early genes removed and a gene or gene/promoter cassette is inserted into the viral genome in place of the removed viral DNA.

A "promoter" is generally a sequence or sequences of DNA that function when in a relatively fixed location in regard to the transcription start site. A "promoter" contains core elements required for basic interaction of RNA polymerase and transcription factors and can contain upstream elements and response elements.

"Enhancer" generally refers to a sequence of DNA that functions at no fixed distance from the transcription start site and can be either 5' (Laimins, 1981) or 3' (Lusky et al., 1983) to the transcription unit. Furthermore, enhancers can be within an intron (Banerji et al., 1983) as well as within the coding sequence itself (Osborne et al., 1984). They are usually between 10 and 300 bp in length, and they function in cis. Enhancers function to increase transcription from nearby promoters. Enhancers, like promoters, also often contain response elements that mediate the regulation of transcription. Enhancers often determine the regulation of expression.

Expression vectors used in eukaryotic host cells (yeast, fungi, insect, plant, animal, human or nucleated cells) can also contain sequences necessary for the termination of transcription which can affect mRNA expression. These regions are transcribed as polyadenylated segments in the untranslated portion of the mRNA encoding tissue factor protein. The 3' untranslated regions also include transcription termination sites. It is preferred that the transcription unit also contain a polyadenylation region. One benefit of this region is that it increases the likelihood that the transcribed unit will be processed and transported like mRNA. The identification and use of polyadenylation signals in expression constructs is well established. It is preferred that homologous polyadenylation signals be used in the transgene constructs.

The vector can include nucleic acid sequence encoding a marker product. This marker product is used to determine if the gene has been delivered to the cell and once delivered is being expressed. Preferred marker genes are the *E. Coli* lacZ gene which encodes β-galactosidase and green fluorescent protein.

In some embodiments the marker can be a selectable marker. When such selectable markers are successfully transferred into a host cell, the transformed host cell can survive if placed under selective pressure. There are two widely used distinct categories of selective regimes. The first category is based on a cell's metabolism and the use of a mutant cell line which lacks the ability to grow independent of a supplemented media. The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, (Southern and Berg,1982), mycophenolic acid, (Mulligan and Berg, 1980) or hygromycin (Sugden et al., 1985).

Gene transfer can be obtained using direct transfer of genetic material, in but not limited to, plasmids, viral vectors, viral nucleic acids, phage nucleic acids, phages, cosmids, and artificial chromosomes, or via transfer of genetic material in cells or carriers such as cationic liposomes. Such methods are well known in the art and readily adaptable for use in the method described herein. Transfer vectors can be any nucleotide construction used to deliver genes into cells (e.g., a plasmid), or as part of a general strategy to deliver genes, e.g., as part of recombinant retrovirus or adenovirus (Ram et al. Cancer Res. 53:83-88, (1993)). Appropriate means for transfection, including viral vectors, chemical transfectants, or physico-mechanical methods such as electroporation and direct diffusion of DNA, are described by, for example, Wolff, J. A., et al., Science, 247, 1465-1468, (1990); and Wolff, J. A. Nature, 352, 815-818, (1991).

### 1. Viral Vectors

Preferred viral vectors are Adenovirus, Adeno-associated virus, Herpes virus, Vaccinia virus, Polio virus, AIDS virus, neuronal trophic virus, Sindbis and other RNA viruses, including these viruses with the HIV backbone. Also preferred are any viral families which share the properties of these viruses which make them suitable for use as vectors. Preferred retroviruses include Murine Maloney Leukemia virus, MMLV, and retroviruses that express the desirable properties of MMLV as a vector. Retroviral vectors are able to carry a larger genetic payload, i.e., a transgene or marker gene, than other viral vectors, and for this reason are a commonly used vector. However, they are not useful in non-proliferating cells. Adenovirus vectors are relatively stable and easy to work with, have high titers, and can be delivered in aerosol formulation, and can transfect non-dividing cells. Pox viral vectors are large and have several sites for inserting genes, they are thermostable and can be stored at room temperature. A preferred embodiment is a viral vector which has been engineered so as to suppress the immune response of the host organism, elicited by the viral antigens. Preferred vectors of this type will carry coding regions for Interleukin 8 or 10.

Viral vectors have higher transaction (ability to introduce genes) abilities than do most chemical or physical methods to introduce genes into cells. Typically, viral vectors contain, nonstructural early genes, structural late genes, an RNA polymerase III transcript, inverted terminal repeats necessary for replication and encapsidation, and promoters to control the transcription and replication of the viral genome. When engineered as vectors, viruses typically have one or more of the early genes removed and a gene or gene/promoter cassette is inserted into the viral genome in place of the removed viral DNA. Constructs of this type can carry up to about 8 kb of foreign genetic material. The necessary functions of the removed early genes are typically supplied by cell lines which have been engineered to express the gene products of the early genes in trans.

### i. Retroviral Vectors

A retrovirus is an animal virus belonging to the virus family of Retroviridae, including any types, subfamilies, genus, or tropisms. Retroviral vectors, in general, are described by Verma, I.M., Retroviral vectors for gene transfer. In Microbiology-1985, American Society for Microbiology, pp. 229-232, Washington, (1985), which is incorporated by reference herein. Examples of methods for using retroviral vectors for gene therapy are described in U.S. Patent Nos. 4,868,116 and 4,980,286; PCT applications WO 90/02806 and WO 89/07136; and Mulligan, (Science 260:926-932 (1993)); the teachings of which are incorporated herein by reference.

A retrovirus is essentially a package which has packed into it nucleic acid cargo. The nucleic acid cargo carries with it a packaging signal, which ensures that the replicated daughter molecules will be efficiently packaged within the package coat. In addition to the package signal, there are a number of molecules which are needed in cis, for the replication, and packaging of the replicated virus. Typically a retroviral genome, contains the gag, pol, and env genes which are involved in the making of the protein coat. It is the gag, pol, and env genes which are typically replaced by the foreign DNA that it is to be transferred to the target cell. Retrovirus vectors typically contain a packaging signal for incorporation into the package coat, a sequence which signals the start of the gag transcription unit, elements necessary for reverse transcription, including a primer binding site to bind the tRNA primer of reverse transcription, terminal repeat sequences that guide the switch of RNA strands during DNA synthesis, a purine rich sequence 5' to the 3' LTR that serve as the priming site for the synthesis of the second strand of DNA synthesis, and specific sequences near the ends of the LTRs that enable the insertion of the DNA state of the retrovirus to insert into the host genome. The removal of the gag, pol, and env genes allows for about 8 kb of foreign sequence to be inserted into the viral genome, become reverse transcribed , and upon replication be packaged into a new retroviral particle. This amount of nucleic acid is sufficient for the delivery of a one to many genes depending on the size of each transcript. It is preferable to include either positive or negative selectable markers along with other genes in the insert.

Since the replication machinery and packaging proteins in most retroviral vectors have been removed (gag, pol, and env), the vectors are typically generated by placing them into a packaging cell line. A packaging cell line is a cell line which has been transfected or transformed with a retrovirus that contains the replication and packaging machinery, but lacks any packaging signal. When the vector carrying the DNA of choice is transfected into these cell lines, the vector containing the gene of interest is replicated and packaged into new retroviral particles, by the machinery provided in cis by the helper cell. The genomes for the machinery are not packaged because they lack the necessary signals.

### ii. Adenoviral Vectors

The construction of replication-defective adenoviruses has been described (Berkner et al., J. Virology 61:1213-1220 (1987); Massie et al., Mol. Cell. Biol. 6:2872-2883 (1986); Haj-Ahmad et al., J. Virology 57:267-274 (1986); Davidson et al., J. Virology 61:1226-1239 (1987); Zhang "Generation and identification of recombinant adenovirus by liposome-mediated transfection and PCR analysis" BioTechniques 15:868-872 (1993)). The benefit of the use of these viruses as vectors is that they are limited in the extent to which they can spread to other cell types, since they can replicate within an initial infected cell, but are unable to form new infectious viral particles. Recombinant adenoviruses have been shown to achieve high efficiency gene transfer after direct, *in vivo* delivery to airway epithelium, hepatocytes, vascular endothelium, CNS parenchyma and a number of other tissue sites (Morsy, J. Clin. Invest. 92:1580-1586 (1993); Kirshenbaum, J. Clin. Invest. 92:381-387 (1993); Roessler, J. Clin. Invest. 92:1085-1092 (1993); Moullier, Nature Genetics 4:154-159 (1993); La Salle, Science 259:988-990 (1993); Gomez-Foix, J. Biol. Chem. 267:25129-25134 (1992); Rich, Human Gene Therapy 4:461-476 (1993); Zabner, Nature Genetics 6:75-83 (1994); Guzman, Circulation Research 73:1201-1207 (1993); Bout, Human Gene Therapy 5:3-10 (1994); Zabner, Cell 75:207-216 (1993); Caillaud, Eur. J. Neuroscience 5:1287-1291 (1993); and Ragot, J. Gen. Virology 74:501-507 (1993)). Recombinant adenoviruses achieve gene transduction by binding to specific cell surface receptors, after which the virus is internalized by receptor-mediated endocytosis, in the same manner as wild type or replication-defective adenovirus (Chardonnet and Dales, Virology 40:462-477 (1970); Brown and Burlingham, J. Virology 12:386-396 (1973); Svensson and Persson, J. Virology 55:442-449 (1985); Seth, et al., J. Virol. 51:650-655 (1984); Seth, et al., Mol. Cell. Biol. 4:1528-1533 (1984); Varga et al., J. Virology 65:6061-6070 (1991); Wickham et al., Cell 73:309-319 (1993)).

A preferred viral vector is one based on an adenovirus which has had the E1 gene removed and these virons are generated in a cell line such as the human 293 cell line. In another preferred embodiment both the E1 and E3 genes are removed from the adenovirus genome.

Another type of viral vector is based on an adeno-associated virus (AAV). This defective parvovirus is a preferred vector because it can infect many cell types and is nonpathogenic to humans. AAV type vectors can transport about 4 to 5 kb and wild type AAV is known to stably insert into chromosome 19. Vectors which contain this site specific integration property are preferred. An especially preferred embodiment of this type of vector is the P4.1 C vector produced by Avigen, San Francisco, CA, which can contain the herpes simplex virus thymidine kinase gene, HSV-tk, and/or a marker gene, such as the gene encoding the green fluorescent protein, GFP.

The inserted genes in viral and retroviral usually contain promoters, and/or enhancers to help control the expression of the desired gene product. A promoter is generally a sequence or sequences of DNA that function when in a relatively fixed location in regard to the transcription start site. A promoter contains core elements required for basic interaction of RNA polymerase and transcription factors, and can contain upstream elements and response elements.

### 2. Viral Promoters and Enhancers

Preferred promoters controlling transcription from vectors in mammalian host cells can be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. beta actin promoter. The early and late promoters of the SV40 virus are conveniently obtained as an SV40 restriction fragment which also contains the SV40 viral origin of replication (Fiers et al., Nature, 273: 113 (1978)). The immediate early promoter of the human cytomegalovirus is conveniently obtained as a HindIII E restriction fragment (Greenway, P.J. et al., Gene 18: 355-360 (1982)). Of course, promoters from the host cell or related species also are useful herein.

Enhancer generally refers to a sequence of DNA that functions at no fixed distance from the transcription start site and can be either 5' (Laimins, L. et al., Proc. Natl. Acad. Sci. 78: 993 (1981)) or 3' (Lusky, M.L., et al., Mol. Cell Bio. 3: 1108 (1983)) to the transcription unit. Furthermore, enhancers can be within an intron (Banerji, J.L. et al., Cell 33: 729 (1983)) as well as within the coding sequence itself (Osborne, T.F., et al., Mol. Cell Bio. 4: 1293 (1984)). They are usually between 10 and 300 bp in length, and they function in cis. Enhancers function to increase transcription from nearby promoters. Enhancers also often contain response elements that mediate the regulation of transcription. Promoters can also contain response elements that mediate the regulation of transcription. Enhancers often determine the regulation of expression of a gene. While many enhancer sequences are now known from mammalian genes (globin, elastase, albumin, α-fetoprotein and insulin), typically one will use an enhancer from a eukaryotic cell virus. Preferred examples are the SV40 enhancer on the late side of the replication origin (bp 100-270), the cytomegalovirus early promoter enhancer, the polyoma enhancer on the late side of the replication origin, and adenovirus enhancers.

The promoter and/or enhancer can be specifically activated either by light or specific chemical events which trigger their function. Systems can be regulated by reagents such as tetracycline and dexamethasone. There are also ways to enhance viral vector gene expression by exposure to irradiation, such as gamma irradiation, or alkylating chemotherapy drugs.

It is preferred that the promoter and/or enhancer region be active in all eukaryotic cell types. A preferred promoter of this type is the CMV promoter (650 bases). Other preferred promoters are SV40 promoters, cytomegalovirus (full length promoter), and retroviral vector LTF.

It has been shown that all specific regulatory elements can be cloned and used to construct expression vectors that are selectively expressed in specific cell types such as melanoma cells. The glial fibrillary acetic protein (GFAP) promoter has been used to selectively express genes in cells of glial origin.

Expression vectors used in eukaryotic host cells (yeast, fungi, insect, plant, animal, human or nucleated cells) can also contain sequences necessary for the termination of transcription which can affect mRNA expression. These regions are transcribed as polyadenylated segments in the untranslated portion of the mRNA encoding tissue factor protein. The 3' untranslated regions also include transcription termination sites. It is preferred that the transcription unit also contain a polyadenylation region. One benefit of this region is that it increases the likelihood that the transcribed unit will be processed and transported like mRNA. The identification and use of polyadenylation signals in expression constructs is well established. It is preferred that homologous polyadenylation signals be used in the transgene constructs. In a preferred embodiment of the transcription unit, the polyadenylation region is derived from the SV40 early polyadenylation signal and consists of about 400 bases. It is also preferred that the transcribed units contain other standard sequences alone or in combination with the above sequences improve expression from, or stability of, the construct.

### 3. Markers

The vectors can include nucleic acid sequence encoding a marker product. This marker product is used to determine if the gene has been delivered to the cell and once delivered is being expressed. Preferred marker genes are the E. Coli lacZ gene which encodes β-galactosidase and green fluorescent protein.

In some embodiments the marker can be a selectable marker. Examples of suitable selectable markers for mammalian cells are dihydrofolate reductase (DHFR), thymidine kinase, neomycin, neomycin analog G418, hydromycin, and puromycin. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. Two examples are: CHO DHFR- cells and mouse LTK- cells. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in non-supplemented media.

The second category is dominant selection which refers to a selection scheme used in any cell type and does not require the use of a mutant cell line. These schemes typically use a drug to arrest growth of a host cell. Those cells which would express a protein conveying drug resistance and would survive the selection. Examples of such dominant selection use the drugs neomycin, (Southern P. and Berg, P., J. Molec. Appl. Genet. 1: 327 (1982)), mycophenolic acid, (Mulligan, R.C. and Berg, P. Science 209: 1422 (1980)) or hygromycin, (Sugden, B. et al., Mol. Cell. Biol. 5: 410-413 (1985)). The three examples employ bacterial genes under eukaryotic control to convey resistance to the appropriate drug G418 or neomycin (geneticin), xgpt (mycophenolic acid) or hygromycin, respectively. Others include the neomycin analog G418 and puramycin.

### E. Biosensor Riboswitches

Also disclosed are biosensor riboswitches. Biosensor riboswitches are engineered riboswitches that produce a detectable signal in the presence of their cognate trigger molecule. Useful biosensor riboswitches can be triggered at or above threshold levels of the trigger molecules. Biosensor riboswitches can be designed for use *in vivo* or *in vitro.* For example, riboswitches that control alternative splicing can be operably linked to a reporter RNA that encodes a protein that serves as or is involved in producing a signal can be used *in vivo* by engineering a cell or organism to harbor a nucleic acid construct encoding the riboswitch. An example of a biosensor riboswitch for *use in vitro* is a riboswitch that includes a conformation dependent label, the signal from which changes depending on the activation state of the riboswitch. Such a biosensor riboswitch preferably uses an aptamer domain from or derived from a naturally occurring riboswitch.

### F. Reporter Proteins and Peptides

For assessing activation of a riboswitch, or for biosensor riboswitches, a reporter protein or peptide can be used. The reporter protein or peptide can be encoded by the RNA the expression of which is regulated by the riboswitch. The examples describe the use of some specific reporter proteins. The use of reporter proteins and peptides is well known and can be adapted easily for use with riboswitches. The reporter proteins can be any protein or peptide that can be detected or that produces a detectable signal. Preferably, the presence of the protein or peptide can be detected using standard techniques (e.g., radioimmunoassay, radio-labeling, immunoassay, assay for enzymatic activity, absorbance, fluorescence, luminescence, and Western blot). More preferably, the level of the reporter protein is easily quantifiable using standard techniques even at low levels. Useful reporter proteins include luciferases, green fluorescent proteins and their derivatives, such as firefly luciferase (FL) from Photinus pyralis, and Renilla luciferase (RL) from Renilla reniformis.

### G. Conformation Dependent Labels

Conformation dependent labels refer to all labels that produce a change in fluorescence intensity or wavelength based on a change in the form or conformation of the molecule or compound (such as a riboswitch) with which the label is associated. Examples of conformation dependent labels used in the context of probes and primers include molecular beacons, Amplifluors, FRET probes, cleavable FRET probes, TaqMan probes, scorpion primers, fluorescent triplex oligos including but not limited to triplex molecular beacons or triplex FRET probes, fluorescent water-soluble conjugated polymers, PNA probes and QPNA probes. Such labels, and, in particular, the principles of their function, can be adapted for use with riboswitches. Several types of conformation dependent labels are reviewed in Schweitzer and Kingsmore, Curr. Opin. Biotech. 12:21-27 (2001).

Stem quenched labels, a form of conformation dependent labels, are fluorescent labels positioned on a nucleic acid such that when a stem structure forms a quenching moiety is brought into proximity such that fluorescence from the label is quenched. When the stem is disrupted (such as when a riboswitch containing the label is activated), the quenching moiety is no longer in proximity to the fluorescent label and fluorescence increases. Examples of this effect can be found in molecular beacons, fluorescent triplex oligos, triplex molecular beacons, triplex FRET probes, and QPNA probes, the operational principles of which can be adapted for use with riboswitches.

Stem activated labels, a form of conformation dependent labels, are labels or pairs of labels where fluorescence is increased or altered by formation of a stem structure. Stem activated labels can include an acceptor fluorescent label and a donor moiety such that, when the acceptor and donor are in proximity (when the nucleic acid strands containing the labels form a stem structure), fluorescence resonance energy transfer from the donor to the acceptor causes the acceptor to fluoresce. Stem activated labels are typically pairs of labels positioned on nucleic acid molecules (such as riboswitches) such that the acceptor and donor are brought into proximity when a stem structure is formed in the nucleic acid molecule. If the donor moiety of a stem activated label is itself a fluorescent label, it can release energy as fluorescence (typically at a different wavelength than the fluorescence of the acceptor) when not in proximity to an acceptor (that is, when a stem structure is not formed). When the stem structure forms, the overall effect would then be a reduction of donor fluorescence and an increase in acceptor fluorescence. FRET probes are an example of the use of stem activated labels, the operational principles of which can be adapted for use with riboswitches.

### H. Detection Labels

To aid in detection and quantitation of riboswitch activation, deactivation or blocking, or expression of nucleic acids or protein produced upon activation, deactivation or blocking of riboswitches, detection labels can be incorporated into detection probes or detection molecules or directly incorporated into expressed nucleic acids or proteins. As used herein, a detection label is any molecule that can be associated with nucleic acid or protein, directly or indirectly, and which results in a measurable, detectable signal, either directly or indirectly. Many such labels are known to those of skill in the art. Examples of detection labels suitable for use in the disclosed method are radioactive isotopes, fluorescent molecules, phosphorescent molecules, enzymes, antibodies, and ligands.

Examples of suitable fluorescent labels include fluorescein isothiocyanate (FITC), 5,6-carboxymethyl fluorescein, Texas red, nitrobenz-2-oxa-1,3-diazol-4-yl (NBD), coumarin, dansyl chloride, rhodamine, amino-methyl coumarin (AMCA), Eosin, Erythrosin, BODIPY^{®}, Cascade Blue^{®}, Oregon Green^{®}, pyrene, lissamine, xanthenes, acridines, oxazines, phycoerythrin, macrocyclic chelates of lanthanide ions such as quantum dye™, fluorescent energy transfer dyes, such as thiazole orange-ethidium heterodimer, and the cyanine dyes Cy3, Cy3.5, Cy5, Cy5.5 and Cy7. Examples of other specific fluorescent labels include 3-Hydroxypyrene 5,8,10-Tri Sulfonic acid, 5-Hydroxy Tryptamine (5-HT), Acid Fuchsin, Alizarin Complexon, Alizarin Red, Allophycocyanin, Aminocoumarin, Anthroyl Stearate, Astrazon Brilliant Red 4G, Astrazon Orange R, Astrazon Red 6B, Astrazon Yellow 7 GLL, Atabrine, Auramine, Aurophosphine, Aurophosphine G, BAO 9 (Bisaminophenyloxadiazole), BCECF, Berberine Sulphate, Bisbenzamide, Blancophor FFG Solution, Blancophor SV, Bodipy F1, Brilliant Sulphoflavin FF, Calcien Blue, Calcium Green, Calcofluor RW Solution, Calcofluor White, Calcophor White ABT Solution, Calcophor White Standard Solution, Carbostyryl, Cascade Yellow, Catecholamine, Chinacrine, Coriphosphine O, Coumarin-Phalloidin, CY3.1 8, CY5.1 8, CY7, Dans (1-Dimethyl Amino Naphaline 5 Sulphonic Acid), Dansa (Diamino Naphtyl Sulphonic Acid), Dansyl NH-CH3, Diamino Phenyl Oxydiazole (DAO), Dimcthylamino-5-Sulphonic acid, Dipyrrometheneboron Difluoridc, Diphcnyl Brilliant Flavine 7GFF, Dopamine, Erythrosin ITC, Euchrysin, FIF (Formaldehyde Induced Fluorescence), Flazo Orange, Fluo 3, Fluorescamine, Fura-2, Genacryl Brilliant Red B, Genacryl Brilliant Yellow 10GF, Genacryl Pink 3G, Genacryl Yellow 5GF, Gloxalic Acid, Granular Blue, Haematoporphyrin, Indo-1, Intrawhite Cf Liquid, Leucophor PAF, Leucophor SF, Leucophor WS, Lissamine Rhodamine B200 (RD200), Lucifer Yellow CH, Lucifer Yellow VS, Magdala Red, Marina Blue, Maxilon Brilliant Flavin 10 GFF, Maxilon Brilliant Flavin 8 GFF, MPS (Methyl Green Pyronine Stilbene), Mithramycin, NBD Amine, Nitrobenzoxadidole, Noradrenaline, Nuclear Fast Red, Nuclear Yellow, Nylosan Brilliant Flavin E8G, Oxadiazole, Pacific Blue, Pararosaniline (Feulgen), Phorwite AR Solution, Phorwite BKL, Phorwite Rev, Phorwite RPA, Phosphine 3R, Phthalocyanine, Phycoerythrin R, Polyazaindacene Pontochrome Blue Black, Porphyrin, Primuline, Procion Yellow, Pyronine, Pyronine B, Pyrozal Brilliant Flavin 7GF, Quinacrine Mustard, Rhodamine 123, Rhodamine 5 GLD, Rhodamine 6G, Rhodamine B, Rhodamine B 200, Rhodamine B Extra, Rhodamine BB, Rhodamine BG, Rhodamine WT, Serotonin, Sevron Brilliant Red 2B, Sevron Brilliant Red 4G, Sevron Brilliant Red B, Sevron Orange, Sevron Yellow L, SITS (Primuline), SITS (Stilbene Isothiosulphonic acid), Stilbene, Snarf 1, sulpho Rhodamine B Can C, Sulpho Rhodamine G Extra, Tetracycline, Thiazine Red R, Thioflavin S, Thioflavin TCN, Thioflavin 5, Thiolyte, Thiozol Orange, Tinopol CBS, True Blue, Ultralite, Uranine B, Uvitex SFC, Xylene Orange, and XRITC.

Useful fluorescent labels are fluorescein (5-carboxyfluorescein-N-hydroxysuccinimide ester), rhodamine (5,6-tetramethyl rhodamine), and the cyanine dyes Cy3, Cy3.5, Cy5, Cy5.5 and Cy7. The absorption and emission maxima, respectively, for these fluors are: FITC (490 nm; 520 nm), Cy3 (554 nm; 568 nm), Cy3.5 (581 nm; 588 nm), Cy5 (652 nm: 672 nm), Cy5.5 (682 nm; 703 nm) and Cy7 (755 nm; 778 nm), thus allowing their simultaneous detection. Other examples of fluorescein dyes include 6-carboxyfluorescein (6-FAM), 2',4',1,4,-tetrachlorofluorescein (TET), 2',4',5',7',1,4-hexachlorofluorescein (HEX), 2',7'-dimethoxy-4', 5'-dichloro-6-carboxyrhodamine (JOE), 2'-chloro-5'-fluoro-7',8'-fused phenyl-1,4-dichloro-6-carboxyfluorescein (NED), and 2'-chloro-7'-phenyl-1,4-dichloro-6-carboxyfluorescein (VIC). Fluorescent labels can be obtained from a variety of commercial sources, including Amersham Pharmacia Biotech, Piscataway, NJ; Molecular Probes, Eugene, OR; and Research Organics, Cleveland, Ohio.

Additional labels of interest include those that provide for signal only when the probe with which they are associated is specifically bound to a target molecule, where such labels include: "molecular beacons" as described in Tyagi & Kramer, Nature Biotechnology (1996) 14:303 and EP 0 070 685 B1. Other labels of interest include those described in U.S. Pat. No. 5,563,037; WO 97/17471 and WO 97/17076.

Labeled nucleotides are a useful form of detection label for direct incorporation into expressed nucleic acids during synthesis. Examples of detection labels that can be incorporated into nucleic acids include nucleotide analogs such as BrdUrd (5-bromodeoxyuridine, Hoy and Schimke, Mutation Research 290:217-230 (1993)), aminoallyldeoxyuridine (Henegariu et al., Nature Biotechnology 18:345-348 (2000)), 5-methylcytosine (Sano et al., Biochim. Biophys. Acta 951:157-165 (1988)), bromouridine (Wansick et al., J. Cell Biology 122:283-293 (1993)) and nucleotides modified with biotin (Langer et al., Proc. Natl. Acad. Sci. USA 78:6633 (1981)) or with suitable haptens such as digoxygenin (Kerkhof, Anal. Biochem. 205:359-364 (1992)). Suitable fluorescence-labeled nucleotides are Fluorescein-isothiocyanate-dUTP, Cyanine-3-dUTP and Cyanine-5-dUTP (Yu et al., Nucleic Acids Res., 22:3226-3232 (1994)). A preferred nucleotide analog detection label for DNA is BrdUrd (bromodeoxyuridine, BrdUrd, BrdU, BUdR, Sigma-Aldrich Co). Other useful nucleotide analogs for incorporation of detection label into DNA are AA-dUTP (aminoallyl-deoxyuridine triphosphate, Sigma-Aldrich Co.), and 5-methyl-dCTP (Roche Molecular Biochemicals). A useful nucleotide analog for incorporation of detection label into RNA is biotin-16-UTP (biotin-16-uridine-5'-triphosphate, Roche Molecular Biochemicals). Fluorescein, Cy3, and Cy5 can be linked to dUTP for direct labeling. Cy3.5 and Cy7 are available as avidin or anti-digoxygenin conjugates for secondary detection of biotin- or digoxygenin-labeled probes.

Detection labels that are incorporated into nucleic acid, such as biotin, can be subsequently detected using sensitive methods well-known in the art. For example, biotin can be detected using streptavidin-alkaline phosphatase conjugate (Tropix, Inc.), which is bound to the biotin and subsequently detected by chemiluminescence of suitable substrates (for example, chemiluminescent substrate CSPD: disodium, 3-(4-methoxyspiro-[1,2,-dioxetane-3-2'-(5'-chloro)tricyclo [3.3.1.1^{3,7}]decane]-4-yl) phenyl phosphate; Tropix, Inc.). Labels can also be enzymes, such as alkaline phosphatase, soybean peroxidase, horseradish peroxidase and polymerases, that can be detected, for example, with chemical signal amplification or by using a substrate to the enzyme which produces light (for example, a chemiluminescent 1,2-dioxetane substrate) or fluorescent signal.

Molecules that combine two or more of these detection labels are also considered detection labels. Any of the known detection labels can be used with the disclosed probes, tags, molecules and methods to label and detect activated or deactivated riboswitches or nucleic acid or protein produced in the disclosed methods. Methods for detecting and measuring signals generated by detection labels are also known to those of skill in the art. For example, radioactive isotopes can be detected by scintillation counting or direct visualization; fluorescent molecules can be detected with fluorescent spectrophotometers; phosphorescent molecules can be detected with a spectrophotometer or directly visualized with a camera; enzymes can be detected by detection or visualization of the product of a reaction catalyzed by the enzyme; antibodies can be detected by detecting a secondary detection label coupled to the antibody. As used herein, detection molecules are molecules which interact with a compound or composition to be detected and to which one or more detection labels are coupled.

### 1. Sequence Similarities

It is understood that as discussed herein the use of the terms homology and identity mean the same thing as similarity. Thus, for example, if the use of the word homology is used between two sequences (non-natural sequences, for example) it is understood that this is not necessarily indicating an evolutionary relationship between these two sequences, but rather is looking at the similarity or relatedness between their nucleic acid sequences. Many of the methods for determining homology between two evolutionarily related molecules are routinely applied to any two or more nucleic acids or proteins for the purpose of measuring sequence similarity regardless of whether they are evolutionarily related or not.

In general, it is understood that one way to define any known variants and derivatives or those that might arise, of the disclosed riboswitches, aptamers, expression platforms, genes and proteins herein, is through defining the variants and derivatives in terms of homology to specific known sequences. This identity of particular sequences disclosed herein is also discussed elsewhere herein. In general, variants of riboswitches, aptamers, expression platforms, genes and proteins herein disclosed typically have at least, about 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99 percent homology to a stated sequence or a native sequence. Those of skill in the art readily understand how to determine the homology of two proteins or nucleic acids, such as genes. For example, the homology can be calculated after aligning the two sequences so that the homology is at its highest level.

Another way of calculating homology can be performed by published algorithms. Optimal alignment of sequences for comparison can be conducted by the local homology algorithm of Smith and Waterman Adv. Appl. Math. 2: 482 (1981), by the homology alignment algorithm of Needleman and Wunsch, J. MoL Biol. 48: 443 (1970), by the search for similarity method of Pearson and Lipman, Proc. Natl. Acad. Sci. U.S.A. 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by inspection.

The same types of homology can be obtained for nucleic acids by for example the algorithms disclosed in Zuker, M. Science 244:48-52, 1989, Jaeger et al. Proc. Natl. Acad. Sci. USA 86:7706-7710, 1989, Jaeger et al. Methods Enzymol. 183:281-306, 1989 which are herein incorporated by reference for at least material related to nucleic acid alignment. It is understood that any of the methods typically can be used and that in certain instances the results of these various methods can differ, but the skilled artisan understands if identity is found with at least one of these methods, the sequences would be said to have the stated identity.

For example, as used herein, a sequence recited as having a particular percent homology to another sequence refers to sequences that have the recited homology as calculated by any one or more of the calculation methods described above. For example, a first sequence has 80 percent homology, as defined herein, to a second sequence if the first sequence is calculated to have 80 percent homology to the second sequence using the Zuker calculation method even if the first sequence does not have 80 percent homology to the second sequence as calculated by any of the other calculation methods. As another example, a first sequence has 80 percent homology, as defined herein, to a second sequence if the first sequence is calculated to have 80 percent homology to the second sequence using both the Zuker calculation method and the Pearson and Lipman calculation method even if the first sequence does not have 80 percent homology to the second sequence as calculated by the Smith and Waterman calculation method, the Needleman and Wunsch calculation method, the Jaeger calculation methods, or any of the other calculation methods. As yet another example, a first sequence has 80 percent homology, as defined herein, to a second sequence if the first sequence is calculated to have 80 percent homology to the second sequence using each of calculation methods (although, in practice, the different calculation methods will often result in different calculated homology percentages).

### J. Hybridization and Selective Hybridization

The term hybridization typically means a sequence driven interaction between at least two nucleic acid molecules, such as a primer or a probe and a riboswitch or a gene. Sequence driven interaction means an interaction that occurs between two nucleotides or nucleotide analogs or nucleotide derivatives in a nucleotide specific manner. For example, G interacting with C or A interacting with T are sequence driven interactions. Typically sequence driven interactions occur on the Watson-Crick face or Hoogsteen face of the nucleotide. The hybridization of two nucleic acids is affected by a number of conditions and parameters known to those of skill in the art. For example, the salt concentrations, pH, and temperature of the reaction all affect whether two nucleic acid molecules will hybridize.

Parameters for selective hybridization between two nucleic acid molecules are well known to those of skill in the art. For example, in some embodiments selective hybridization conditions can be defined as stringent hybridization conditions. For example, stringency of hybridization is controlled by both temperature and salt concentration of either or both of the hybridization and washing steps. For example, the conditions of hybridization to achieve selective hybridization can involve hybridization in high ionic strength solution (6X SSC or 6X SSPE) at a temperature that is about 12-25°C below the Tm (the melting temperature at which half of the molecules dissociate from their hybridization partners) followed by washing at a combination of temperature and salt concentration chosen so that the washing temperature is about 5°C to 20°C below the Tm. The temperature and salt conditions are readily determined empirically in preliminary experiments in which samples of reference DNA immobilized on filters are hybridized to a labeled nucleic acid of interest and then washed under conditions of different stringencies. Hybridization temperatures are typically higher for DNA-RNA and RNA-RNA hybridizations. The conditions can be used as described above to achieve stringency, or as is known in the art (Sambrook et al., Molecular Cloning: A Laboratory Manual, 2nd Ed., Cold Spring Harbor Laboratory, Cold Spring Harbor, New York, 1989; Kunkel et al. Methods Enzymol. 1987:154:367, 1987 which is herein incorporated by reference for material at least related to hybridization of nucleic acids). A preferable stringent hybridization condition for a DNA:DNA hybridization can be at about 68°C (in aqueous solution) in 6X SSC or 6X SSPE followed by washing at 68°C. Stringency of hybridization and washing, if desired, can be reduced accordingly as the degree of complementarity desired is decreased, and further, depending upon the G-C or A-T richness of any area wherein variability is searched for. Likewise, stringency of hybridization and washing, if desired, can be increased accordingly as homology desired is increased, and further, depending upon the G-C or A-T richness of any area wherein high homology is desired, all as known in the art.

Another way to define selective hybridization is by looking at the amount (percentage) of one of the nucleic acids bound to the other nucleic acid. For example, in some embodiments selective hybridization conditions would be when at least about, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the limiting nucleic acid is bound to the non-limiting nucleic acid. Typically, the non-limiting nucleic acid is in for example, 10 or 100 or 1000 fold excess. This type of assay can be performed at under conditions where both the limiting and non-limiting nucleic acids are for example, 10 fold or 100 fold or 1000 fold below their k_{d}, or where only one of the nucleic acid molecules is 10 fold or 100 fold or 1000 fold or where one or both nucleic acid molecules are above their k_{d}.

Another way to define selective hybridization is by looking at the percentage of nucleic acid that gets enzymatically manipulated under conditions where hybridization is required to promote the desired enzymatic manipulation. For example, in some embodiments selective hybridization conditions would be when at least about, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the nucleic acid is enzymatically manipulated under conditions which promote the enzymatic manipulation, for example if the enzymatic manipulation is DNA extension, then selective hybridization conditions would be when at least about 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the nucleic acid molecules are extended. Preferred conditions also include those suggested by the manufacturer or indicated in the art as being appropriate for the enzyme performing the manipulation.

Just as with homology, it is understood that there are a variety of methods herein disclosed for determining the level of hybridization between two nucleic acid molecules. It is understood that these methods and conditions can provide different percentages of hybridization between two nucleic acid molecules, but unless otherwise indicated meeting the parameters of any of the methods would be sufficient. For example if 80% hybridization was required and as long as hybridization occurs within the required parameters in any one of these methods it is considered disclosed herein.

It is understood that those of skill in the art understand that if a composition or method meets any one of these criteria for determining hybridization either collectively or singly it is a composition or method that is disclosed herein.

### K. Nucleic Acids

There are a variety of molecules disclosed herein that are nucleic acid based, including, for example, riboswitches, aptamers, and nucleic acids that encode riboswitches and aptamers. The disclosed nucleic acids can be made up of for example, nucleotides, nucleotide analogs, or nucleotide substitutes. Non-limiting examples of these and other molecules are discussed herein. It is understood that for example, when a vector is expressed in a cell, that the expressed mRNA will typically be made up of A, C, G, and U. Likewise, it is understood that if a nucleic acid molecule is introduced into a cell or cell environment through for example exogenous delivery, it is advantageous that the nucleic acid molecule be made up of nucleotide analogs that reduce the degradation of the nucleic acid molecule in the cellular environment.

So long as their relevant function is maintained, riboswitches, aptamers, expression platforms and any other oligonucleotides and nucleic acids can be made up of or include modified nucleotides (nucleotide analogs). Many modified nucleotides are known and can be used in oligonucleotides and nucleic acids. A nucleotide analog is a nucleotide which contains some type of modification to either the base, sugar, or phosphate moieties. Modifications to the base moiety would include natural and synthetic modifications of A, C, G, and T/U as well as different purine or pyrimidine bases, such as uracil-5-yl, hypoxanthin-9-yl (I), and 2-aminoadenin-9-yl. A modified base includes but is not limited to 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine. Additional base modifications can be found for example in U.S. Pat. No. 3,687,808, Englisch et al., Angewandte Chemie, International Edition, 1991, 30, 613, and Sanghvi, Y. S., Chapter 15, Antisense Research and Applications, pages 289-302, Crooke, S. T. and Lebleu, B. ed., CRC Press, 1993. Certain nucleotide analogs, such as 5-substituted pyrimidines, 6-azapyrimidines and N-2, N-6 and O-6 substituted purines, including 2-aminopropyladenine, 5-propynyluracil and 5-propynylcytosine. 5-methylcytosine can increase the stability of duplex formation. Other modified bases are those that function as universal bases. Universal bases include 3-nitropyrrole and 5-nitroindole. Universal bases substitute for the normal bases but have no bias in base pairing. That is, universal bases can base pair with any other base. Base modifications often can be combined with for example a sugar modification, such as 2'-O-methoxyethyl, to achieve unique properties such as increased duplex stability. There are numerous United States patents such as 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; and 5,681,941, which detail and describe a range of base modifications. Each of these patents is herein incorporated by reference in its entirety, and specifically for their description of base modifications, their synthesis, their use, and their incorporation into oligonucleotides and nucleic acids.

Nucleotide analogs can also include modifications of the sugar moiety. Modifications to the sugar moiety would include natural modifications of the ribose and deoxyribose as well as synthetic modifications. Sugar modifications include but are not limited to the following modifications at the 2'position: OH; F; O-, S-, or N-alkyl; O-, S-, or N-alkenyl; O-, S- or N-alkynyl; or O-alkyl-O-alkyl, wherein the alkyl, alkenyl and alkynyl can be substituted or unsubstituted C1 to C10, alkyl or C2 to C10 alkenyl and alkynyl. 2' sugar modifications also include but are not limited to -O[(CH₂)n O]m CH₃, - O(CH₂)n OCH₃, -O(CH₂)n NH₂, -O(CH₂)n CH₃, -O(CH₂)n -ONH₂, and - O(CH₂)nON[(CH₂)n CH₃)]₂, where n and m are from 1 to about 10.

Other modifications at the 2' position include but are not limited to: C1 to C10 lower alkyl, substituted lower alkyl, alkaryl, aralkyl, O-alkaryl or O-aralkyl, SH, SCH₃, OCN, C1, Br, CN, CF₃, OCF₃, SOCH₃, SO₂ CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. Similar modifications can also be made at other positions on the sugar, particularly the 3' position of the sugar on the 3' terminal nucleotide or in 2'-5' linked oligonucleotides and the 5' position of 5' terminal nucleotide. Modified sugars would also include those that contain modifications at the bridging ring oxygen, such as CH₂ and S. Nucleotide sugar analogs can also have sugar mimetics such as cyclobutyl moieties in place of the pentofuranosyl sugar. There are numerous United States patents that teach the preparation of such modified sugar structures such as 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; and 5,700,920, each of which is herein incorporated by reference in its entirety, and specifically for their description of modified sugar structures, their synthesis, their use, and their incorporation into nucleotides, oligonucleotides and nucleic acids.

Nucleotide analogs can also be modified at the phosphate moiety. Modified phosphate moieties include but are not limited to those that can be modified so that the linkage between two nucleotides contains a phosphorothioate, chiral phosphorothioate, phosphorodithioate, phosphotriester, aminoalkylphosphotriester, methyl and other alkyl phosphonates including 3'-alkylene phosphonate and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates. It is understood that these phosphate or modified phosphate linkages between two nucleotides can be through a 3'-5' linkage or a 2'-5' linkage, and the linkage can contain inverted polarity such as 3'-5' to 5'-3' or 2'-5' to 5'-2'. Various salts, mixed salts and free acid forms are also included. Numerous United States patents teach how to make and use nucleotides containing modified phosphates and include but are not limited to, 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5,625,050, each of which is herein incorporated by reference its entirety, and specifically for their description of modified phosphates, their synthesis, their use, and their incorporation into nucleotides, oligonucleotides and nucleic acids.

It is understood that nucleotide analogs need only contain a single modification, but can also contain multiple modifications within one of the moieties or between different moieties.

Nucleotide substitutes are molecules having similar functional properties to nucleotides, but which do not contain a phosphate moiety, such as peptide nucleic acid (PNA). Nucleotide substitutes are molecules that will recognize and hybridize to (base pair to) complementary nucleic acids in a Watson-Crick or Hoogsteen manner, but which are linked together through a moiety other than a phosphate moiety. Nucleotide substitutes are able to conform to a double helix type structure when interacting with the appropriate target nucleic acid.

Nucleotide substitutes are nucleotides or nucleotide analogs that have had the phosphate moiety and/or sugar moieties replaced. Nucleotide substitutes do not contain a standard phosphorus atom. Substitutes for the phosphate can be for example, short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH2 component parts. Numerous United States patents disclose how to make and use these types of phosphate replacements and include but are not limited to 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439, each of which is herein incorporated by reference its entirety, and specifically for their description of phosphate replacements, their synthesis, their use, and their incorporation into nucleotides, oligonucleotides and nucleic acids.

It is also understood in a nucleotide substitute that both the sugar and the phosphate moieties of the nucleotide can be replaced, by for example an amide type linkage (aminoethylglycine) (PNA). United States patents 5,539,082; 5,714,331; and 5,719,262 teach how to make and use PNA molecules, each of which is herein incorporated by reference. (See also Nielsen et al., Science 254:1497-1500 (1991)).

Oligonucleotides and nucleic acids can be comprised of nucleotides and can be made up of different types of nucleotides or the same type of nucleotides. For example, one or more of the nucleotides in an oligonucleotide can be ribonucleotides, 2'-O-methyl ribonucleotides, or a mixture of ribonucleotides and 2'-O-methyl ribonucleotides; about 10% to about 50% of the nucleotides can be ribonucleotides, 2'-O-methyl ribonucleotides, or a mixture of ribonucleotides and 2'-O-methyl ribonucleotides; about 50% or more of the nucleotides can be ribonucleotides, 2'-O-methyl ribonucleotides, or a mixture of ribonucleotides and 2'-O-methyl ribonucleotides; or all of the nucleotides are ribonucleotides, 2'-O-methyl ribonucleotides, or a mixture of ribonucleotides and 2'-O-methyl ribonucleotides. Such oligonucleotides and nucleic acids can be referred to as chimeric oligonucleotides and chimeric nucleic acids.

### L. Solid Supports

Solid supports are solid-state substrates or supports with which molecules (such as trigger molecules) and riboswitches (or other components used in, or produced by, the disclosed methods) can be associated. Riboswitches and other molecules can be associated with solid supports directly or indirectly. For example, analytes (e.g., trigger molecules, test compounds) can be bound to the surface of a solid support or associated with capture agents (e.g., compounds or molecules that bind an analyte) immobilized on solid supports. As another example, riboswitches can be bound to the surface of a solid support or associated with probes immobilized on solid supports. An array is a solid support to which multiple riboswitches, probes or other molecules have been associated in an array, grid, or other organized pattern.

Solid-state substrates for use in solid supports can include any solid material with which components can be associated, directly or indirectly. This includes materials such as acrylamide, agarose, cellulose, nitrocellulose, glass, gold, polystyrene, polyethylene vinyl acetate, polypropylene, polymethacrylate, polyethylene, polyethylene oxide, polysilicates, polycarbonates, teflon, fluorocarbons, nylon, silicon rubber, polyanhydrides, polyglycolic acid, polylactic acid, polyorthoesters, functionalized silane, polypropylfumerate, collagen, glycosaminoglycans, and polyamino acids. Solid-state substrates can have any useful form including thin film, membrane, bottles, dishes, fibers, woven fibers, shaped polymers, particles, beads, microparticles, or a combination. Solid-state substrates and solid supports can be porous or non-porous. A chip is a rectangular or square small piece of material. Preferred forms for solid-state substrates are thin films, beads, or chips. A useful form for a solid-state substrate is a microtiter dish. In some embodiments, a multiwell glass slide can be employed.

An array can include a plurality of riboswitches, trigger molecules, other molecules, compounds or probes immobilized at identified or predefined locations on the solid support. Each predefined location on the solid support generally has one type of component (that is, all the components at that location are the same). Alternatively, multiple types of components can be immobilized in the same predefined location on a solid support. Each location will have multiple copies of the given components. The spatial separation of different components on the solid support allows separate detection and identification.

Although useful, it is not required that the solid support be a single unit or structure. A set of riboswitches, trigger molecules, other molecules, compounds and/or probes can be distributed over any number of solid supports. For example, at one extreme, each component can be immobilized in a separate reaction tube or container, or on separate beads or microparticles.

Methods for immobilization of oligonucleotides to solid-state substrates are well established. Oligonucleotides, including address probes and detection probes, can be coupled to substrates using established coupling methods. For example, suitable attachment methods are described by Pease et al., Proc. Natl. Acad. Sci. USA 91(11):5022-5026 (1994), and Khrapko et al., Mol Biol (Mosk) (USSR) 25:718-730 (1991). A method for immobilization of 3'-amine oligonucleotides on casein-coated slides is described by Stimpson et al., Proc. Natl. Acad. Sci. USA 92:6379-6383 (1995). A useful method of attaching oligonucleotides to solid-state substrates is described by Guo et al., Nucleic Acids Res. 22:5456-5465 (1994).

Each of the components (for example, riboswitches, trigger molecules, or other molecules) immobilized on the solid support can be located in a different predefined region of the solid support. The different locations can be different reaction chambers. Each of the different predefined regions can be physically separated from each other of the different regions. The distance between the different predefined regions of the solid support can be either fixed or variable. For example, in an array, each of the components can be arranged at fixed distances from each other, while components associated with beads will not be in a fixed spatial relationship. In particular, the use of multiple solid support units (for example, multiple beads) will result in variable distances.

Components can be associated or immobilized on a solid support at any density. Components can be immobilized to the solid support at a density exceeding 400 different components per cubic centimeter. Arrays of components can have any number of components. For example, an array can have at least 1,000 different components immobilized on the solid support, at least 10,000 different components immobilized on the solid support, at least 100,000 different components immobilized on the solid support, or at least 1,000,000 different components immobilized on the solid support.

### M. Kits

The materials described above as well as other materials can be packaged together in any suitable combination as a kit useful for performing, or aiding in the performance of, the disclosed method. It is useful if the kit components in a given kit are designed and adapted for use together in the disclosed method. For example disclosed are kits for detecting compounds, the kit comprising one or more biosensor riboswitches. The kits also can contain reagents and labels for detecting activation of the riboswitches.

### N. Mixtures

Disclosed are mixtures formed by performing or preparing to perform the disclosed method. For example, disclosed are mixtures comprising riboswitches and trigger molecules.

Whenever the method involves mixing or bringing into contact compositions or components or reagents, performing the method creates a number of different mixtures. For example, if the method includes 3 mixing steps, after each one of these steps a unique mixture is formed if the steps are performed separately. In addition, a mixture is formed at the completion of all of the steps regardless of how the steps were performed. The present disclosure contemplates these mixtures, obtained by the performance of the disclosed methods as well as mixtures containing any disclosed reagent, composition, or component, for example, disclosed herein.

### O. Systems

Disclosed are systems useful for performing, or aiding in the performance of, the disclosed method. Systems generally comprise combinations of articles of manufacture such as structures, machines, devices, and the like, and compositions, compounds, materials, and the like. Such combinations that are disclosed or that are apparent from the disclosure are contemplated. For example, disclosed and contemplated are systems comprising biosensor riboswitches, a solid support and a signal-reading device.

### P. Data Structures and Computer Control

Disclosed are data structures used in, generated by, or generated from, the disclosed method. Data structures generally are any form of data, information, and/or objects collected, organized, stored, and/or embodied in a composition or medium. Riboswitch structures and activation measurements stored in electronic form, such as in RAM or on a storage disk, is a type of data structure.

The disclosed method, or any part thereof or preparation therefor, can be controlled, managed, or otherwise assisted by computer control. Such computer control can be accomplished by a computer controlled process or method, can use and/or generate data structures, and can use a computer program. Such computer control, computer controlled processes, data structures, and computer programs are contemplated and should be understood to be disclosed herein.

### Methods

Disclosed herein are methods for regulating splicing of RNA comprising introducing into the RNA a construct comprising a riboswitch, wherein the riboswitch is capable of regulating splicing of RNA. The riboswitch can, for example, regulaate alternative splicing. The riboswitch can comprise an aptamer domain and an expression platform domain, wherein the aptamer domain and the expression platform domain are heterologous. The riboswitch can be in an intron of the RNA. The riboswitch can be activated by a trigger molecule, such as TPP. The riboswitch can be a TPP-responsive riboswitch. The riboswitch can activate alternative splicing. The riboswitch can repress alternative splicing. The splicing can occur non-naturally. The region of the aptamer with alternative splicing control can be found, for example, in loop 5. The region of the aptamer with alternative splicing control can also found, for example, in stem P2. The splice sites can be located, for example, at positions between -6 to -24 relative to the 5' end of the aptamer. The splice sites can follow, for example, the sequence GUA in the aptamer.

By "regulating splicing of RNA" is meant a riboswitch that can control splicing of RNA, thereby causing a different mRNA molecule to be formed, and potentially (though not always) a different protein. The riboswitch can, for example, regulaate alternative splicing.

Further disclosed are methods for activating, deactivating or blocking a riboswitch that regulates splicing of RNA. Such methods can involve, for example, bringing into contact a riboswitch and a compound or trigger molecule that can activate, deactivate or block the riboswitch. Riboswitches function to control gene expression through the binding or removal of a trigger molecule. Compounds can be used to activate, deactivate or block a riboswitch. The trigger molecule for a riboswitch (as well as other activating compounds) can be used to activate a riboswitch. Compounds other than the trigger molecule generally can be used to deactivate or block a riboswitch (such as TPP). Riboswitches can also be deactivated by, for example, removing trigger molecules from the presence of the riboswitch. Thus, the disclosed method of deactivating a riboswitch can involve, for example, removing a trigger molecule (or other activating compound) from the presence or contact with the riboswitch. A riboswitch can be blocked by, for example, binding of an analog of the trigger molecule that does not activate the riboswitch.

Also disclosed are methods for altering expression of an RNA molecule, or of a gene encoding an RNA molecule, where the RNA molecule includes a riboswitch that regulates splicing, by bringing a compound into contact with the RNA molecule. The riboswitch can, for example, regulate alternative spicing of the RNA molecule. Riboswitches function to control gene expression through the binding or removal of a trigger molecule. Thus, subjecting an RNA molecule of interest that includes a riboswitch to conditions that activate, deactivate or block the riboswitch can be used to alter expression of the RNA. Expression can be altered as a result of, for example, termination of transcription or blocking of ribosome binding to the RNA. Binding of a trigger molecule can, depending on the nature of the riboswitch and the type of alternative splicing that occurs, reduce or prevent expression of the RNA molecule or promote or increase expression of the RNA molecule.

Also disclosed are methods for regulating expression of a naturally occurring gene or RNA that contains a riboswitch that regulates splicing by activating, deactivating or blocking the riboswitch. The riboswitch can regulate, for example, alternative spicing of the RNA. If the gene is essential for survival of a cell or organism that harbors it, activating, deactivating or blocking the riboswitch can result in death, stasis or debilitation of the cell or organism. For example, activating a naturally occurring riboswitch in a naturally occurring gene that is essential to survival of a microorganism can result in death of the microorganism (if activation of the riboswitch controls alternative splicing, which in turn up-regulates or down-regulates a crucial protein). This is one basis for the use of the disclosed compounds and methods for antimicrobial and antifungal effects. The compounds that have these antimicrobial effects are considered to be bacteriostatic or bacteriocidal, or fungicidal.

Also disclosed are methods for selecting and identifying compounds that can activate, deactivate or block a riboswitch that regulates splicing. The riboswitch can regulate, for example, alternative spicing. Activation of a riboswitch refers to the change in state of the riboswitch upon binding of a trigger molecule. A riboswitch can be activated by compounds other than the trigger molecule and in ways other than binding of a trigger molecule. The term trigger molecule is used herein to refer to molecules and compounds that can activate a riboswitch. This includes the natural or normal trigger molecule for the riboswitch and other compounds that can activate the riboswitch. Natural or normal trigger molecules are the trigger molecule for a given riboswitch in nature or, in the case of some non-natural riboswitches, the trigger molecule for which the riboswitch was designed or with which the riboswitch was selected (as in, for example, in *vitro* selection or *in vitro* evolution techniques). Non-natural trigger molecules can be referred to as non-natural trigger molecules.

Also disclosed is a method of inhibiting fungal growth, the method comprising: identifying a subject with a fungal infection; administering to the subject an effective amount of a compound that inhibits a TPP-responsive riboswitch, thereby inhibiting fungal growth. Inhibiting fungal growth can comprise a 10, 20, 30, 40, 50, 60, 70, 80, 90, or 100% reduction in fungal biomass.

Also disclosed are methods of identifying compounds that activate, deactivate or block a riboswitch that regulates splicing. For example, compounds that activate a riboswitch can be identified by bringing into contact a test compound and a riboswitch and assessing activation of the riboswitch by either measuring the alternative splicing of the gene product, or measuring the differential level of the protein expressed as a result of the splicing event. If the riboswitch is activated, the test compound is identified as a compound that activates the riboswitch. Activation of a riboswitch can be assessed in any suitable manner. For example, the riboswitch can be linked to a reporter RNA and expression, expression level, or change in expression level of the reporter RNA can be measured in the presence and absence of the test compound. As another example, the riboswitch can include a conformation dependent label, the signal from which changes depending on the activation state of the riboswitch. Such a riboswitch preferably uses an aptamer domain from or derived from a naturally occurring riboswitch. As can be seen, assessment of activation of a riboswitch can be performed with the use of a control assay or measurement or without the use of a control assay or measurement. Methods for identifying compounds that deactivate a riboswitch can be performed in analogous ways.

In addition to the methods disclosed elsewhere herein, identification of compounds that block a riboswitch that regulates splicing can be accomplished in any suitable manner. For example, an assay can be performed for assessing activation or deactivation of a riboswitch in the presence of a compound known to activate or deactivate the riboswitch and in the presence of a test compound. If activation or deactivation is not observed as would be observed in the absence of the test compound, then the test compound is identified as a compound that blocks activation or deactivation of the riboswitch.

Also disclosed are methods of detecting compounds using biosensor riboswitches that regulate alternative splicing. The method can include bringing into contact a test sample and a biosensor riboswitch and assessing the activation of the biosensor riboswitch. Activation of the biosensor riboswitch indicates the presence of the trigger molecule for the biosensor riboswitch in the test sample. Biosensor riboswitches are engineered riboswitches that produce a detectable signal in the presence of their cognate trigger molecule. Useful biosensor riboswitches can be triggered at or above threshold levels of the trigger molecules. Biosensor riboswitches can be designed for use *in vivo* or *in vitro.* For example, biosensor riboswitches that regulate alternative binding can be operably linked to a reporter RNA that encodes a protein that serves as or is involved in producing a signal that can be used *in vivo* by engineering a cell or organism to harbor a nucleic acid construct encoding the riboswitch/reporter RNA. An example of a biosensor riboswitch for use *in vitro* is riboswitch that includes a conformation dependent label, the signal from which changes depending on the activation state of the riboswitch. Such a biosensor riboswitch preferably uses an aptamer domain from or derived from a naturally occurring TPP riboswitch.

Also disclosed are compounds made by identifying a compound that activates, deactivates or blocks a riboswitch and manufacturing the identified compound. This can be accomplished by, for example, combining compound identification methods as disclosed elsewhere herein with methods for manufacturing the identified compounds. For example, compounds can be made by bringing into contact a test compound and a riboswitch, assessing activation of the riboswitch, and, if the riboswitch is activated by the test compound, manufacturing the test compound that activates the riboswitch as the compound.

Also disclosed are compounds made by checking activation, deactivation or blocking of a riboswitch by a compound and manufacturing the checked compound. This can be accomplished by, for example, combining compound activation, deactivation or blocking assessment methods as disclosed elsewhere herein with methods for manufacturing the checked compounds. For example, compounds can be made by bringing into contact a test compound and a riboswitch, assessing activation of the riboswitch, and, if the riboswitch is activated by the test compound, manufacturing the test compound that activates the riboswitch as the compound. Checking compounds for their ability to activate, deactivate or block a riboswitch refers to both identification of compounds previously unknown to activate, deactivate or block a riboswitch and to assessing the ability of a compound to activate, deactivate or block a riboswitch where the compound was already known to activate, deactivate or block the riboswitch.

### A. Identification of Antifungal Compounds

A compound can be identified as activating a riboswitch or can be determined to have riboswitch activating activity if the signal in a riboswitch assay is increased in the presence of the compound by at least 1 fold, 2 fold, 3 fold, 4 fold, 5 fold, 50%, 75%, 100%, 125%, 150%, 175%, 200%, 250%, 300%, 400%, or 500% compared to the same riboswitch assay in the absence of the compound (that is, compared to a control assay). The riboswitch assay can be performed using any suitable riboswitch construct. Riboswitch constructs that are particularly useful for riboswitch activation assays are described elsewhere herein. The identification of a compound as activating a riboswitch or as having a riboswitch activation activity can be made in terms of one or more particular riboswitches, riboswitch constructs or classes of riboswitches. For convenience, compounds identified as activating a riboswitch that controls alternative splicing can be so identified for particular riboswitches.

### B. Methods of Using Antifungal Compounds

Disclosed herein are *in vivo* and *in vitro* anti-fungal methods. By "anti-fungal" is meant inhibiting or preventing fungal growth, killing fungi, or reducing the number of fungi. Thus, disclosed is a method of inhibiting or preventing fungal growth comprising contacting a fungus with an effective amount of one or more compounds disclosed herein. Additional structures for the disclosed compounds are provided herein.

Disclosed is a method of inhibiting fungal cell growth, the method comprising: bringing into contact a cell and a compound that binds a TPP-responsive riboswitch, wherein the cell comprises a gene encoding an RNA comprising a TPP-responsive riboswitch, wherein the compound inhibits bacterial cell growth by binding to the TPP-responsive riboswitch, thereby limiting TPP production. This method can yield at least a 10% decrease in bacterial cell growth compared to a cell that is not in contact with the compound. The compound and the cell can be brought into contact by administering the compound to a subject. The cell can be a fungal cell in the subject, wherein the compound kills or inhibits the growth of the fungal cell.The subject can have a fungal infection. The compound can be administered in combination with another fungal compound.

The fungus can be selected from the group comprising: *Absidia coerulea, Absidia glauca, Absidia corymbifera, Acremonium strictum, Alternaria alternata, Apophysomyces elegans, Saksena vasiformis, Aspergillus flavus, Aspergillus oryzae, Aspergillus fumigatus, Neosartoryta fischeri, Aspergillus niger, Aspergillus foetidus, Aspergillus phoenicus, Aspergillus nomius, Aspergillus ochraceus, Aspergillus ostianus, Aspergillus auricomus, Aspergillus parasiticus, Aspergillus sojae, Aspergillus restrictus, Aspergillus caesillus, Aspergillus conicus, Aspergillus sydowii, Aspergillus tamarii, Aspergillus terreus, Aspergillus ustus, Aspergillus versicolor, Aspergillus ustus, Aspergillus versicolor, Chaetomium globosum, Cladosporium cladosporioides, Cladosporium herbarum, Cladosporium sphaerospermum, Conidiobolus coronatus, Conidiobolus incongruus, Cunninghamella elegans, Emericella nidulans, Emericella rugulosa, Emericilla quadrilineata, Apicoccum nigrum, Eurotium amstelodami, Eurotium chevalieri, Eurotium herbariorum, Eurotium rubrum, Eurotium repens, Geotrichum candidum, Geotrichum klebahnii, Memnoniella echinata, Mortierella polycephala, Mortierella wolfii, Mucor mucedo, Mucor amphibiorum, Mucor circinelloides, Mucor heimalis, Mucor indicus, Mucor racemosus, Mucor ramosissimus, Rhizopus azygosporous, Rhizopus homothalicus, Rhizopus rnicrosporus, Rhizopus oligosporus, Rhizopus oryzae, Myrothecium verrucaria, Myrothecium roridum, Paecilomyces lilacinus, Paecilomyces variotii, Penicillium freii, Penicillium verrucosum, Penicillium hirsutum, Penicillium alberechii, Penicillum aurantiogriseum, Penicillium polonicum, Penicillium viridicatum, Penicillium hirsutum, Penicillium brevicompactum, Penicillium chrysogenum, Penicillium griseofulvum, Penicillium glandicola, Penicillium coprophilum, Eupenicillium crustaceum, Eupenicillium egyptiacum, Penicillium crustosum, Penicillium citrinum, Penicillium sartoryi, Penicillium westlingi, Penicillium corylophilum, Penicillium decumbens, Penicillium echinulatum, Penicillium solitum, Penicillium camembertii, Penicillium commune, Penicillium echinulatum, Penicillium sclerotigenum, Penicillium italicum, Penicillium expansum, Penicillium fellutanum, Penicillium charlesii, Penicillium janthinellum, Penicillium raperi, Penicillium madriti, Penicillium gladioli, Penicillium oxalicum, Penicillium roquefortii, Penicillium simplicissimum, Penicillium ochrochloron, Penicillium spinulosum, Penicillium glabrum, Penicillum thomii, Penicillium pupurescens, Eupenicillium lapidosum, Rhizomucor miehei, Rhizomucor pusillus, Rhizomucor variabilis, Rhizopus stolonifer, Scopulariopsis asperula, Scopulariopsis brevicaulis, Scopulariopsis fusca, Scopulariopsis brumptii, Scopulariopsis chartarum, Scopulariopsis sphaerospora, Trichoderma asperellum, Trichoderma hamatum, Trichoderma viride, Trichoderma harzianum, Trichoderma longibrachiatum, Trichoderma citroviride, Trichoderma atroviride, Trichoderma koningii, Ulocladium atrum, Ulocladium chartarum, Ulocladium botrytis, Wallemia sebi, Stachybotrys chartarum,* for example. Fungal growth can also be inhibited in any context in which fungi are found. For example, fungi growth in fluids, biofilms, and on surfaces can be inhibited. The compounds disclosed herein can be administered or used in combination with any other compound or composition. For example, the disclosed compounds can be administered or used in combination with another antifungal compound.

"Inhibiting fungal growth" is defined as reducing the ability of a single fungus to divide into daughter cells, or reducing the ability of a population of fungus to form daughter cells. The ability of the fungus to reproduce can be reduced by about 10%, about 20%, about 30%, about 40%, about 50%, about 60%, about 70%, about 80%, about 90%, or 100% or more.

Also provided is a method of inhibiting the growth of and/or killing a fungus or population of fungi comprising contacting the fungus with one or more of the compounds disclosed and described herein.

"Killing a fungus" is defined as causing the death of a single fungi, or reducing the number of a plurality of fungi, such as those in a colony. When the fungi are referred to in the plural form, the "killing of fungus" is defined as cell death of a given population of fungi at the rate of 10% of the population, 20% of the population, 30% of the population, 40% of the population, 50% of the population, 60% of the population, 70% of the population, 80% of the population, 90% of the population, or less than or equal to 100% of the population.

The compounds and compositions disclosed herein have anti-fungal activity *in vitro* or *in vivo,* and can be used in conjunction with other compounds or compositions, which can be fungicidal as well.

By the term "therapeutically effective amount" of a compound as provided herein is meant a nontoxic but sufficient amount of the compound to provide the desired reduction in one or more symptoms. As will be pointed out below, the exact amount of the compound required will vary from subject to subject, depending on the species, age, and general condition of the subject, the severity of the disease that is being treated, the particular compound used, its mode of administration, and the like. Thus, it is not possible to specify an exact "effective amount." However, an appropriate effective amount may be determined by one of ordinary skill in the art using only routine experimentation.

The compositions and compounds disclosed herein can be administered *in vivo* in a pharmaceutically acceptable carrier. By "pharmaceutically acceptable" is meant a material that is not biologically or otherwise undesirable, i.e., the material may be administered to a subject without causing any undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art.

The compositions or compounds disclosed herein can be administered orally, parenterally (e.g., intravenously), by intramuscular injection, by intraperitoneal injection, transdermally, extracorporeally, topically or the like, including topical intranasal administration or administration by inhalant. As used herein, "topical intranasal administration" means delivery of the compositions into the nose and nasal passages through one or both of the nares and can comprise delivery by a spraying mechanism or droplet mechanism, or through aerosolization of the nucleic acid or vector. Administration of the compositions by inhalant can be through the nose or mouth via delivery by a spraying or droplet mechanism. Delivery can also be directly to any area of the respiratory system (e.g., lungs) via intubation. The exact amount of the compositions required will vary from subject to subject, depending on the species, age, weight and general condition of the subject, the severity of the allergic disorder being treated, the particular nucleic acid or vector used, its mode of administration and the like. Thus, it is not possible to specify an exact amount for every composition. However, an appropriate amount can be determined by one of ordinary skill in the art using only routine experimentation given the teachings herein.

Parenteral administration of the composition or compounds, if used, is generally characterized by injection. Injectables can be prepared in conventional forms, either as liquid solutions or suspensions, solid forms suitable for solution of suspension in liquid prior to injection, or as emulsions. A more recently revised approach for parenteral administration involves use of a slow release or sustained release system such that a constant dosage is maintained. See, e.g., U.S. Patent No. 3,610,795, which is incorporated by reference herein.

The compositions and compounds disclosed herein can be used therapeutically in combination with a pharmaceutically acceptable carrier. Suitable carriers and their formulations are described in Remington: The Science and Practice of Pharmacy (19th ed.) ed. A.R. Gennaro, Mack Publishing Company, Easton, PA 1995. Typically, an appropriate amount of a pharmaceutically-acceptable salt is used in the formulation to render the formulation isotonic. Examples of the pharmaceutically-acceptable carrier include, but are not limited to, saline, Ringer's solution and dextrose solution. The pH of the solution is preferably from about 5 to about 8, and more preferably from about 7 to about 7.5. Further carriers include sustained release preparations such as semipermeable matrices of solid hydrophobic polymers containing the antibody, which matrices are in the form of shaped articles, e.g., films, liposomes or microparticles. It will be apparent to those persons skilled in the art that certain carriers may be more preferable depending upon, for instance, the route of administration and concentration of composition being administered.

Pharmaceutical carriers are known to those skilled in the art. These most typically would be standard carriers for administration of drugs to humans, including solutions such as sterile water, saline, and buffered solutions at physiological pH. The compositions can be administered intramuscularly or subcutaneously. Other compounds will be administered according to standard procedures used by those skilled in the art.

Pharmaceutical compositions may include carriers, thickeners, diluents, buffers, preservatives, surface active agents and the like in addition to the molecule of choice. Pharmaceutical compositions may also include one or more active ingredients such as antimicrobial agents, antiinflammatory agents, anesthetics, and the like.

The pharmaceutical composition may be administered in a number of ways depending on whether local or systemic treatment is desired, and on the area to be treated. Administration may be topically (including ophthalmically, vaginally, rectally, intranasally), orally, by inhalation, or parenterally, for example by intravenous drip, subcutaneous, intraperitoneal or intramuscular injection. The disclosed antibodies can be administered intravenously, intraperitoneally, intramuscularly, subcutaneously, intracavity, or transdermally.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

Formulations for topical administration may include ointments, lotions, creams, gels, drops, suppositories, sprays, liquids and powders. Conventional pharmaceutical carriers, aqueous, powder or oily bases, thickeners and the like may be necessary or desirable.

Compositions for oral administration include powders or granules, suspensions or solutions in water or non-aqueous media, capsules, sachets, or tablets. Thickeners, flavorings, diluents, emulsifiers, dispersing aids or binders may be desirable.

Some of the compositions may potentially be administered as a pharmaceutically acceptable acid- or base- addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-,di-, trialkyl and aryl amines and substituted ethanolamines.

Therapeutic compositions as disclosed herein may also be delivered by the use of monoclonal antibodies as individual carriers to which the compound molecules are coupled. The therapeutic compositions of the present disclosure may also be coupled with soluble polymers as targetable drug carriers. Such polymers can include, but are not limited to, polyvinyl-pyrrolidone, pyran copolymer, polyhydroxypropylmethacrylamidephenol, polyhydroxyethylaspartamidephenol, or polyethyl-eneoxidepolylysine substituted with palmitoyl residues. Furthermore, the therapeutic compositions of the present disclosure may be coupled to a class of biodegradable polymers useful in achieving controlled release of a drug, for example, polylactic acid, polyepsilon caprolactone, polyhydroxy butyric acid, polyorthoesters, polyacetals, polydihydro-pyrans, polycyanoacrylates and cross-linked or amphipathic block copolymers of hydrogels.

Preferably at least about 3%, more preferably about 10%, more preferably about 20%, more preferably about 30%, more preferably about 50%, more preferably 75% and even more preferably about 100% of the fungal infection is reduced due to the administration of the compound. A reduction in the infection is determined by such parameters as reduced white blood cell count, reduced fever, reduced inflammation, reduced number of fungi, or reduction in other indicators of fungal infection. To increase the percentage of fungal infection reduction, the dosage can increase to the most effective level that remains non-toxic to the subject.

As used throughout, "subject" refers to an individual. Preferably, the subject is a mammal such as a non-human mammal or a primate, and, more preferably, a human. "Subjects" can include domesticated animals (such as cats, dogs, etc.), livestock (e.g., cattle, horses, pigs, sheep, goats, etc.), laboratory animals (e.g., mouse, rabbit, rat, guinea pig, etc.) and fish.

A "fungal infection" is defined as the presence of fungi in a subject or sample. Such fungi can be an outgrowth of naturally occurring fungi in or on the subject or sample, or can be due to the invasion of a foreign organism.

### Examples

### Example 1: Control of Alternative RNA Splicing and Gene Expression by Eukaryotic Riboswitches

A previous study (Kubodera et al. (2003)) of the fungus Aspergillus oryzae thiA mRNA carrying a TPP aptamer revealed that thiamine (vitamin B1) supplementation of growth medium results in reduced gene expression, and that deletion of riboswitch aptamer portions disrupts thiamine responsiveness. These findings show that thiamine enters cells, is phosphorylated to generate TPP, and the resulting coenzyme serves as a ligand for riboswitch-mediated control of RNA splicing in fungi (Sudarsan et al. (2003)). In this study, the functions of TPP aptamers (Figure 5) were examined as present in three genes in *N. crassa.* Two of these genes, NMT1 (Fig. 1a) and CyPBP37 (Fig. 1b; a homolog of THI4 as termed hereafter), are known to be thiamine metabolism genes (McColl et al. (2003), Faou & Tropschug (2003), Faou Tropschug (2004)). A third gene, NCU01977.1 (Fig. 1c), codes for a protein of unknown function.

The examination focused primarily on the NMT1 gene, which is known to be repressed by excess thiamine in *N. crassa* (McColl et al. (2003)) and in *Schizosaccharomyces pombe* (Maundrell (1989)). All three precursor mRNAs carry the riboswitch in an intron residing near the 5' terminus (Fig. 1, Figure 6). Reverse transcription and polymerase chain reaction (RT-PCR) methods were used to establish the relative amounts and the nucleotide sequences of the 5' ends of the transcripts produced when *N. crassa* was grown in the absence or presence of thiamine (Fig. 1d). Cloning and sequencing of RNA products revealed the presence of precursor transcripts that matched the sequence of the genomic DNA, and of other sequences that are consistent with the RNA splicing products depicted in Fig. 1. The results confirm that thiamine causes alternative splicing of the NMT1 and THI4 precursor mRNAs, and causes an increase in splicing of the NCU01977.1 precursor mRNA. Thiamine does not affect splicing of an RNA that does not carry the TPP riboswitch (Figure 7).

TPP binding by NMT1 RNA constructs was confirmed by in-line probing (Soukup & Breaker (1999)) (Figure 8 and 9). Nucleotides that exhibit major structural modulations are known to be involved in the binding of TPP (Thore et al. (2006), Serganov et al. (2006),Edwards & Ferré-D'Amaré (2006)), and the apparent dissociation constant (KD) of∼300 pM measured for both constructs is similar to those exhibited by bacterial TPP riboswitches (Winkler et al. (2002), Welz & Breaker (2007)). Thiamine control of NMT1 RNA alternative splicing was further investigated by using RT-PCR to establish transcript types and amounts isolated from *N. crassa* grown in minimal medium and sampled at various times after thiamine supplementation (Fig. 2a). In the absence of added thiamine (t = 0 min), transcripts are processed to yield splicing product I-3. Within the first hour after thiamine supplementation, the splicing product I-2 and the unspliced NMT1 precursor RNA I-1 appear. Within four hours, I-3 is almost completely replaced by I-2 and I-1. These results show that the precipitous decrease of I-3 after the addition of thiamine is responsible for decreased NMT1 expression.

Constructs carrying the NMT1 5' UTR or its variants (Fig. 2b) with the start codon of the main ORF fused in frame with a luciferase (LUC) reporter gene were used to assess the importance of the TPP aptamer for gene control. Substantial repression of the wild-type (WT) LUC reporter construct occurs with *N. crassa* grown overnight in medium supplemented with 30 µM thiamine (Fig. 2c, Top). Moreover, the RT-PCR products derived from the reporter construct and the native NMT1 mRNAs are equivalent (Fig. 2c, Bottom). Most mutant constructs exhibit a two- to four-fold increase in reporter activity compared to WT when cells are grown in thiamine-free medium. Mutations that weaken TPP binding affinity possibly also eliminate the modest level of gene repression caused by synthesis of TPP in cells growing in minimal medium.

Mutations in stems P1 through P5 that disrupt and subsequently restore base pairing within the aptamer (constructs M1 through M9, Fig. 2b) mostly yield gene regulation characteristics that correlate with the ability of the RNAs to bind TPP. Although the extended portion of P3 is disrupted in M5, this change occurs outside the TPP-binding core of the aptamer and has little effect on gene control. In addition, the majority of the extended P3 stem can be deleted (M10, analogous to 115 NMT1 RNA in Figure 8) without complete loss of function. Mutations in M1 cause a dramatic decrease of LUC activity and the typical mRNA products are not detected (Fig. 2c), indicating that some nucleotides and structures within the aptamer can influence mRNA transcription or processing in addition to their role in binding TPP.

The most revealing results were found with M9, which carries compensatory mutations to the disruptive mutations in the P5 stem of M8. M9 exhibits only partial restoration of thiamine-dependent gene control, but at a level of expression that is far below WT or other compensation mutants (Fig. 2c). These unusual characteristics of M9 are consistent with a mechanism whereby nucleotides within P5 participate in the control of alternative splicing (see below).

The effects of most mutations on thiamine regulation led us to speculate that unspliced or alternatively spliced RNAs are inactive due to the presence of start codons upstream of the main ORF (Fig. 1a). This hypothesis was tested by examining additional LUC reporter constructs fused downstream of WT or mutant versions of the three types of NMT1 5' UTRs (Fig. 3a). The resulting construct I-3R, which lacks upstream start codons and mimics the short (or fully) spliced mRNA that predominates in the absence of added thiamine, yields robust reporter activity (Fig. 3b). In contrast, the analogous I-2R construct exhibits almost no reporter activity, which is consistent with the natural production of this splice variant when thiamine is present and gene expression is reduced (Fig. 2c). The levels of LUC expression with constructs I-2R and I-3R are unchanged by the addition of thiamine, as expected since the TPP riboswitch is absent.

Disruption of the first (M11), second (M12) or both (M13) start codons in the alternatively spliced I-2 construct (Fig. 3a) upstream of the main NMT1 ORF results in constructs that yield progressively more reporter expression. It has been observed that short upstream ORFs (uORFs) in the 5' UTR of fungal genes decrease expression of the main ORF (Vilela & McCarthy (2003)). Therefore, restoration of LUC expression upon disruption of both uORF start codons in I-2 is consistent with the hypothesis that uORF translation is responsible for reduced expression of the main NMT1 ORF.

Transcripts carrying mutations (Fig. 3a) at the first 5' splice site (M14), the splicing branch site (M15) or the 3' splice site (M16) result in uniformly low reporter expression (Fig. 3b, Top). RT-PCR analysis revealed that M14 yields I-2R RNA splicing product, while M15 and M16 do not undergo splicing (Fig. 3b, Bottom). These findings demonstrate that proper splicing is required to remove uORFs and permit main ORF expression.

For many bacterial riboswitches, metabolite binding alters folding of the expression platform located downstream of the aptamer without involving proteins (Winkler et al. (2002), Mironov et al. (2002), Serganov et al. (2006)). To assess whether splicing regulation by the NMT1 TPP riboswitch is due to protein-independent structural modulation of the aptamer flanks, NMT1 UTR constructs were subjected to in-line probing (Soukup & Breaker (1999)). Interestingly, the addition of TPP causes nucleotides at the branch site to become more structured (Figure 10), and yields a more flexible structure at the second 5' splice site (Fig. 4a). Furthermore, it was observed that 12 nucleotides of the P4 and P5 elements of the aptamer are complementary to most of the nucleotides at the second 5' splice site that are structurally sequestered when ligand is absent (Fig. 4b). The P4 and P5 elements are required for recognition of the pyrophosphate moiety of TPP and, therefore, TPP binding and 5' splice site occlusion are mutually exclusive.

The unusual characteristics of construct M9 in the in vivo reporter assays are consistent with this model for riboswitch function. In-line probing confirms that the M9 mutations disrupt base pairing between aptamer and the second 5' splice site (Figure 11), and this structural defect is expected to favor the observed production of long spliced mRNA and the loss of reporter expression (Fig. 2c). Moreover, similar alternative base pairing potential exists for all TPP riboswitches associated with NMT1 genes from other fungal species (Figure 12), indicating that this conserved alternative secondary structure is an important feature of the TPP riboswitch expression platform. It has been demonstrated that, when presented with two 5' splice sites, the spliceosome from the fungus *S. pombe* greatly prefers using the site proximal to the 3' splice site (Romfo et al. (2000)). Given this 5' splice site preference, the TPP riboswitches in NMT1 mRNAs can maintain complete control over the distribution of alternative splicing products simply by modulating base pairing between the P4-P5 aptamer region and the second 5' splice site. TPP riboswitches in other fungal genes appear to use different mechanisms for gene control (Figrue 13).

The data is consistent with a mechanism for TPP riboswitch-mediated splicing regulation wherein metabolite binding alters the availability of alternative splice site and branch site components of the intron (Fig. 4c). When TPP concentration is low, the newly transcribed mRNA adopts a structure that occludes the second 5' splice site, while leaving the branch site available for splicing. Pre-mRNA splicing from the first 5' splice site leads to production of the I-3 form of mRNA and expression of the NMT1 protein. When TPP concentration is high, ligand binding to the TPP aptamer causes allosteric changes in RNA folding to increase the structural flexibility near the second 5' splice site and to occlude nucleotides near the branch site. The combined effect of these changes is a reduction in splicing efficiency of the I-1 mRNA and a redirection of those that do undergo processing to yield the alternatively spliced I-2 mRNA. Both I-1 and I-2 carry uORFs that compete with the translation of the main ORF and repress NMT1 expression.

The involvement of alternative splicing in eukaryotic gene control is becoming increasingly apparent (Matlin et al. (2005), Blencowe (2006)), and these findings reveal how riboswitches can modulate splicing efficiency and splice site choice without requiring protein factors. Given the enormous diversity of RNA folding possibilities, structured RNA domains are likely to be widely used to control splicing (Buratti & Baralle (2004)) through the direct read-out of physical changes such as temperature (Colot et al. (2005)) or changes in metabolite concentrations (Sudarsan et al. (2003), Kubodera et al. (2003), Borsuk et al. (2007)). Furthermore, an example of ligand-mediated control of splicing using an engineered aptamer has recently been reported (Kim et al. (2005)), which demonstrates that direct ligand-mRNA interactions can be harnessed for gene control applications. Observations with fungal TPP riboswitches further reveal the versatility of riboswitches from separate domains of life and hint at the possible involvement of undiscovered riboswitch classes in other gene control processes.

### METHODS SUMMARY

*Oligonucleotides and Chemicals.* RNAs were synthesized, synthetic DNAs (Figure 14) and reagents were purchased, and DNA constructs were created as noted in detailed METHODS.

*RNA Analyses.* RT-PCR analyses were conducted using RNA from untransformed N. crassa inoculated into 100 ml of Vogel's minimal medium supplemented with 0.5 mg ml-1 L-histidine. Cultures were grown at 30°C with shaking at 150 rpm for 24 h either in the absence or presence of supplemented 30 µM thiamine. The cDNA was used as a template for PCR amplification of the 5' regions of the three genes using primers and methods as described in Supplementary Information. All splicing products were confirmed by cloning and sequencing.

*Reporter Gene Assays.* Transformation of N. crassa was conducted using electroporation of freshly suspended macroconidia and insertion of the target gene was verified by PCR with insert specific primers from genomic DNA. Transcription of luciferase reporter constructs was constitutively driven by the N. crassa beta-tubulin (BTUB) promoter inserted upstream of the NMT1 5' UTR. N. crassa was grown overnight at 30°C in 2% glucose minimal medium in the absence or presence of 30 µM thiamine. Samples were isolated and assayed for luciferase activity as described below.

### METHODS

*Bioinformatics Searches and Fungal TPP Riboswitches.* We examined the "fungi" division of the RefSeq database (version 13) using covariance model searches with manually curated seed sequence alignments adapted from known TPP riboswitch representatives. Covariance models (Eddy et al. (1994)) were created using the infernal software package (Eddy, S. R, Department of Genetics, Washington University School of Medicine. St. Louis, Missouri)) (version 0.55). See also Supplementary Information for additional details.

*DNA oligonucleotides and chemicals.* Synthetic DNAs were purchased from the HHMI Keck Foundation Biotechnology Resource Center at Yale University. TPP, thiamine, sodium iodoacetate (IAA) and L-histidine were purchased from Sigma-Aldrich. [γ-32P]ATP was purchased from Amersham Pharmacia.

*In vitro transcription.* DNA templates were produced by PCR amplification from genomic DNA of N. crassa using primers designed to introduce a T7 promoter into the construct. The sequence CC was added to the template strand transcription start site to promote efficient in vitro transcription, thus producing RNAs that carry GG at their 5' terminus. RNAs were prepared using a RiboMax Transcription Kit (Promega) according to the manufacturer's directions. RNAs were purified by denaturing polyacrylamide gel electrophoresis (PAGE), and 5' 32P-labeled as described previously (Seetharaman et al. (2001)).

*In-line probing of RNA constructs.* 5' 32P-labeled RNAs were incubated at 23°C for 40 hours in 50 mM Tris-HCl (pH 8.3 at 25°C), 20 mM MgC12 and 100 mM KCl in the presence or absence of TPP as defined. Cleavage products were separated by denaturing 10% PAGE, visualized by PhosphorImager (GE Healthcare), and quantified using ImageQuant software. KD values were determined by plotting the normalized fraction of RNA cleaved versus the logarithm of ligand concentration used.

*Strains, plasmids and media.* N. crassa 87-74 (bd; frq+ a; his-3) (Froehlich et al. (2003)) was used as a host strain for transformation. The plasmid pLL07 (kindly provided by the laboratory of J.C. Dunlap) (Mehra et al. (2002)), which carries a firefly luciferase (LUC) reporter gene, was used for reporter gene construction. The start codon for the LUC reporter gene in pLL07 was removed by QuikChange (Stratagene) site directed mutagenesis to obtain the plasmid pLL09.

The promoter for the beta-tubulin gene in N. crassa ranging from positions 1 to 355 (accession number M13630) (Orbach et al. (1986)) was amplified from genomic DNA by PCR using primers DNA3 and DNA4. The amplified DNA fragment was digested with MfeI and EcoRI and inserted into the EcoRI site of pLL09 to obtain pLUC. The sequence of pLUC was confirmed by sequencing (HHMI Keck Foundation Biotechnology Resource Center at Yale University). E. coli Top 10 cells (Invitrogen) were used as a host during manipulation of plasmids.

Cloning of the 5' UTR of the NMT1 gene (accession number AY007661) was achieved by PCR amplification of a 378 bp fragment (beginning with the annotated transcription start site) from genomic DNA of N. crassa with primers DNA5 and DNA6. The resulting wild-type (WT) PCR DNA was first cloned using a TOPO TA cloning kit (Invitrogen). The NMT1 fragment was released from the vector by EcoRI and XbaI restriction enzyme digestion and cloned into appropriate sites of pLUC.

For generation of the aptamer mutants M1 through M9 and the splice site mutants M14 through M16, PCR mutagenesis was performed on the WT NMT1 containing TOPO vector (see primer list). After confirmation of mutagenesis by DNA sequencing, each mutant NMT1 fragment was cloned into EcoRI/XbaI sites of pLUC. For cloning of the P3 deletion construct (M10) NMT1 was amplified in two fragments with primers DNA5/DNA25 and DNA26/DNA6, in which the overlapping region deletes much of the natural P3a stem. These two fragments were used as a template in a subsequent PCR with the outer primers DNA5 and DNA6. The resulting fragment was digested with EcoRI and XbaI and cloned into pLUC. Preparation of the I-2R and I-3R constructs and variants of NMT1 was achieved by RT-PCR amplification of these two alternatively spliced products with primers DNA5 and DNA6. The resulting PCR products were cloned and mutated as described above.

To generate a NCU01977.15' region-LUC reporter fusion (Fig. 13), a 478 nucleotide fragment starting 94 nucleotides upstream of the predicted start codon was amplified from genomic DNA of N. crassa with primers DNA41 and DNA42 and cloned into EcoRI/XbaI sites of pLUC as described above. The integrity of all constructs was confirmed by sequencing (HHMI Keck Foundation Biotechnology Resource Center at Yale University). In all constructs, the original start codon of the main ORF (NMT1 or NCU01977.1) was fused in frame with the LUC reporter sequence.

Standard liquid medium used for growth of N. crassa contained 2% glucose, 0.5% L-arginine, 1 X Vogel's minimal medium, and 50 ng/ml biotin. Solid medium used for N. crassa growth (slants) contained 1 X Vogel's minimal medium, 2% sucrose and 1.5% agar. Medium used for selection of N. crassa transformants contained 1X Vogel's minimal medium, 2% agar, 2% L-sorbose, 0.05% fructose and 0.05% glucose. For homokaryon isolation, 0.1 X Westergaard's medium containing 1% IAA was used.

*N. crassa transformations and reporter assays.* Transformation of N. crassa was conducted using electroporation of freshly suspended macroconidia as previously described (Davis (2000), Loros. & Dunlap (1991), Vann (1995)). Insertion of the target gene was verified by PCR with insert specific primers from genomic DNA. Homokaryotic strains were isolated as previously described (Ebbole & Sachs (1990)).

*Luciferase reporter gene assays.* Mycelia from N. crassa transformed with LUC reporter constructs were isolated by filtration and approximately 100 mg of tissue was ground to a fine powder. After addition of 100 µl 1 X Passive Lysis Buffer (Promega), the samples were vigorously mixed and incubated on ice for 30 min followed by centrifugation for 15 minutes at 13,000 g. Luciferase activity was determined for the resulting supernatant using the Luciferase Assay System (Promega) and a plate-reading luminometer (Wallac). Luciferase activity was normalized over total protein concentration of the extract as determined by Bradford Protein Assay (BioRad) and finally expressed relative to a reference construct. Luciferase background activity (untransformed N. crassa) was 0.05% relative to the wild-type NMT1 construct in cells grown without added thiamine.

*Reverse transcriptase-polymerase chain reaction (RT-PCR) analyses.* Total RNA was isolated from mycelia using TRIzol® LS reagent (Invitrogen) according to the manufacturer's directions. 5 µg of total RNA was treated with RNase free DNase I (Promega) for 30 min at 37°C. cDNA was generated by reverse transcription with a polyT primer for 1 hr at 42°C using SuperScriptTM II Reverse Transcriptase (Invitrogen) according to the manufacturer's directions. To exclude the possibility of amplification products originating from contamination with genomic DNA, control reactions using RNA preparations before RT were performed and, for NMT1, a reverse primer spanning an exon-exon border in the coding region was used.

Bioinformatics Searches and Fungal TPP Riboswitches. The "fungi" division of the RefSeq database (version 13) was examined using covariance model searches with manually curated seed sequence alignments adapted from known TPP riboswitch representatives. Covariance models (Eddy 1994) were created using the INFERNAL software package (version 0.55).

To verify that known riboswitch sequences were being recovered, the final results were compared to a list of TPP riboswitches compiled through an exhaustive comparative genomics analysis of thiamine metabolic genes. This approach successfully identified every riboswitch that had been previously found in microbial species. In addition, TPP riboswitches associated with 23 genes from 11 species of filamentous fungi were identified (Fig. 5).

Several of the riboswitch-associated genes identified in fungi by this approach are known to be involved in thiamine metabolism. Based on the amino acid sequence of their respective ORFs, it was also found that most previously uncharacterized genes in this list are homologs of thiamine metabolic proteins. The TPP aptamers found in fungi are mostly identical to their eubacterial homologs, which is consistent with functional conservation. However, the fungal TPP aptamer representatives have two distinct differences. The first is the consistent absence of P3a stem, which is sometimes present in eubacterial representatives but is not necessary for TPP binding by the aptamer. The second is the considerable heterogeneity in the length of the P3 stem in filamentous fungi, which ranges from 4 to 83 base pairs. The locations of the three TPP riboswitches in *N. crassa* are depicted in Figure 6.

*DNA oligonucleotides and chemicals.* Synthetic DNAs were purchased from the HHMI Keck Foundation Biotechnology Resource Center at Yale University. TPP, thiamine, sodium iodoacetate (IAA) and L-histidine were purchased from Sigma-Aldrich. [γ-³²P]ATP was purchased from Amersham Pharmacia.

*In vitro transcription.* DNA templates were produced by PCR amplification from genomic DNA of *N. crassa* using primers designed to introduce a T7 promoter into the construct. The sequence CC was added to the template strand transcription start site to promote efficient *in vitro* transcription, thus producing RNAs that carry GG at their 5' terminus. RNAs were prepared using a RiboMax Transcription Kit (Promega) according to the manufacturer's directions. RNAs were purified by denaturing polyacrylamide gel electrophoresis (PAGE), and 5' ³²P-labeled as described previously (Seetharaman 2001).

*In-line probing ofRNA constructs.* 5' ³²P-labeled RNAs were incubated at 23°C for 40 hours in 50 mM Tris-HCl (pH 8.3 at 25°C), 20 mM MgCl₂ and 100 mM KCl in the presence or absence of TPP as defined. Cleavage products were separated by denaturing 10% PAGE, visualized by PhosphorImager (GE Healthcare), and quantified using ImageQuant software. *K*_{D} values were determined by plotting the normalized fraction of RNA cleaved versus the logarithm of ligand concentration used. The results for in-line probing of 197 NMT1 and 115 NMT1 are depicted in Figure 8 and Figure 9, the results for 261 NMT1 are depicted in Fig. 10 and the results for 273 *NMT1* are depicted in Fig. 11. Alternative base pairing similar to that being examined in the 273 *NMT1* construct is observed in other fungal TPP riboswitches located in *NMT1* mRNAs (Fig. 12).

*Strains, plasmids and media. N. crassa* 87-74 (*bd*; *frq*⁺ *a*; *his-3*) () was used as a host strain for transformation. The plasmid pLL07 (Froehlich 2004), which carries a firefly luciferase (*LUC*) reporter gene, was used for reporter gene construction. The start codon for the *LUC* reporter gene in pLL07 was removed by QuikChange (Stratagene) site directed mutagenesis to obtain the plasmid pLL09.

The promoter for the beta-tubulin gene in *N. crassa* ranging from positions 1 to 355 (accession number M13630) was amplified from genomic DNA by PCR using primers DNA3 and DNA4. The amplified DNA fragment was digested with *Mfe*I and *Eco*RI and inserted into the *Eco*RI site of pLL09 to obtain pLUC. The sequence of pLUC was confirmed by sequencing (HHMI Keck Foundation Biotechnology Resource Center at Yale University). *E. coli* Top 10 cells (Invitrogen) were used as a host during manipulation of plasmids.

Standard liquid medium used for growth of *N. crassa* contained 2% glucose, 0.5% L-arginine, 1 X Vogel's minimal medium, and 50 ng/ml biotin. Solid medium used for *N. crassα* growth (slants) contained 1 X Vogel's minimal medium, 2% sucrose and 1.5% agar. Medium used for selection of *N. crassa* transformants contained 1X Vogel's minimal medium, 2% agar, 2% L-sorbose, 0.05% fructose and 0.05% glucose. For homokaryon isolation, 0.1 X Westergaard's medium containing 1% IAA was used (Westergaard 1947).

*N. crassa transformations and reporter assays.* Transformation of *N. crassa* was conducted using electroporation of freshly suspended macroconidia as previously described (Davis 2000; Loros 1991; Vann 1995). Insertion of the target gene was verified by PCR with insert specific primers from genomic DNA. Homokaryotic strains were isolated as previously described (Ebbole 1990).

*Construction of 5' UTR-reporter gene plasmids.* Cloning of the 5' UTR of the *NMT1* gene (accession number AY007661) was achieved by PCR amplification of a 378 bp fragment (beginning with the annotated transcription start site) from genomic DNA of *N. crassa* with primers DNA5 and DNA6. The resulting wild-type (WT) PCR DNA was first cloned using a TOPO TA cloning kit (Invitrogen). The *NMT1* fragment was released from the vector by *Eco*RI and *Xba*I restriction enzyme digestion and cloned into appropriate sites of pLUC.

For generation of the aptamer mutants M1 through M9 and the splice site mutants M14 through M16, PCR mutagenesis was performed on the WT *NMT1* containing TOPO vector (see primer list). After confirmation of mutagenesis by DNA sequencing, each mutant *NMT1* fragment was cloned into *Eco*RI/*Xba*I sites of pLUC. For cloning of the P3 deletion construct (M10) *NMT1* was amplified in two fragments with primers DNA5/DNA25 and DNA26/DNA6, in which the overlapping region deletes much of the natural P3a stem. These two fragments were used as a template in a subsequent PCR with the outer primers DNA5 and DNA6. The resulting fragment was digested with EcoRI and *Xba*I and cloned into pLUC. Preparation of the I-2R and I-3R constructs and variants of *NMT1* was achieved by RT-PCR amplification of these two alternatively spliced products with primers DNA5 and DNA6. The resulting PCR products were cloned and mutated as described above.

To generate a *NCU01977.1* 5' region-*LUC* reporter fusion (Fig. 12), a 478 nucleotide fragment starting 94 nucleotides upstream of the predicted start codon was amplified from genomic DNA of *N. crassa* with primers DNA41 and DNA42 and cloned into *Eco*RI/*Xba*I sites of pLUC as described above. The integrity of all constructs was confirmed by sequencing (HHMI Keck Foundation Biotechnology Resource Center at Yale University). In all constructs, the original start codon of the main ORF (*NMT1* or *NCU01977.1*) was fused in frame with the *LUC* reporter sequence.

*Luciferase reporter gene assays.* Mycelia from *N. crassa* transformed with LUC reporter constructs were isolated by filtration and approximately 100 mg of tissue was ground to a fine powder. After addition of 100 µl 1 X Passive Lysis Buffer (Promega), the samples were vigorously mixed and incubated on ice for 30 min followed by centrifugation for 15 minutes at 13,000 g. Luciferase activity was determined for the resulting supernatant using the Luciferase Assay System (Promega) and a plate-reading luminometer (Wallac). Luciferase activity was normalized over total protein concentration of the extract as determined by Bradford Protein Assay (BioRad) and finally expressed relative to a reference construct. Luciferase background activity (untransformed *N. crassa*) was 0.05% relative to the wild-type *NMT1* construct in cells grown without added thiamine.

*Reverse transcriptase - polymerase chain reaction (RT-PCR) analyses.* Total RNA was isolated from mycelia using TRIzol® LS reagent (Invitrogen) according to the manufacturer's directions. 5 µg of total RNA was treated with RNase free DNase I (Promega) for 30 min at 37°C. cDNA was generated by reverse transcription with a polyT primer for 1 hr at 42°C using SuperScript™ II Reverse Transcriptase (Invitrogen) according to the manufacturer's directions. To exclude the possibility of amplification products originating from contamination with genomic DNA, control reactions using RNA preparations before RT were performed and, for *NMT1,* a reverse primer spanning an exon-exon border in the coding region was used. RT-PCR analysis was conducted from cDNA generated by RT with a polyT primer,_but using sequence specific primers for RT gave identical results. Also using a reverse primer for RT-PCR that bound to a downstream exon of the coding region of *NMT1* did not result in any difference (data not shown). This indicates that all transcript forms are polyadenylated and splicing of introns downstream of the riboswitch containing intron is not affected. For sequence identification all amplification products were cloned and confirmed by sequencing several independent clones. Moreover a general effect on splicing control by the addition of thiamine to the growth medium (Fig. 7) was not observed. In addition, RT-PCR was used to determine the extent of splicing from a downstream intron in *NMT1,* which was found to splice constitutively both in the absence and presence of added thiamine in the growth medium.

A specific primer combination was used for every gene with one primer binding to the annotated 5' end of the transcript and a second primer binding immediately downstream of the riboswitch-containing intron. For the *NMT1* gene, the primers used for PCR were DNA37 and DNA38 (Fig. 14), corresponding to the annotated 5' end of the *NMT1* mRNA and a region approximately 50 nucleotides downstream of the TPP riboswitch-containing intron, respectively. For the *THI4* (*CyPBP37*) gene, the primers used were DNA39 and DNA40, corresponding to the 5' end of the mRNA and a region approximately 125 nucleotides downstream of the TPP riboswitch-containing intron, respectively. The 5' region of *NCU01977.1* was amplified with primers DNA41 and DNA42 binding 94 nucleotides in front of the predicted start codon and 22 nucleotides downstream of the predicted 3' end of the riboswitch-containing intron, respectively. The PCR products were separated by 2% agarose gel electrophoresis and visualized by ethidium bromide staining. The different amplification products were purified using QIAquick Gel Extraction Kit (Qiagen) and cloned into the TOPO-TA cloning vector (Invitrogen) according to the manufacturer's instructions. The sequences of multiple clones for every product were analyzed by sequencing (HHMI Keck Foundation Biotechnology Resource Center at Yale University).

For the detection of *NMT1-LUC* fusion transcripts in *N. crassa* transformants, primers DNA37 and DNA43 were used, corresponding to the 5'end of the *NMT1* transcript and a region approximately 130 nucleotides downstream of the start of the *LUC* open reading frame. The native *NMT1* transcript, and some mutant constructs as indicated, carry the extended P3 stem (Fig. 10).

*Mechanisms of other fungal TPP riboswitches.* It is likely that the *N. crassa THI4* gene is repressed by TPP through a similar interplay of riboswitch action, alternative splicing, and uORF translation. In contrast, precursor *NCU01977.1* transcripts carry the TPP riboswitch aptamer in an intron that interrupts the main ORF. Translation of the unspliced transcript is disrupted by premature stop codons present in the intron. RT-PCR analysis of *NCU01977.1* mRNAs (Fig. 1b) shows that the ratio of spliced relative to unspliced mRNAs increases when thiamine is present, revealing that its TPP riboswitch functions as a genetic 'ON' switch that increases splicing and gene expression upon binding TPP. This conclusion also is supported by the analysis of a *NCU01977.1* reporter fusion construct expressed in *N. crassa,* which yields an increase in *LUC* expression in response to thiamine supplementation (Fig. 13).

It is understood that the disclosed method and compositions are not limited to the particular methodology, protocols, and reagents described as these may vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a ", "an", and "the" include plural reference unless the context clearly dictates otherwise. Thus, for example, reference to "a riboswitch" includes a plurality of such riboswitches, reference to "the riboswitch" is a reference to one or more riboswitches and equivalents thereof known to those skilled in the art, and so forth.

"Optional" or "optionally" means that the subsequently described event, circumstance, or material may or may not occur or be present, and that the description includes instances where the event, circumstance, or material occurs or is present and instances where it does not occur or is not present.

Ranges may be expressed herein as from "about" one particular value, and/or to "about" another particular value. When such a range is expressed, also specifically contemplated and considered disclosed is the range from the one particular value and/or to the other particular value unless the context specifically indicates otherwise. Similarly, when values are expressed as approximations, by use of the antecedent "about," it will be understood that the particular value forms another, specifically contemplated embodiment that should be considered disclosed unless the context specifically indicates otherwise. It will be further understood that the endpoints of each of the ranges are significant both in relation to the other endpoint, and independently of the other endpoint unless the context specifically indicates otherwise. Finally, it should be understood that all of the individual values and sub-ranges of values contained within an explicitly disclosed range are also specifically contemplated and should be considered disclosed unless the context specifically indicates otherwise. The foregoing applies regardless of whether in particular cases some or all of these embodiments are explicitly disclosed.

Unless defined otherwise, all technical and scientific terms used herein have the same meanings as commonly understood by one of skill in the art to which the disclosed method and compositions belong. Although any methods and materials similar or equivalent to those described herein can be used in the practice or testing of the present method and compositions, the particularly useful methods, devices, and materials are as described. Publications cited herein and the material for which they are cited are hereby specifically incorporated by reference. Nothing herein is to be construed as an admission that the present invention is not entitled to antedate such disclosure by virtue of prior invention. No admission is made that any reference constitutes prior art. The discussion of references states what their authors assert, and applicants reserve the right to challenge the accuracy and pertinency of the cited documents. It will be clearly understood that, although a number of publications are referred to herein, such reference does not constitute an admission that any of these documents forms part of the common general knowledge in the art.

Throughout the description and claims of this specification, the word "comprise" and variations of the word, such as "comprising" and "comprises," means "including but not limited to," and is not intended to exclude, for example, other additives, components, integers or steps.

Those skilled in the art will recognize, or be able to ascertain using no more than routine experimentation, many equivalents to the specific embodiments of the method and compositions described herein. Such equivalents are intended to be encompassed by the following claims.

### References

Blencowe, B. J. Alternative splicing: new insights from global analyses. Cell 126, 37-47 (2006).
Borsuk, P., et al. L-Arginine influences the structure and function of arginase mRNA in Aspergillus nidulans. Biol. Chem. 388, 135-144 (2007).
Buratti, E. & Baralle, F. E. Influence of RNA secondary structure on the pre-mRNA splicing process. Mol. Cell Biol. 24, 10505-10514 (2004).
Colot, H. V., Loros, J. J. & Dunlap, J. C. Temperature-modulated alternative splicing and promoter use in the circadian clock gene frequency. Mol. Biol. Cell 16, 5563-5571 (2005).
Davis R.H. Neurospora: Contributions of a model organism. Oxford University Press, New York, NY (2000).
Ebbole, D. & Sachs, M. S. A rapid and simple method for isolation of Neurospora crassa homokaryons using microconidia. Fungal Genet. Newsl. 37, 17-18 (1990).
Eddy, S. R. & Durbin, R. RNA sequence analysis using covariance models. Nucleic Acids Res. 22, 2079-2088 (1994).
Eddy, S. R. INFERNAL. Version 0.55. Distributed by the author. Department of Genetics, Washington University School of Medicine. St. Louis, Missouri.
Edwards, T. E. & Ferré-D'Amaré, A. R. Crystal structures of the Thi-box riboswitch bound to thiamine pyrophosphate analogs reveal adaptive RNA-small molecule recognition. Structure 14, 1459-1468 (2006).
Faou, P. & Tropschug, M. Neurospora crassa CyPBP37: a cytosolic stress protein that is able to replace yeast Thi4p function in the synthesis of vitamin Bl. J. Mol. Biol. 344, 1147-1157 (2004).
Faou, P. & Tropschug, M. A novel binding protein for a member of CyP40-type Cyclophilins: N. crassa CyPBP37, a growth and thiamine regulated protein homolog to yeast Thi4p. J. Mol. Biol. 333, 831-844 (2003).
Froehlich, A. C., Loros, J. J. & Dunlap, J. C. Rhythmic binding of a WHITE COLLAR-containing complex to the frequency promoter is inhibited by FREQUENCY. Proc. Natl. Acad. Sci. USA 100, 5914-5919 (2003).
Galagan, J. E., et al. Sequencing of Aspergillus nidulans and comparative analysis with A. fumigatus and A. oryzae. Nature 438, 1105-1115 (2005).
Kim, D.-S., Gusti, V., Pillai, S. G. & Gaur, R. K. An artificial riboswitch for controlling pre-mRNA splicing. RNA 11, 1667-1677 (2005)
Kubodera, T., et al., Thiamine-regulated gene expression of Aspergillus oryzae thiA requires splicing of the intron containing a riboswitch-like domain in the 5'-UTR. FEBS Lett. 555, 516-520 (2003).
Loros, J. J. & Dunlap, J.C. Neurospora crassa clock-controlled genes are regulated at the level of transcription. Mol. Cell. Biol. 11, 558-563 (1991).
McColl, D., Valencia, C. A. & Vierula, P. J. Characterization and expression of the Neurospora crassa nmt-1 gene. Curr. Genet. 44, 216-223 (2003).
Mandal, M. & Breaker, R. R. Gene regulation by riboswitches. Nature Rev. Mol. Cell Biol. 5, 451-463 (2004).
Matlin, A. J., Clark, F. & Smith, C. W. Understanding alternative splicing: towards a cellular code. Nat. Rev. Mol. Cell Biol. 6, 386-398 (2005).
Maundrell, K. nmt1 of fission yeast: a highly expressed gene completely repressed by thiamine. J. Biol. Chem. 265, 10857-10864 (1989).
Mehra, A., Morgan, L., Bell-Pedersen, D., Loros, J. & Dunlap, J. C. Watching the Neurospora Clock Tick. Abstract in: Soc. Res. Biol. Rhythms, Amelia Island, FL, Society for Research on Biological Rhythms 27 (2002).
Mironov, A. S., et al. Sensing small molecules by nascent RNA: a mechanism to control transcription in bacteria. Cell 111, 747-756 (2002).
Nahvi, A., Sudarsan, N., Ebert, M. S., Zou, X., Brown, K. L. & Breaker, R. R. Genetic control by a metabolite binding mRNA. Chem. Biol. 9, 1043-1049 (2002).
Orbach, M. J., Porro, E. B. & Yanofsky, C. Cloning and characterization of the gene for beta-tubulin from a benomyl-resistant mutant of Neurospora crassa and its use as a dominant selectable marker. Mol. Cell. Biol. 6, 2452-2461 (1986).
Romfo, C. M., Alvarez, C. J., van Heeckeren, W. J., Webb, C. J. & Wise, J. A. Evidence for splice site pairing via intron definition in Schizosaccharomyces pombe. Mol. Cell. Biol. 20, 7955-7970 (2000).
Seetharaman, S., Zivarts, M., Sudarsan, N. & Breaker R. R. Immobilized RNA switches for the analysis of complex chemical and biological mixtures. Nature Biotechnol. 19, 336-341 (2001).
Serganov, A., Polonskaia, A., Phan, A. T., Breaker, R. R. & Patel, D.J. Structural basis for gene regulation by a thiamine pyrophosphate-sensing riboswitch. Nature 441, 1167-1171 (2006).
Soukup, G. A. & Breaker, R. R. Relationship between internucleotide linkage geometry and the stability of RNA. RNA 5, 1308-1325 (1999).
Sudarsan N., Barrick J. E. & Breaker R. R. Metabolite-binding RNA domains are present in the genes of eukaryotes. RNA 9, 644-647 (2003).
Thore, S., Leibundgut, M. & Ban, N. Structure of the eukaryotic thiamine pyrophosphate riboswitch with its regulatory ligand. Science 312, 1208-1211 (2006).
Vann, D. C. Electroporation-based transformation of freshly harvested conidia of Neurospora crassa. Fungal Genet. Newsl. 42A, 53 (1995).
Vilela, C. & McCarthy, J. E. Regulation of fungal gene expression via short open reading frames in the mRNA 5' untranslated region. Mol. Microbiol. 49, 859-867 (2003).
Welz, R. & Breaker, R. R. Ligand binding and gene control characteristics of tandem riboswitches in Bacillus anthracis. RNA 13, (Advance Online Article) (2007).
Westergaard, M. & Mitchell, H. K. Neurospora V. A synthetic medium favoring sexual reproduction. Amer. J. Bot. 34, 573-577 (1947).
Winkler, W. C. & Breaker, R. R. Regulation of bacterial gene expression by riboswitches. Annu. Rev. Microbiol. 59, 487-517 (2005).
Winkler, W. C., Nahvi, A. & Breaker, R. R. Thiamine derivatives bind messenger RNAs directly to regulate bacterial gene expression. Nature 419, 952-956 (2002).
Mandal, M. & Breaker, R. R. Gene regulation by riboswitches. Nature Rev. Mol. Cell Biol. 5, 451-463 (2004).
Fuchs, R. T., Grundy, F. J. & Henkin, T. M. The S(MK) box is a new SAM-binding RNA for translational regulation of SAM synthetase. Nat. Struct. Mol. Biol. 13, 226-233 (2006).
Roth, A. et al. A eubacterial riboswitch selective for the queuosine precursor preQ1 contains an unusually small aptamer domain. Nat. Struct. Mol. Biol. (in press) (2007).
Rodionov, D. A., Vitreschak, A. G., Mironov, A. A. & Gelfand, M. S. Comparative genomics of thiamine biosynthesis in prokaryotes. J. Biol. Chem. 277, 48949-48959 (2002).
Vitreschak, A. G., Rodionov, D. A., Mironov, A. A. & Gelfand, M. S. Regulation of riboflavin biosynthesis and transport genes in bacteria by transcriptional and translational attenuation. Nucleic Acids Res. 30, 3141-3151 (2002).
Vitreschak, A. G., Rodionov, D. A., Mironov, A. A. & Gelfand, M. S. Regulation of the vitamin B12 metabolism and transport in bacteria by a conserved RNA structural element. RNA 9, 1084-1097 (2003).
Nahvi, A., Barrick, J. E. & Breaker, R. R. Coenzyme B12 riboswitches are widespread genetic control elements in prokaryotes. Nucleic Acids Res. 32, 143-150 (2004).
Batey, R.T., Gilbert, S.D. & Montange R.K. Structure of a natural guanine-responsive riboswitch complexed with the metabolite hypoxanthine. Nature 432, 411-415 (2004).
Serganov, A. et al. Structural basis for discriminative regulation of gene expression by adenine- and guanine-sensing mRNAs. Chem. Biol. 11, 1-13 (2004).
Montange, R. K. & Batey, R. T. Structure of the S-adenosylmethionine riboswitch regulatory mRNA element. Nature 441, 1172-1175 (2006).
Thore, S., Leibundgut, M. & Ban, N. Structure of the eukaryotic thiamine pyrophosphate riboswitch with its regulatory ligand. Science 312, 1208-1211 (2006).
Serganov, A., Polonskaia, A., Phan, A. T., Breaker, R. R. & Patel, D.J. Structural basis for gene regulation by a thiamine pyrophosphate-sensing riboswitch. Nature 441, 1167-1171 (2006).
Edwards, T. E. & Ferré-D'Amaré, A. R. Crystal structures of the Thi-box riboswitch bound to thiamine pyrophosphate analogs reveal adaptive RNA-small molecule recognition. Structure 14, 1459-1468 (2006).
Sudarsan N., Barrick J. E. & Breaker R. R. Metabolite-binding RNA domains are present in the genes of eukaryotes. RNA 9, 644-647 (2003).

## Claims

1. A method of inhibiting fungal growth, the method comprising:
(a) identifying a subject with a fungal infection;
(b) administering to the subject an effective amount of a compound that binds a TPP-responsive riboswitch, wherein the compound inhibits fungal growth by binding to a TPP-responsive riboswitch, thereby inhibiting fungal growth.

2. The method of claim 1, wherein inhibiting fungal growth comprises a 10% or more reduction in fungal biomass.

3. The method of claim 1 or 2, wherein the compound has the formula: 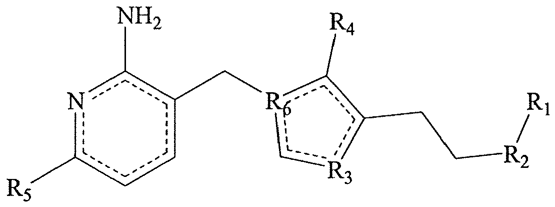 wherein the compound can bind a TPP-responsive riboswitch or derivative thereof,
wherein R₁ is positively charged,
wherein R₂ and R₃ are each independently C, O, or S,
wherein R₄ is CH₃, NH₂, OH, SH, H or not present,
wherein R₅ is CH₃, NH₂, OH, SH, or H,
wherein R₆ is C or N, and
wherein ------ each independently represent a single or double bond.

4. The method of claim 3, wherein R₁ is phosphate, diphosphate or triphosphate.

5. The method of claim 3 or 4, wherein R₁ is diphosphate.

6. The method of any of claims 3 to 5, wherein R₂ is O.

7. The method of any of claims 3 to 6, wherein R₃ is S.

8. The method of any of claims 3 to 7, wherein R₄ is CH₃.

9. The method of any of claims 3 to 8, wherein R₅ is CH₃.

10. The method of any of claims 3 to 9, wherein R₆ is N.

11. The method of any of claims 1 to 10, wherein the compound is not thiamine pyrophosphate, thiamine phosphate, thiamine, pyrithiamine, or pyrithiamine pyrophosphate.
